# EUROPEAN PATENT APPLICATION

(11) **EP 1 876 238 A1**
(43) Date of publication of application: **09.01.2008**
(21) Application number: 06732494.7
(22) Date of filing: 26.04.2006
(51) Int. Cl.: C12N 15/09, A01K 67/027, A61K 31/7088, A61K 38/00, A61K 39/395, A61K 45/00, A61K 48/00, A61P 3/04, A61P 9/12, A61P 13/00, A61P 13/12, A61P 35/00, C07K 14/575, C07K 16/26, C12N 1/15, C12N 1/19, C12N 1/21, C12N 5/10, G01N 33/15, G01N 33/50, G01N 33/53

(54) **NOVEL POLYPEPTIDE AND USE THEREOF**

(30) Priority: 27.04.2005 JP 2005130191
(71) Applicant: Takeda Pharmaceutical Company Limited, Osaka-shi, Osaka 541-0045 (JP)
(72) Inventor: TAKAO, Toshifumi c/o Res Cent for struct. & Functional Proteins, Suita-shi, Osaka 565-0871 (JP); NAKAZATO, Masamitsu c/o Neurology, Faculty of Medicine, Miyazaki-gun, Miyazaki 889-1692 (JP)
(74) Representative: Kalhammer, Georg
(86) International application number: PCT/JP2006/309192
(87) International publication number: WO 2006/118313

(57) **Abstract**

The present invention provides various useful polypeptides and so on. In particular, a polypeptide comprising the amino acid sequence represented by SEQ ID NO: 4, SEQ ID NO: 5, SEQ ID NO: 6, SEQ ID NO: 7, SEQ ID NO: 8 or SEQ ID NO: 9, a compound or its salt that promotes the activity of said polypeptide, etc. can be utilized as a preventive/therapeutic agent for, e.g., renal edema, dysuria, hyponatremia, syndrome of inappropriate secretion of antidiuretic hormone, hypertension or the like. Furthermore, a compound or its salt that inhibits the activity of the polypeptide is useful as a preventive/therapeutic agent for, e.g., urine storage disorders, polyuria, diabetes insipidus, hypernatremia, metabolic alkalosis, hypokalemia, Cushing syndrome or the like. Moreover, the polypeptide comprising the amino acid sequence represented by SEQ ID NO: 7, SEQ ID NO: 8 or SEQ ID NO: 9 can be utilized as a preventive/therapeutic agent for anorexia, etc., an eating (appetite) stimulant, or the like.

## Description

### TECHNICAL FIELD

The present invention relates to novel polypeptides or salts thereof, polynucleotides encoding the same, use thereof, and so on.

### BACKGROUND ART

VGF is a secretory polypeptide found in neurons and endocrine cells. The vgf gene was originally isolated as a gene rapidly induced in rat pheochromocytoma PC12 cell line by treatment withNGF (Nat. Cell Biol., 2, 703-708 (2000)). VGF is synthesized in nerve cells and neuroendocrine cells, transported to dense core vesicles and released via the regulated secretory pathway (EMBO J., 8, 2217-2223 (1989)). It is reported that VGF takes part in energy homeostasis through targeted ablation of VGF (Neuron, 23, 537-548 (1999), Mt. Sinai J. Med., 70, 93-100 (2003)). The nucleotides and amino acid sequences of human and rat VGF are reported; and it is also reported that VGF has physiological activities including engagement in metabolism, long term memory, etc. (International Publication Pamphlet Nos. 2001/07074, 2001/07477, 2000/70042, 2001/22920, 2001/64835, 2001/74298, 2003/062395, 2004/030615 and 2004/048938).

It is further reported that mRNA expression levels of the VGF gene are increased by NGF stimulation in rat PC12 cells (Science 229 (4711), 393-395 (1985)), VGF proteins are strongly expressed in the rat suprachiasmatic nucleus (J. Neurosci., 9 (12), 4122-4137 (1989)), mRNA expression levels of the VGF gene are enhanced in the golden hamster suprachiasmatic nucleus 3 to 9 hours after light exposure and VGF is involved in the regulation of a circadian rhythm (J. Comp. Neurol., 378 (2), 229-238 (1997)), and homozygotes lacking the VGF gene are small, hypermetabolic, hyperactive and infertile, mRNA expression of the VGF gene is induced in the hypothalamic arcuate nuclei of fasted wild-type mice, and VGF has a possibility to be associated with eating and energy metabolism (Neuron, 23 (3), 537-548 (1999)).

It is furthermore reported that partial peptides of VGF were isolated from the bovine posterior pituitary (Endocrinology, 135 (6), 2742-2748 (1994)) and partial peptides of VGF were isolated from the rat brain extracts (J. Neurochem., 81 (3), 565-574 (2002)). It is also reported that administration of the C-terminal peptides of VGF to the hypothalamic paraventricular nucleus of male rats induced penile erection dose-dependently (Eur. J. Neurosci., 20 (11), 3035-3040 (2004)).

### DISCLOSURE OF THE INVENTION

Currently, various preventive/therapeutic agents for dysuria, hypertension, anorexia, urine storage disorders, diabetes insipidus, obesity, etc. are known and more excellent preventive/therapeutic agents for these diseases have been desired.

The present inventors have made intensive studies to solve the foregoing problems and found that the polypeptide comprising the amino acid sequence represented by SEQ ID NO: 4, SEQ ID NO: 5 or SEQ ID NO: 6 has vasopressin release inhibiting activity. Also, the polypeptide having the amino acid sequence represented by SEQ ID NO: 7, SEQ ID NO: 8 or SEQ ID NO: 9 has eating stimulating activity as well as vasopressin release inhibiting activity. Based on these findings, further investigations were continued. As a result, the inventors have come to accomplish the present invention.

That is, the present invention provides the following features, and so on.
(1) A polypeptide comprising the same or substantially the same amino acid sequence as the amino acid sequence represented by SEQ ID NO: 4, SEQ ID NO: 5 or SEQ ID NO: 6 (with the proviso that a polypeptide consisting of the amino acid sequence represented by SEQ ID NO: 1, SEQ ID NO: 2 or SEQ ID NO: 3 is excluded), its amide or its ester, or a salt thereof.
(2) The polypeptide according to (1) above, which consists of the same or substantially the same amino acid sequence as the amino acid sequence represented by SEQ ID NO: 4, SEQ ID NO: 5 or SEQ ID NO: 6; its amide or its ester, or a salt thereof.
(3) The polypeptide according to (1) above, which consists of the amino acid sequence represented by SEQ ID NO: 4, SEQ ID NO: 5 or SEQ ID NO: 6, its amide or its ester, or a salt thereof.
   (3a) The polypeptide according to (1) above, which consists of the amino acid sequence represented by SEQ ID NO: 4, its amide or its ester, or a salt thereof.
   (3b) The polypeptide according to (1) above, which consists of the amino acid sequence represented by SEQ ID NO: 5 or SEQ ID NO: 6, its amide or its ester, or a salt thereof.
(4) A partial peptide of the polypeptide according to (1) above, its amide or its ester, or a salt thereof.
(5) A polypeptide comprising the same or substantially the same amino acid sequence as the amino acid sequence represented by SEQ ID NO: 7, SEQ ID NO: 8 or SEQ ID NO: 9 (with the proviso that a polypeptide consisting of the amino acid sequence represented by SEQ ID NO: 1, SEQ ID NO: 2 or SEQ ID NO: 3 is excluded), its amide or its ester, or a salt thereof.
(6) The polypeptide according to (5) above, which consists of the same or substantially the same amino acid sequence as the amino acid sequence represented by SEQ ID NO: 7, SEQ ID NO: 8 or SEQ ID NO: 9; its amide or its ester, or a salt thereof.
(7) The polypeptide according to (5) above, which consists of the amino acid sequence represented by SEQ ID NO: 7, SEQ ID NO: 8 or SEQ ID NO: 9; its amide or its ester, or a salt thereof.
   (7a) The polypeptide according to (5) above, which consists of the amino acid sequence represented by SEQ ID NO: 7, its amide or its ester, or a salt thereof.
   (7b) The polypeptide according to (5) above, which consists of the amino acid sequence represented by SEQ ID NO: 8 or SEQ ID NO: 9, its amide or its ester, or a salt thereof.
(8) A partial peptide of the polypeptide according to (5) above, its amide or its ester, or a salt thereof.
(9) A polynucleotide comprising a polynucleotide encoding the polypeptide according to (1) above or its partial peptide.
(10) The polynucleotide according to (9) above, which is a DNA.
(11) The DNA according to (10) above, which comprises the base sequence represented by SEQ ID NO: 12 or SEQ ID NO: 13.
   (11a) The DNA according to (10) above, which comprises the base sequence represented by SEQ ID NO: 12, SEQ ID NO: 13 or SEQ ID NO: 17.
   (11b) The DNA according to (10) above, which comprises the base sequence represented by SEQ ID NO: 12.
   (11c) The DNA according to (10) above, which comprises the base sequence represented by SEQ ID NO: 13 or SEQ ID NO: 17.
(12) The DNA according to (10) above, which consists of the base sequence represented by SEQ ID NO: 12 or SEQ ID NO: 13.
   (12a) The DNA according to (10) above, which consists of the base sequence represented by SEQ ID NO: 12, SEQ ID NO: 13 or SEQ ID NO: 17.
   (12b) The DNA according to (10) above, which consists of the base sequence represented by SEQ ID NO: 12.
   (12c) The DNA according to (10) above, which consists of the base sequence represented by SEQ ID NO: 13 or SEQ ID NO: 17.
(13) A polynucleotide comprising a polynucleotide encoding the polypeptide according to (5) above or its partial peptide.
(14) The polynucleotide according to (13) above, which is a DNA.
(15) The DNA according to (14) above, which comprises the base sequence represented by SEQ ID NO: 14 or SEQ ID NO: 15.
   (15a) The DNA according to (14) above, which comprises the base sequence represented by SEQ ID NO: 14; SEQ ID NO: 15 or SEQ ID NO: 18.
   (15b) The DNA according to (14) above, which comprises the base sequence represented by SEQ ID NO: 14.
   (15c) The DNA according to (14) above, which comprises the base sequence represented by SEQ ID NO: 15 or SEQ ID NO: 18.
(16) The DNA according to (14) above, which consists of the base sequence represented by SEQ ID NO: 14 or SEQ ID NO: 15.
   (16a) The DNA according to (14) above, which consists of the base sequence represented by SEQ ID NO: 14, SEQ ID NO: 15 or SEQ ID NO: 18.
   (16b) The DNA according to (14) above, which consists of the base sequence represented by SEQ ID NO: 14.
   (16c) The DNA according to (14) above, which consists of the base sequence represented by SEQ ID NO: 15 or SEQ ID NO: 18.
(17) A recombinant vector comprising the polynucleotide according to (9) or (13) above.
(18) A transformant transformed with the recombinant vector according to (17) above.
(19) A method for manufacturing the polypeptide according to (1) or (5) above, its partial peptide, or a salt thereof, which comprises culturing the transformant according to (18) above, forming and accumulating said polypeptide or the partial peptide thereof, and recovering it.
(20) An antibody against the polypeptide according to (1) above, its partial peptide, its amide or its ester, or a salt thereof.
(21) A method for quantifying the polypeptide according to (1) above, which comprises using the antibody according to (20) above.
(22) A method for diagnosing diseases associated with the function of the polypeptide according to (1) above, which comprises using the quantifying method according to (21) above.
(23) An antibody against the polypeptide according to (5) above, its partial peptide, its amide or its ester, or a salt thereof.
(24) A method for quantifying the polypeptide according to (5) above, which comprises using the antibody according to (23) above.
(25) A method for diagnosing diseases associated with the function of the polypeptide according to (5) above, which comprises using the quantifying method according to (24) above.
(26) An antisense polynucleotide comprising an entire or part of a base sequence complementary or substantially complementary to the base sequence of the polynucleotide according to (9) above.
(27) An antisense polynucleotide comprising an entire or part of a base sequence complementary or substantially complementary to the base sequence of the polynucleotide according to (13) above.
(28) A method for screening a compound or a salt thereof that promotes or inhibits the activity of the polypeptide according to (1) above, which comprises using said polypeptide, its partial peptide, its amide or its ester, or a salt thereof.
(29) A kit for screening a compound or a salt thereof that promotes or inhibits the activity of the polypeptide according to (1) above, which comprises said polypeptide, its partial peptide, its amide or its ester, or a salt thereof.
(30) A method for screening a compound or a salt thereof that promotes or inhibits the activity of the polypeptide according to (5) above, which comprises using said polypeptide, its partial peptide, its amide or its ester, or a salt thereof.
(31) A kit for screening a compound or a salt thereof that promotes or inhibits the activity of the polypeptide according to (5) above, which comprises said polypeptide, its partial peptide, its amide or its ester, or a salt thereof.
(32) A medicament comprising the polypeptide according to (1) above, its partial peptide, its amide or its ester, or a salt thereof.
(33) A medicament comprising the polynucleotide according to (9) above.
(34) A medicament comprising a compound or a salt thereof that promotes the activity of the polypeptide according to (1) above, its partial peptide, its amide or its ester, or a salt thereof.
(35) The medicament according to any one of claims (32) to (34) above, which is an agent for preventing/treating renal edema, dysuria, hyponatremia, syndrome of inappropriate secretion of antidiuretic hormone or hypertension.
(36) A medicament comprising a compound or a salt thereof that inhibits the activity of the polypeptide according to (1) above, its partial peptide, its amide or its ester, or a salt thereof.
(37) A medicament comprising the antibody according to (20) above.
(38) A medicament comprising the antisense polynucleotide according to (26) above.
(39) The medicament according to any one of claims (36) to (38) above, which is an agent for preventing/treating urine storage disorders, polyuria, diabetes insipidus, hypernatremia, metabolic alkalosis, hypokalemia or Cushing syndrome.
(40) A medicament comprising the polypeptide according to (5) above, its partial peptide, its amide or its ester, or a salt thereof.
(41) A medicament comprising the polynucleotide according to (13) above.
(42) A medicament comprising a compound or a salt thereof that promotes the activity of the polypeptide according to (5) above, its partial peptide, its amide or its ester, or a salt thereof.
(43) The medicament according to any one of claims (40) to (42) above, which is an agent for preventing/treating renal edema, dysuria, hyponatremia, syndrome of inappropriate secretion of antidiuretic hormone, hypertension or anorexia, or eating stimulant.
(44) A medicament comprising a compound or a salt thereof that inhibits the activity of the polypeptide according to (5) above, its partial peptide, its amide or its ester, or a salt thereof.
(45) A medicament comprising the antibody according to (23) above.
(46) A medicament comprising the antisense polynucleotide according to (27) above.
(47) The medicament according to any one of claims (44) to (46) above, which is an agent for preventing/treating urine storage disorders, polyuria, diabetes insipidus, hypernatremia, metabolic alkalosis, hypokalemia, Cushing syndrome, obesity or hyperphagia.
(48) A non-human transgenic animal bearing the polynucleotide according to (9) or (13) above, which is exogenous.
(49) A method for preventing/treating renal edema, dysuria, hyponatremia, syndrome of inappropriate secretion of antidiuretic hormone or hypertension, which comprises promoting the activity of the polypeptide according to (1) above, its partial peptide, its amide or its ester, or a salt thereof.
(50) A method for preventing/treating renal edema, dysuria, hyponatremia, syndrome of inappropriate secretion of antidiuretic hormone or hypertension, which comprises administering to a mammal an effective dose of the polypeptide according to (1) above, its partial peptide, its amide or its ester, or a salt thereof, the polynucleotide according to (9) above, or a compound or a salt thereof that promotes the activity of said polypeptide, its partial peptide, its amide or its ester, or a salt thereof.
(51) Use of the polypeptide according to (1) above, its partial peptide, its amide or its ester, or a salt thereof, the polynucleotide according to (9) above, or a compound or a salt thereof that promotes the activity of said polypeptide, its partial peptide, its amide or its ester, or a salt thereof, for the manufacture of an agent for preventing/treating renal edema, dysuria, hyponatremia, syndrome of inappropriate secretion of antidiuretic hormone or hypertension.
(52) A method for preventing/treating urine storage disorders, polyuria, diabetes insipidus, hypernatremia, metabolic alkalosis, hypokalemia or Cushing syndrome, which comprises inhibiting the activity of the polypeptide according to (1) above, its partial peptide, its amide or its ester, or a salt thereof.
(53) A method for preventing/treating urine storage disorders, polyuria, diabetes insipidus, hypernatremia, metabolic alkalosis, hypokalemia or Cushing syndrome, which comprises administering to a mammal an effective dose of a compound or a salt thereof that inhibits the activity of the polypeptide according to (1) above, its partial peptide, its amide or its ester, or a salt thereof, the antibody according to (20) above, or the antisense polynucleotide according to (26) above.
(54) Use of a compound or a salt thereof that inhibits the activity of the polypeptide according to (1) above, its partial peptide, its amide or its ester, or a salt thereof, the antibody according to (20) above, or the antisense polynucleotide according to (26) above, for the manufacture of an agent for preventing/treating urine storage disorders, polyuria, diabetes insipidus, hypernatremia, metabolic alkalosis, hypokalemia or Cushing syndrome.
(55) A method for preventing/treating renal edema, dysuria, hyponatremia, syndrome of inappropriate secretion of antidiuretic hormone, hypertension or anorexia, or a method for promoting eating, which comprises promoting the activity of the polypeptide according to (5) above, its partial peptide, its amide or its ester, or a salt thereof.
(56) A method for preventing/treating renal edema, dysuria, hyponatremia, syndrome of inappropriate secretion of antidiuretic hormone, hypertension or anorexia, or a method for promoting eating, which comprises administering to a mammal an effective dose of the polypeptide according to (5) above, its partial peptide, its amide or its ester, or a salt thereof, the polynucleotide according to (13) above, or a compound or a salt thereof that promotes the activity of said polypeptide, its partial peptide, its amide or its ester, or a salt thereof.
(57) Use of the polypeptide according to (5) above, its partial peptide, its amide or its ester, or a salt thereof, the polynucleotide according to (13) above, or a compound of a salt thereof that promotes the activity of said polypeptide, its partial peptide, its amide or its ester, or a salt thereof, for the manufacture of an agent for preventing/treating renal edema, dysuria, hyponatremia, syndrome of inappropriate secretion of antidiuretic hormone, hypertension or anorexia, or an eating stimulant.
(58) A method for preventing/treating urine storage disorders, polyuria, diabetes insipidus, hypernatremia, metabolic alkalosis, hypokalemia, Cushing syndrome, obesity or hyperphagia, which comprises inhibiting the activity of the polypeptide according to (5) above, its partial peptide, its amide or its ester, or a salt thereof.
(59) A method for preventing/treating urine storage disorders, polyuria, diabetes insipidus, hypernatremia, metabolic alkalosis, hypokalemia, Cushing syndrome, obesity or hyperphagia, which comprises administering to a mammal an effective dose of a compound or a salt thereof that inhibits the activity of the polypeptide according to (5) above, its partial peptide, its amide or its ester, or a salt thereof, the antibody according to (23) above, or the antisense polynucleotide according to (27) above.
(60) Use of a compound or a salt thereof that inhibits the activity of the polypeptide according to (5) above, its partial peptide, its amide or its ester, or a salt thereof, the antibody according to (23) above, or the antisense polynucleotide according to (27) above, for the manufacture of an agent for preventing/treating urine storage disorders, polyuria, diabetes insipidus, hypernatremia, metabolic alkalosis, hypokalemia, Cushing syndrome, obesity or hyperphagia.

Furthermore, the present invention provides a polypeptide, its amid or its ester, or a salt thereof selected from:
(I) The polypeptide according to (1) above, its amide or its ester, or a salt thereof; wherein substantially the same amino acid sequence as the amino acid sequence represented by SEQ ID NO: 4, SEQ ID NO: 5 or SEQ ID NO: 6 has at least 50% homology, preferably at least 60% homology, more preferably at least 70% homology, much more preferably at least 80% homology, still more preferably at least 90% homology, most preferably at least 95% homology, to the amino acid sequence represented by SEQ ID NO: 4, SEQ ID NO: 5 or SEQ ID NO: 6;
(II) The polypeptide according to (1) above, its amide or its ester, or a salt thereof, comprising the following amino acid sequence, wherein substantially the same amino acid sequence represented by SEQ ID NO: 4, SEQ ID NO: 5 or SEQ ID NO: 6 is (i) the amino acid sequence represented by SEQ ID NO: 4, SEQ ID NO: 5 or SEQ ID NO: 6, of which at least 1 or 2 (e.g., approximately 1 to 15, preferably approximately 1 to 10, more preferably several (1 to 5)) amino acids are deleted; (ii) the amino acid sequence represented by SEQ ID NO: 4, SEQ ID NO: 5 or SEQ ID NO: 6, to which at least 1 or 2 (e.g., approximately 1 to 15, preferably approximately 1 to 10, and more preferably several (1 to 5)) amino acids are added; (iii) the amino acid sequence represented by SEQ ID NO: 4, SEQ ID NO: 5 or SEQ ID NO: 6, wherein at least 1 or 2 (e.g., approximately 1 to 15, preferably approximately 1 to 10, and more preferably several (1 to 5)) amino acids are substituted by other amino acids; (iv) the amino acid sequence represented by SEQ ID NO: 4, SEQ ID NO: 5 or SEQ ID NO: 6, into which at least 1 or 2 (e.g., approximately 1 to 15, preferably approximately 1 to 10, more preferably several (1 to 5)) amino acids are inserted; or (v) combination of the amino acid sequences described above;
(III) A polynucleotide hybridizable to the polynucleotide according to (9) above under high stringent conditions;
(IV) The polypeptide according to (5) above, its amide or its ester, or a salt thereof; wherein substantially the same amino acid sequence as the amino acid sequence represented by SEQ ID NO: 7, SEQ ID NO: 8 or SEQ ID NO: 9 has at least 50% homology, preferably at least 60% homology, more preferably at least 70% homology, much more preferably at least 80% homology, still more preferably at least 90% homology, most preferably at least 95% homology, to the amino acid sequence represented by SEQ ID NO: 7, SEQ ID NO: 8 or SEQ ID NO: 9;
(V) The polypeptide according to (5) above, its amide or its ester, or a salt thereof comprising the following amino acid sequence, wherein substantially the same amino acid sequence represented by SEQ ID NO: 7, SEQ ID NO: 8 or SEQ ID NO: 9 is (i) the amino acid sequence represented by SEQ ID NO: 4, SEQ ID NO: 5 or SEQ ID NO: 6, of which at least 1 or 2 (e.g., approximately 1 to 15, preferably approximately 1 to 10, more preferably several (1 to 5)) amino acids are deleted; (ii) the amino acid sequence represented by SEQ ID NO: 7, SEQ ID NO: 8 or SEQ ID NO: 9, to which at least 1 or 2 (e.g., approximately 1 to 15, preferably approximately 1 to 10, and more preferably several (1 to 5)) amino acids are added; (iii) the amino acid sequence represented by SEQ ID NO: 7, SEQ ID NO: 8 or SEQ ID NO: 9, wherein at least 1 or 2 (e.g., approximately 1 to 15, preferably approximately 1 to 10, and more preferably several (1 to 5)) amino acids are substituted by other amino acids; (iv) the amino acid sequence represented by SEQ ID NO: 7, SEQ ID NO: 8 or SEQ ID NO: 9, into which at least 1 or 2 (e.g., approximately 1 to 15, preferably approximately 1 to 10, more preferably several (1 to 5)) amino acids are inserted; or (v) combination of the amino acid sequences described above; and
(VI) A polynucleotide hybridizable to the polynucleotide according to (13) above under high stringent conditions, and having substantially the same activities as the polypeptide or polynucleotide of the present invention, such as the vasopressin release inhibiting activity or eating stimulating activity, and the like.

### BRIEF DESCRIPTION OF THE DRAWINGS

FIG 1 shows the endogenous presence of NERP. In the figure, a through d indicate the analysis results of immunoreactive NERP-1 (open circle) and NERP-2 (solid circle) by RP-HPLC, respectively, in (a) human plasma, (b) rat hypothalamus, (c) rat anterior pituitary and (d) rat posterior pituitary. Arrow 1 and arrow 2 denote the eluted positions of synthetic NERP-1 and synthetic NERP-2, respectively.
FIG 2 shows the localization of NERP-immunoreactive cells in rat endocrine tissues.
FIG 3 shows the colocalization of ERP 1 (red) and NERP-2 (red) with AVP (green) and oxytocin (green) in the SON. Scale bars designate 50 µm. The inset drawing shows the representative immunoelectron micrographs ofNERP in rat SON neurons, indicating the colocalization ofNERP (10 nm gold particles, red arrow) with AVP and oxytocin (5 nm gold particles, green arrow) in the SON. Scale bars designate 1 µm.
FIG 4 shows the increased VGF gene expression in the PVN (arrow) and SON (arrowhead) following 48 hour water deprivation.
FIG 5 shows the results of quantitative densitometric analysis of film autoradiography (FIG 4) in PVN and SON by a densitometry, wherein asterisk * denotes P<0.001 (as compared to the control group).
FIG 6 shows the effects ofNERP on AVP secretion in vivo. An asterisk * denotes P<0.05 and two asterisks ** denotes P<0.01.
FIG 7 shows the effects ofNERP on AVP secretion from static incubation of PVN and SON in vitro. Black, white and shaded bars indicate the administration periods of NERP-1, AT-II and KCl, respectively, wherein an asterisk * denotes P<0.05 and two asterisks ** denotes P<0.01.
FIG 8 shows the effects of NERP-1 on AVP secretion in vitro from static incubation in the posterior pituitary. Black, white and shaded bars indicate the administration periods of NERP-1, AT-II and KCl, respectively, wherein an asterisk * denotes P<0.05 and two asterisks ** denotes P<0.01.
FIG 9 shows the increased frequency of IPSC in slice samples in vitro in response to NERP-1. Typical spontaneous IPSC obtained at time points of 1, 2 and 3, before, during and after application of NERP-2 are enlarged along the time axis and shown within a box.
FIG 10 shows the effects of NERP-1 of IPSC and EPSC in the SON neurons on the frequency, wherein an asterisk * denotes P<0.05 and two asterisks ** denotes P<0.01.
FIG 11 shows the effects of tetrodotoxin (TTX, 10⁻⁶ M) on NER-P-1-induced IPSC intensification. TTX was added 5 minutes after application of NERP-1.
FIG 12 shows the effects of hemoglobin (Hb, 10⁻⁶ M) on NERP-1-induced IPSC intensification. Hb was added 5 minutes after application of NERP-1.
FIG 13 shows the feeding of rats under free feeding who received ICV injection of NERP-1 (gray), NERP-2 (black) and NERP-2-Gly (shade), wherein an asterisk * denotes P<0.05 and two asterisks ** denotes P<0.001.
FIG 14 shows the results of immunohistochemical double staining of NERP-2 (red) and orexin (green) or MCH (green) in the lateral hypothalamus, wherein fx designates fornix and bars designate 100 µm. Costaining of NERP-2 and orexin is shown by yellow (center).
FIG 15 shows the effects of anti-orexin-A IgG (0.25 µg) and anti-orexin-B IgG (0.25 µg/10 µl) or anti-MCH IgG (0.5 µg/10 µl) on NERP-2-induced feeding in rats.
FIG 16 shows one-hour feeding of orexin-knockout mice and wild-type littermates who received ICV injection of NERP-2 (1 nmol/2 µl) or MCH (1 nmol/2 µl), wherein an asterisk * denotes P<0.05.
FIG 17 shows the locomotor activities of orexin-knockout mice and wild-type littermates who received ICV injection of NERP-2 (1 nmol), wherein an asterisk * denotes P<0.01.

### BEST MODE FOR CARRYING OUT THE INVENTION

In the specification, the polypeptide comprising the amino acid sequence represented by SEQ ID NO: 4, SEQ ID NO: 5 or SEQ ID NO: 6, its amide or its ester, or salts thereof are sometimes briefly referred to as "NERP-1". Further, the polypeptide comprising the amino acid sequence represented by SEQ ID NO: 7, SEQ ID NO: 8 or SEQ ID NO: 9, its amide or its ester, or salts thereof are sometimes briefly referred to as "NERP-2".

The polypeptide comprising the same or substantially the same amino acid sequence as the amino acid sequence represented by SEQ ID NO: 4, SEQ ID NO: 5 or SEQ ID NO: 6 (hereinafter sometimes referred to as "polypeptide A of the present invention") and the polypeptide comprising the same or substantially the same amino acid sequence as the amino acid sequence represented by SEQ ID NO: 7, SEQ ID NO: 8 or SEQ ID NO: 9 (hereinafter sometimes referred to as "polypeptide B of the present invention") may be any polypeptide derived from any cells of human and warm-blooded animals (e.g., guinea pig, rat, mouse, swine, sheep, bovine, monkey, dog, etc.) (e.g., splenocytes, nerve cells, glial cells, β cells of pancreas, pancreatic Langerhans islet, bone marrow cells, mesangial cells, Langerhans' cells, epidermic cells, epithelial cells, endothelial cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testicles, testis, ovary, placenta, uterus, bone, joint, skeletal muscle, etc.; the polypeptide may also be synthetic peptide.

The amino acid sequence comprising substantially the same amino acid sequence as that represented by SEQ ID NO: 4, SEQ ID NO: 5 or SEQ ID NO: 6 includes amino acid sequences having at least about 50% homology, preferably at least about 60% homology, more preferably at least about 70% homology, much more preferably at least about 80% homology, still more preferably at least about 90% homology and most preferably at least about 95% homology, to the amino acid sequence shown by SEQ ID NO: 4, SEQ ID NO: 5 or SEQ ID NO: 6; etc.

Homology of the amino acid sequences can be measured by using a homology scoring algorithm NCBI BLAST (National Center for Biotechnology Information Basic Local Alignment Search Tool).

Preferred examples of the polypeptides comprising substantially the same amino acid sequence as the amino acid sequence represented by SEQ ID NO: 4, SEQ ID NO: 5 or SEQ ID NO: 6 include polypeptides comprising substantially the same amino acid sequence as the amino acid sequence represented by SEQ ID NO: 4, SEQ ID NO: 5 or SEQ ID NO: 6 and having activities substantially equivalent to those of the polypeptides comprising the amino acid sequence represented by SEQ ID NO: 4, SEQ ID NO: 5 or SEQ ID NO: 6, etc.

The substantially equivalent activity includes, for example, activities possessed by the polypeptide of the present invention e.g., vasopressin release inhibiting activities, binding activities to receptors, cell stimulating activities on receptor-expressed cells (e.g., the activities that promote arachidonic acid release, acetylcholine release, intracellular Ca²⁺ release, intracellular cAMP production, intracellular cAMP production suppression, intracellular cGMP production, inositol phosphate production, change in cell membrane potential, phosphorylation of intracellular proteins, activation of c-fos, pH reduction, GTPγS binding activity, activation of cAMP-dependent protein kinase, activation of cGMP-dependent protein kinase, activation of phospholipid-dependent protein kinase, activation of mitogen-activated protein phosphorylase (MAP kinase)), preventive/therapeutic activities against the diseases described above. The term substantially equivalent is used to mean that the nature of these properties is equivalent in terms of quality (e.g., physiologically or pharmacologically). Thus, vasopressin release inhibiting activities, binding activities to receptors, cell stimulating activities on receptor-expressed cells, preventive/therapeutic activities against the diseases, etc. are preferably equivalent (e.g., about 0.01 to 100 times, preferably about 0.5 to 20 times, more preferably 0.5 to 2 times), but differences in degree such as a level of these activities, quantitative factors such as a molecular weight of the polypeptide, etc. may be present and allowable.

The activities such as vasopressin release inhibiting activities, binding activities to receptors, cell stimulating activities on receptor-expressed cells, preventive/therapeutic activities of the diseases, etc. can be determined according to publicly known methods. For example, vasopressin secretion inhibiting activities can be determined by the method in EXAMPLE 3 described below, etc.

The polypeptides comprising the following amino acid sequences are also used as polypeptide A of the present invention: (i) the amino acid sequence represented by SEQ ID NO: 4, SEQ ID NO: 5 or SEQ ID NO: 6, of which at least 1 or 2 (e.g., approximately 1 to 15, preferably approximately 1 to 10, more preferably several (1 to 5)) amino acids are deleted; (ii) the amino acid sequence represented by SEQ ID NO: 4, SEQ ID NO: 5 or SEQ ID NO: 6, to which at least 1 or 2 (e.g., approximately 1 to 15, preferably approximately 1 to 10, and more preferably several (1 to 5)) amino acids are added; (iii) the amino acid sequence represented by SEQ ID NO: 4, SEQ ID NO: 5 or SEQ ID NO: 6, wherein at least 1 or 2 (e.g., approximately 1 to 15, preferably approximately 1 to 10, and more preferably several (1 to 5)) amino acids are substituted by other amino acids; (iv) the amino acid sequence represented by SEQ ID NO: 4, SEQ ID NO: 5 or SEQ ID NO: 6, into which at least 1 or 2 (e.g., approximately 1 to 15, preferably approximately 1 to 10, more preferably several (1 to 5)) amino acids are inserted; or (v) combination of the amino acid sequences described above; and the like.

Specific examples of polypeptide A of the present invention include a polypeptide having the amino acid sequence represented by SEQ ID NO: 4, its amide or its ester, or salts thereof, a polypeptide having the amino acid sequence represented by SEQ ID NO: 5, its amide or its ester, or salts thereof, a polypeptide having the amino acid sequence represented by SEQ ID NO: 6, its amide or its ester, or salts thereof, and the like.

The partial peptide of polypeptide A of the present invention is not particularly limited but may be any polypeptide as long as it is a polypeptide having the activity substantially equivalent to that of polypeptide A of the present invention. In terms of the number of amino acids in the partial peptide, there are used peptides having, e.g., at least 5, preferably at least 10, preferably at least 15, and preferably at least 20 amino acid sequences, in the constituent amino acid sequence of polypeptide A of the present invention; etc.

Herein, the term "substantially equivalent activity" has the same meaning as described above. The "substantially equivalent activity" can be assayed in the same manner as described above.

The partial peptides may be (i) those wherein at least 1 or 2 (preferably several (1 to 4)) amino acids are deleted of the amino acid sequences described above, (ii) those wherein at least 1 or 2 (preferably approximately 1 to 15, more preferably approximately 1 to 10 and most preferably several (1 to 5)) amino acids are added to the amino acid sequences described above, or (iii) those wherein at least 1 or 2 (preferably several (1 to 4)) amino acids are substituted by other amino acids.

Specific examples of the partial peptides include a peptide having, e.g., the 10th to 26th amino acid sequence in the amino acid sequence represented by SEQ ID NO: 4, a peptide having e.g., the 10th to 25th amino acid sequence in the amino acid sequence represented by SEQ ID NO: 5, a peptide having e.g., the 10th to 25th amino acid sequence in the amino acid sequence represented by SEQ ID NO: 6, and the like. Among them, preferred are peptides having the amino acid sequences of 12th to 26th, 13th to 26th, 14th to 26th, 15th to 26th, 16th to 26th, 17th to 26th, 18th to 26th, 19th to 26th, 20th to 26th and 21 st to 26th in the amino acid sequence represented by SEQ ID NO: 4, peptides having the amino acid sequences of 11th to 25th, 12th to 25th, 13th to 25th, 14th to 25th, 15th to 25th, 16th to 25th, 17th to 25th, 18th to 25th, 19th to 25th and 20th to 25th in the amino acid sequence represented by SEQ ID NO: 5 or SEQ ID NO: 6, and the like.

Hereinafter, polypeptide A of the present invention and its partial peptides are sometimes collectively referred to as "polypeptide A of the present invention."

The amino acid sequence comprising substantially the same amino acid sequence as the amino acid sequence represented by SEQ ID NO: 7, SEQ ID NO: 8 or SEQ ID NO: 9 includes amino acid sequences having at least about 50% homology, preferably at least about 60% homology, more preferably at least about 70% homology, much more preferably at least about 80% homology, still more preferably at least about 90% homology and most preferably at least about 95% homology, to the amino acid sequence shown by SEQ ID NO: 7, SEQ ID NO: 8 or SEQ ID NO: 9; etc.

Homology of the amino acid sequences can be measured by using a homology scoring algorithm NCBI BLAST (National Center for Biotechnology Information Basic Local Alignment Search Tool).

Preferred examples of the polypeptides comprising substantially the same amino acid sequence as the amino acid sequence represented by SEQ ID NO: 7, SEQ ID NO: 8 or SEQ ID NO: 9 include polypeptides comprising substantially the same amino acid sequence as the amino acid sequence represented by SEQ ID NO: 7, SEQ ID NO: 8 or SEQ ID NO: 9 and having activities substantially equivalent to those of the polypeptides comprising the amino acid sequence represented by SEQ ID NO: 7, SEQ ID NO: 8 or SEQ ID NO: 9, etc.

The substantially equivalent activity includes, e.g., activities possessed by the polypeptide of the present invention, for example, a vasopressin release inhibiting activity, an eating stimulating activity, a binding activity to receptors, cell stimulating activities on receptor-expressed cells (e.g., the activities that promote arachidonic acid release, acetylcholine release, intracellular Ca²⁺ release, intracellular cAMP production, intracellular cAMP production suppression, intracellular cGMP production, inositol phosphate production, change in cell membrane potential, phosphorylation of intracellular proteins, activation of c-fos, pH reduction, GTPγS binding activity, activation of cAMP-dependent protein kinase, activation of cGMP-dependent protein kinase, activation of phospholipid-dependent protein kinase, activation of mitogen-activated protein phosphorylase (MAP kinase), preventive/therapeutic activities against the diseases described above. The term substantially equivalent is used to mean that the nature of these properties is equivalent in terms of quality (e.g., physiologically or pharmacologically). Thus, the vasopressin release inhibiting activity, eating stimulating activity, binding activity to receptors, cell stimulating activity on receptor-expressed cells, preventive/therapeutic activities of diseases, etc. are preferably equivalent (e.g., about 0.01 to 100 times, preferably about 0.5 to 20 times, more preferably 0.5 to 2 times), but differences in degree such as a level of these activities, quantitative factors such as a molecular weight of the polypeptide, etc. may be present and allowable.

The activities such as the vasopressin release inhibiting activity, eating stimulating activity, binding activity to receptors, cell stimulating activity on receptor-expressed cells, preventive/therapeutic activities of diseases, etc. can be determined according to publicly known methods. For example, the vasopressin release inhibiting activity can be determined by the method in EXAMPLE 3 described below, etc. and the eating stimulating activity can be determined by the method in EXAMPLE 5 described below, etc.

The polypeptides comprising the following amino acid sequences are used as polypeptide B of the present invention: (i) the amino acid sequence represented by SEQ ID NO: 7, SEQ ID NO: 8 or SEQ ID NO: 9, of which at least 1 or 2 (e.g., approximately 1 to 15, preferably approximately 1 to 10, more preferably several (1 to 5)) amino acids are deleted; (ii) the amino acid sequence represented by SEQ ID NO: 7, SEQ ID NO: 8 or SEQ ID NO: 9, to which at least 1 or 2 (e.g., approximately 1 to 15, preferably approximately 1 to 10, and more preferably several (1 to 5)) amino acids are added; (iii) the amino acid sequence represented by SEQ ID NO: 7, SEQ ID NO: 8 or SEQ ID NO: 9; wherein at least 1 or 2 (e.g., approximately 1 to 15, preferably approximately 1 to 10, and more preferably several (1 to 5)) amino acids are substituted by other amino acids; (iv) the amino acid sequence represented by SEQ ID NO: 7, SEQ ID NO: 8 or SEQ ID NO: 9, into which at least 1 or 2 (e.g., approximately 1 to 15, preferably approximately 1 to 10, more preferably several (1 to 5)) amino acids are inserted; or (v) combination of the amino acid sequences described above; and the like.

Specific examples of polypeptide B of the present invention include a polypeptide having the amino acid sequence represented by SEQ ID NO: 7, a polypeptide having the amino acid sequence represented by SEQ ID NO: 8, a polypeptide having the amino acid sequence represented by SEQ ID NO: 9, and the like.

The partial peptide of polypeptide B of the present invention is not particularly limited but may be any polypeptide as long as it is a polypeptide having the activity substantially equivalent to that of polypeptide B of the present invention. In terms of the number of amino acids in the partial peptide, there are used peptides having, e.g., at least 5, preferably at least 10, preferably at least 15 and preferably at least 20 amino acid sequences, in the constituent amino acid sequence of polypeptide B of the present invention; etc.

Herein, the term "substantially equivalent activity" has the same meaning as described above. The "substantially equivalent activity" can be assayed in the same manner as described above.

The partial peptides may be (i) those wherein at least 1 or 2 (preferably several (1 to 4)) amino acids are deleted of the amino acid sequences described above, (ii) those wherein at least 1 or 2 (preferably approximately 1 to 15, more preferably approximately 1 to 10 and most preferably several (1 to 5)) amino acids are added to the amino acid sequences described above, or (iii) those wherein at least 1 or 2 (preferably several (1 to 4)) amino acids are substituted by other amino acids.

Specific examples of the partial peptides include a peptide having e.g., the 20th to 38th amino acid sequence in the amino acid sequence represented by SEQ ID NO: 7, SEQ ID NO: 8 or SEQ ID NO: 9, and the like. Among them, preferred are peptides having the amino acid sequences of 24th to 38th, 25th to 38th, 26th to 38th, 27th to 38th, 28th to 38th, 29th to 38th, 30th to 38th, 31 st to 38th, 32nd to 38th and 33rd to 38th, and the like.

Hereinafter, polypeptide B of the present invention and its partial peptides are sometimes collectively referred to as "polypeptide B of the present invention."

Likewise, "polypeptide A of the present invention" and "polypeptide B of the present invention" are sometimes collectively referred to as the "polypeptide of the present invention."

As salts of the polypeptide of the present invention or its partial peptide, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Throughout the specification, the polypeptides and proteins are represented in accordance with the conventional way of describing peptides, that is, the N-terminus (amino terminus) at the left hand and the C-terminus (carboxyl terminus) at the right hand. In the polypeptides comprising the amino acid sequence represented by SEQ ID NO: 1, etc., the C-terminus may be in the form of a carboxyl group (-COOH), a carboxylate (-COO⁻), an amide (-CONH₂) or an ester (-COOR). Herein, examples of the ester group represented by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.; an α-naphthyl-C₁₋₂ alkyl group such as α-naphthylmethyl, etc.; and the like. In addition, pivaloyloxymethyl or the like, which is used widely as an ester for oral administration, may also be used.

In the polypeptide of the present invention, the C-terminal carboxyl group (-COOH) is preferably in the form of an amide (-CONH₂).

Specifically, there are preferably used, for example, polypeptides consisting of the amino acid sequence represented by SEQ ID NO: 4, 5, 6, 7, 8 or 9, in which the C-terminal amino acid residue is amidated, and the like.

Where the polypeptide of the present invention contains a carboxyl group (or a carboxylate) at a position other than the C-terminus, the carboxyl group may be amidated or esterified and such an amide or ester is also included within the polypeptide of the present invention. The aforesaid C-terminal esters or the like are used as the ester in this case.

The polypeptide of the present invention also includes, in the polypeptide described above, those wherein the amino group at the N-terminal methionine residue is protected with a protecting group (e.g., a C₁₋₆ acyl group such as a C₂₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₂₋₆ alkanoyl group, e.g., formyl group, acetyl group, etc.); those wherein a substituent on the side chain of an amino acid in the molecule is protected with a suitable protecting group, or conjugated peptides such as so-called glycopeptides having sugar chains; etc.

Preferably, polypeptide B of the present invention is pyroglutaminated at the N-terminal glutamine residue; in this case, the C-terminal amino acid residue may be in the form of an amide or ester and the amide is more preferred.

Specifically, there are preferably used the polypeptide consisting of the amino acid sequence represented by SEQ ID NO: 7, SEQ ID NO: 8 or SEQ ID NO: 9, wherein the glutamine residue is pyroglutaminated at the N-terminal end; the polypeptide consisting of the amino acid sequence represented by SEQ ID NO: 7, SEQ ID NO: 8 or SEQ ID NO: 9, wherein the amino acid residue is amidated at the C-terminal end and the glutamine residue is pyroglutaminated at the N-terminal end, etc. Also, in the partial peptide of the present invention, the C-terminus may be in any form of a carboxyl group (-COOH), a carboxylate (-COO⁻), an amide (-CONH₂) or an ester (-COOR). Where the partial peptide of the present invention contains a carboxyl group (or a carboxylate) at a position other than the C-terminus, the carboxyl group may be amidated or esterified and such an amide or ester is also included within the partial peptide of the present invention. Examples of the ester group in this case may be the C-terminal esters described above, etc.

As the partial peptide of the present invention, peptides wherein the C-terminal carboxyl group (-COOH) is in an amide form (-CONH₂) are preferred.

Furthermore, the partial peptide of the present invention includes those wherein the amino group at the N-terminal methionine residue is protected with a protecting group; those wherein the N-terminal glutamine residue is pyroglutaminated; those wherein the N-terminal region is cleaved in vivo and the glutamine residue thus formed is pyroglutaminated; those wherein a substituent on the side chain of an amino acid in the molecule is protected with a suitable protecting group, or conjugated peptides such as so-called glycopeptides having sugar chains; etc., in the same way as in the polypeptide of the present invention described above.

The polypeptide of the present invention or salts thereof can be prepared from the human or mammalian cells or tissues described above by publicly known methods for purification of peptides/proteins, or can also be prepared by culturing a transformant bearing DNA encoding the polypeptide comprising the amino acid sequence represented by SEQ ID NO: 4, SEQ ID NO: 5, SEQ ID NO: 6, SEQ ID NO: 7, SEQ ID NO: 8 or SEQ ID NO: 9. They can also be prepared by the method for synthesis of peptides/proteins, which will be later described.

Where the polypeptide of the present invention or its salts are prepared from the tissues or cells of human or mammal, the tissues or cells are homogenized, then extracted with an acid, an organic solvent, etc., and the extract can be purified and isolated by a combination of salting-out, dialysis, gel filtration, chromatography techniques such as reverse phase chromatography, ion exchange chromatography, affinity chromatography, or the like.

As described above, the polypeptide of the present invention can be prepared by publicly known methods for synthesis of polypeptides, or by cleaving polypeptides including the polypeptide of the present invention with an appropriate peptidase. As the methods for synthesis of polypeptides, for example, either solid phase synthesis or liquid phase synthesis may be used. In other words, the partial peptides or amino acids that can construct the polypeptide of the present invention are condensed with the remaining part. Where the product contains protecting groups, these protecting groups are removed to give the desired polypeptide. Publicly known methods for condensation and elimination of the protecting groups are described in (1) to (5) below.
(1) M. Bodanszky & M. A. Ondetti: Peptide Synthesis, Interscience Publishers, New York(1966)
(2) Schroeder & Luebke: The Peptide, Academic Press, New York (1965)
(3) Nobuo Izumiya, et al.: Peptide Gosei-no-Kiso to Jikken (Basics and experiments of peptide synthesis), published by Maruzen Co. (1975)
(4) Haruaki Yajima & Shunpei Sakakibara: Seikagaku Jikken Koza (Biochemical Experiment) 1, Tanpakushitsu no Kagaku (Chemistry of Proteins) IV, 205 (1977)
(5) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the polypeptide of the present invention can be purified and isolated by a combination of conventional methods for purification such as solvent extraction, distillation, column chromatography, liquid chromatography, recrystallization, etc. Where the polypeptide of the present invention obtained by the above methods is in a free form, it can be converted into an appropriate salt by a publicly known method or its modifications; where the polypeptide is obtained in a salt form, it can be converted into a free form by a publicly known method.

To synthesize amides of the polypeptide of the present invention, commercially available resins for peptide synthesis that are suitable for amide formation may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective peptide according to various condensation methods publicly known. At the end of the reaction, the polypeptide is excised from the resin and at the same time, the protecting groups are removed to obtain the objective polypeptide.

For condensation of the protected amino acids described above, a variety of activation reagents for polypeptide synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin. Solvents suitable for use to activate the protected amino acids or condense with the resin may be chosen from solvents that are known to be usable for polypeptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to peptide binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the amino groups of starting amino acids include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc. Examples of the protecting groups for a carboxyl group include 2-adamantyl, 4-nitrobenzyl, 4-methoxybenzyl, 4-chlorobenzyl and phenacyl as well as benzyloxycarbonyl hydrazide, t-butoxycarbonyl hydrazide, trityl hydrazide, and the like, in addition to the C₁₋₆ alkyl group, C₃₋₈ cycloalkyl group and C₇₋₁₄ aralkyl group described as R above.

The hydroxyl group of serine and threonine can be protected through, for example, its esterification or etherification. Examples of groups appropriately used for the esterification include a lower alkanoyl group such as acetyl group, etc., an aroyl group such as benzoyl group, etc., and a group derived from carbon such as benzyloxycarbonyl group, ethoxycarbonyl group, etc. Examples of groups appropriately used for the etherification include benzyl group, tetrahydropyranyl group, t-butyl group, etc.

Examples of groups for protecting the phenolic hydroxyl group of tyrosine include Bzl, C12-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], etc. As the amino acids in which the amino groups are activated in the starting material, the corresponding phosphoric amides, etc. are employed.

To eliminate (split off) the protecting groups, there are used catalytic reduction under hydrogen gas flow in the presence of a catalyst such as Pd-black, Pd-carbon, etc.; an acid treatment with anhydrous hydrogen fluoride, methanesulfonic acid, trifluoromethanesulfonic acid, trifluoroacetic acid, a mixture solution of these acids, etc.; a treatment with a base such as diisopropylethylamine, triethylamine, piperidine, piperazine, etc.; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. described above, as well as by a treatment with an alkali such as a dilute sodium hydroxide solution, dilute ammonia, etc.

Protection of functional groups that should not be involved in the reaction of the starting materials, protecting groups, elimination of the protecting groups, activation of functional groups involved in the reaction, etc. may be appropriately selected from publicly known groups and publicly known means.

In another method for producing the amides of the polypeptide of the present invention, for example, the α-carboxyl group of the carboxy terminal amino acid is first protected by amidation; the peptide chain is then extended to a desired length toward the amino group side. Thereafter, a peptide in which only the protecting group of the N-terminal α-amino group in the peptide chain has been eliminated from the peptide and a peptide (or amino acids) in which only the protecting group of the C-terminal carboxyl group has been eliminated are prepared. The two peptides are condensed in a mixture of the solvents described above. The details of the condensation reaction are the same as described above. After the protected peptide obtained by the condensation is purified, all the protecting groups are eliminated by the method described above to give the desired crude polypeptide. This crude polypeptide is purified by various known purification means. Lyophilization of the major fraction gives the amide of the desired polypeptide.

To prepare the esterified polypeptide of the present invention, for example, the α-carboxyl group in the carboxy terminal amino acid is condensed with a desired alcohol to prepare the amino acid ester to give the ester form of the desired polypeptide, in the same way as in the amide of the polypeptide.

The partial peptide of the polypeptide of the present invention can be prepared by digesting the polypeptide of the present invention with an appropriate peptidase or can be prepared in accordance with the aforesaid method for synthesis of polypeptides. The amide or ester of the partial peptide of the polypeptide of the present invention can also be prepared by a modification of the method for preparing the amide or ester described above. Also, salts of the partial peptide of the polypeptide of the present invention are the same as given for the salts of the polypeptide of the present invention described above.

Substantially the same substituent(s) of an amino acid(s) in the amino acid sequence can be selected, e.g., from other amino acids of the class to which the amino acid(s) belongs. Examples of nonpolar (hydrophobic) amino acids include alanine, leucine, isoleucine, valine, proline, phenylalanine, tryptophan, methionine, etc. Examples of polar (neutral) amino acids include glycine, serine, threonine, cystein, tyrosine, asparagine, glutamine, etc. Examples of positively charged (basic) amino acids include arginine, lysine, histidine, etc. Examples of negatively charged (acidic) amino acids include aspartic acid, glutamic acid, etc.

A labeled form of the polypeptides of the present invention or the partial peptides includes those labeled with an isotope, those labeled with fluorescence (fluorescence labeling with, e.g., fluorescein, etc.), those labeled with biotin, those labeled with an enzyme, etc., by publicly known methods.

Specifically, the polypeptides of the present invention, which is labeled with [³H], [¹²⁵I], [¹⁴C], [³⁵S], etc. by publicly known methods, can be used.

The polynucleotide encoding the polypeptide of the present invention may be any polynucleotide so long as it contains a polynucleotide comprising the base sequence encoding the polypeptide of the present invention. A DNA is preferred. The DNA may be any DNA so long as it contains a DNA comprising the DNA encoding the peptide comprising the same or substantially the same amino acid sequence as the amino acid sequence represented by SEQ ID NO: 4, SEQ ID NO: 5, SEQ ID NO: 6, SEQ ID NO: 7, SEQ ID NO: 8 or SEQ ID NO: 9. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid, phagemid, etc. The DNA may also be directly amplified by reverse transcriptase polymerase chain reaction (hereinafter abbreviated as RT-PCR) using RNA fraction prepared from the cells and tissues described above.

Examples of the DNA encoding the polypeptide of the present invention may be any one of a DNA comprising the base sequence represented by SEQ ID NO: 12, SEQ ID NO: 13 or SEQ ID NO: 17; or a DNA comprising a base sequence hybridizable to the base sequence represented by SEQ ID NO: 12, SEQ ID NO: 13 or SEQ ID NO: 17 under high stringent conditions and encoding a polypeptide which has the properties of substantially the same nature as those of the polypeptide comprising the amino acid sequence represented by SEQ ID NO: 4, SEQ ID NO: 5 or SEQ ID NO: 6 described above; or a DNA comprising the base sequence represented by SEQ ID NO: 14, SEQ ID NO: 15 or SEQ ID NO: 18; or a DNA comprising a base sequence hybridizable to the base sequence represented by SEQ ID NO: 14, SEQ ID NO: 15 or SEQ ID NO: 18 under high stringent conditions and encoding a polypeptide which has the properties of substantially the same nature as those of the polypeptide comprising the amino acid sequence represented by SEQ ID NO: 7, SEQ ID NO: 8 or SEQ ID NO: 9 described above.

As the DNA that is hybridizable to the base sequence represented by SEQ ID NO: 12, SEQ ID NO: 13, SEQ ID NO: 14, SEQ ID NO: 15, SEQ ID NO: 17 or SEQ ID NO: 18 under high stringent conditions, there are employed, for example, DNAs comprising base sequences having at least about 85% homology, preferably at least about 90% homology, preferably at least about 95% homology, preferably at least about 98% homology, to the base sequence represented by SEQ ID NO: 12, SEQ ID NO: 13, SEQ ID NO: 14, SEQ ID NO: 15, SEQ ID NO: 17 or SEQ ID NO: 18; and the like.

Homology of the base sequences can be measured under the following conditions (Expectation value = 10; gaps are allowed; filtering = ON; match score = 1; mismatch score = -3) using a homology scoring algorithm NCBI BLAST (National Center for Biotechnology Information Basic Local Alignment Search Tool).

The hybridization can be carried out by publicly known methods or by a modification thereof, for example, according to the method described in Molecular Cloning, 2nd. (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred. More specifically, as the DNA encoding the polypeptide containing the amino acid sequence represented by SEQ ID NO: 4, there may be employed a DNA containing the base sequence represented by SEQ ID NO: 12, etc., as the DNA encoding the polypeptide containing the amino acid sequence represented by SEQ ID NO: 5, there may be employed a DNA containing the base sequence represented by SEQ ID NO: 13, etc., as the DNA encoding the polypeptide containing the amino acid sequence represented by SEQ ID NO: 6, there may be employed a DNA containing the base sequence represented by SEQ ID NO: 17, etc., as the DNA encoding the polypeptide containing the amino acid sequence represented by SEQ ID NO: 7, there may be employed a DNA containing the base sequence represented by SEQ ID NO: 14, etc., as the DNA encoding the polypeptide containing the amino acid sequence represented by SEQ ID NO: 8, there may be employed a DNA containing the base sequence represented by SEQ ID NO: 15, etc., as the DNA encoding the polypeptide containing the amino acid sequence represented by SEQ ID NO: 9, there may be employed a DNA containing the base sequence represented by SEQ ID NO: 18, etc.

The polynucleotide (e.g., DNA) encoding the partial peptide of the present invention may be any polynucleotide so long as it contains the base sequence encoding the partial peptide of the present invention described above. The polynucleotide may also be any of genomic DNA, genomic DNA library, cDNA derived from the cells and tissues described above, cDNA library derived from the cells and tissues described above and synthetic DNA.

As the DNA encoding the partial peptide of the present invention, there are employed, for example, a DNA comprising a part of the DNA having the base sequence represented by SEQ ID NO: 12, SEQ ID NO: 13, SEQ ID NO: 14, SEQ ID NO: 15, SEQ ID NO: 17 or SEQ ID NO: 18, or a DNA comprising a base sequence hybridizable to the base sequence represented by SEQ ID NO: 12, SEQ ID NO: 13, SEQ ID NO: 14, SEQ ID NO: 15, SEQ ID NO: 17 or SEQ ID NO: 18 under high stringent conditions and comprising a part of DNA encoding a polypeptide having the activities of substantially the same nature as those of the polypeptide of the present invention, and the like.

The DNA hybridizable to the base sequence represented by SEQ ID NO: 12, SEQ ID NO: 13, SEQ ID NO: 14, SEQ ID NO: 15, SEQ ID NO: 17 or SEQ ID NO: 18 has the same significance as described above.

Methods for the hybridization and the high stringent conditions that can be used are the same as those described above.

### (Cloning of DNA)

The DNA encoding the polypeptide of the present invention can also be prepared by the following genetic engineering methods.

For cloning of the DNA that completely encodes the polypeptide of the present invention, the DNA may be amplified by PCR using synthetic DNA primers containing a part of the base sequence of the polypeptide of the present invention, or the DNA inserted into an appropriate vector can be selected by hybridization with a labeled DNA fragment or synthetic DNA that encodes a part or entire region of the polypeptide of the present invention. The hybridization can be performed, for example, according to the method described in Molecular Cloning (2nd ed., J. Sambrook et al., Cold Spring Harbor Lab. Press, 1989). The hybridization may also be performed using commercially available library in accordance with the protocol described in the attached instructions. The highly stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration of about 19 mM at a temperature of about 65°C are most preferred.

Conversion of the base sequence of the DNA can be effected by publicly known methods such as the ODA-LA PCR method, the Gapped duplex method or the Kunkel method or its modifications by using publicly known kits available as Mutan^{™}-super Express Km (manufactured by Takara Shuzo Co., Ltd.), Mutan^{™}-K (manufactured by Takara Shuzo Co., Ltd.), etc.

The cloned DNA encoding the polypeptide of the present invention can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof these translation initiation and termination codons may also be added by using an appropriate synthetic DNA adapter.

### (Expression vector)

The expression vector for the polypeptide of the present invention can be manufactured, for example, by (i) excising the desired DNA fragment from the DNA encoding the polypeptide of the present invention or the protein of the present invention, and then (ii) ligating the DNA fragment with an appropriate expression vector downstream a promoter in the vector.

Examples of the vector include plasmids derived form E. coli (e.g., pBR322, pBR325, pUC12 or pUC13), plasmids derived from Bacillus subtilis (e.g., pUB110, pTP5 or pC194), plasmids derived from yeast (e.g., pSH19 or pSH15), bacteriophages such as λ phage, etc., animal viruses such as retrovirus, vaccinia virus, baculovirus, etc. The promoter used in the present invention may be any promoter if it matches well with a host to be used for gene expression.

In the case of using animal cells as the host, examples of the promoter include SV40-derived promoter, retroviral LTR promoter, metallothionein (HT) promoter, heatshock promoter, cytomegalovirus (CMV) promoter, SRα promoter, etc. Where the host is bacteria of the genus Escherichia, preferred examples of the promoter include trp promoter, T7 promoter, lac promoter, recA promoter, λP_{L} promoter, lpp promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH1 promoter, GAL promoter, etc. When insect cells are used as the host, preferred examples of the promoter include polyhedrin prompter, P10 promoter, etc.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker in CHO (dhfr⁻) cells, selection can also be made on thymidine free media.

If necessary, a signal sequence that matches a host is added to the N-terminus of the polypeptide or its partial peptide. Examples of the signal sequence that can be used are PhoA signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

### (Transformant)

Using the vector bearing the thus constructed DNA encoding the polypeptide of the present invention, transformants can be manufactured.

Examples of the host, which may be employed, are bacteria belonging to the genus Escherichia, bacteria belonging to the genus Bacillus, yeasts, insects or insect cells, animal cells, etc.

Specific examples of the bacteria belonging to the genus Escherichia include Escherichia coli K12 DH1 [Proc. Natl. Acad. Sci. U.S.A., 60, 160 (1968)), JM103 (Nucleic Acids Research, 9, 309 (1981)], JA221 [Journal of Molecular Biology, 120, 517 (1978)], HB101 [Journal of Molecular Biology, 41, 459 (1969)), C600 (Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeasts include Saccharomyces cereviseae AH22, AH22R⁻, NA87-11A, DKD-5D, 20B-12, etc.

As the insect, for example, a larva of Bombyx mori, etc. can be used [Maeda, et al., Nature, 315, 592 (1985)].

Examples of insect cells include, for the virus AcNPV, Spodoptera frugiperda cells (Sf cells), MG1 cells derived from mid-intestine ofTrichoplusia ni, High Five^{™} cells derived from egg ofTrichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells [both cells are described in Vaughn, J. L. et al., In Vivo, 13, 213-217 (1977)], etc.

Examples of animal cells include monkey cells COS-7, Vero cells, Chinese hamster cells CHO, DHFR gene-deficient Chinese hamster cells CHO (dhfr⁻-CHO cells), mouse L cells, mouse 3T3 cells, mouse myeloma cells, human HEK293 cells, human FL cells, 293 cells, C127 cells, BALB3T3 cells, Sp-2/O cells, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Virology, 52, 456(1973).

Methods for introducing the expression vectors into the cells include, for example, the lipofection method [Proceedings of the National Academy of Sciences of the United States of America, 84, 7413 (1987)], the calcium phosphate method [Virology, 52, 456-467 (1973)], the electroporation method [EMBO J., 1, 841-845 (1982)], etc.

As described above, the transformant transformed by the expression vector comprising the DNA encoding the polypeptide of the present invention can be obtained.

Methods for stably expressing the polypeptide of the present invention using animal cells include methods of selecting the cells by clone selection in which the expression vectors described above are introduced into chromosomes. Specifically, transformants can be selected based on the selection markers described above. Further, repeated clone selections on the transformants thus obtained using the selection markers enable to acquire stable animal cell lines capable of highly expressing the polypeptide of the present invention. Furthermore, when the dhfr gene is used as the selection marker, incubation may be carried out by gradually increasing the concentration of MTX to select resistant cells, whereby the DNA encoding the polypeptide of the present invention is amplified in the cells concurrently with the dhfr gene to acquire animal cell lines with higher expression.

The transformants described above are cultured under conditions capable of expressing the DNA encoding the polypeptide of the present invention to produce and accumulate the polypeptide of the present invention, whereby the polypeptide of the present invention can be produced.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultivated generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G A. et al., Proc. Natl. Acad. Sci. U.S.A., 81, 5330 (1984)]. Preferably, pH of the medium is adjusted to about 5 to about 8. In general, the transformant is cultivated at about 20°C to about 35°C for about 24 to about 72 hours. If necessary, the culture can be aerated or agitated.

Where insect cells are used as the host, the transformant is cultivated in, for example, Grace's Insect Medium (Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 to 5 days and, if necessary and desired, the culture can be aerated or agitated.

Where animal cells are employed as the host, the transformant is cultivated in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of The Society for The Biological Medicine, 73, 1 (1950)], etc. Preferably, pH of the medium is adjusted to about 6 to about 8. The transformant is usually cultivated at about 30°C to about 40°C for about 15 to 60 hours and, if necessary and desired, the culture can be aerated or agitated.

Especially when CHO (dhfr⁻) cells and dhfr gene are used as selection markers, it is preferred to use substantially thymidine-free DMEM medium supplemented with dialyzed fetal calf serum.

### (Separation and purification of the polypeptide of the present invention)

The polypeptide of the present invention can be separated and purified from the culture described above, e.g., by the procedures described below.

When the polypeptide of the present invention is extracted from the culture or cells, after incubation the transformants or cells are collected by a publicly known method and suspended in an appropriate buffer. The transformants or cells are then disrupted by publicly known methods such as ultrasonication, a treatment with lysozyme and/or freeze-thaw cycling, followed by centrifugation, filtration, etc. Thus, the crude extract of the polypeptide of the present invention can be obtained. The buffer used for the procedures may contain a protein modifier such as urea or guanidine hydrochloride, or a surfactant such as Triton (registered trademark) X-100, etc.

When the polypeptide of the present invention is released in the culture, after completion of the incubation the supernatant can be separated from the transformants or cells to collect the supernatant by a publicly known method.

The polypeptide of the present invention contained in the culture supernatant or the extract thus obtained can be purified by appropriately combining publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis, chromatofocusing, etc.; and the like.

In the case where the polypeptide of the present invention thus obtained is in a free form, it can be converted into its salts by publicly known methods or modifications thereof. On the other hand, when the polypeptide is obtained in the form of a salt, it can be converted into its free form or into a different salt by publicly known methods or modifications thereof.

The polypeptide of the present invention produced by the recombinant can be treated, prior to or after the purification, with an appropriate protein modifying enzyme to appropriately modify the same or partially remove a (poly)peptide. Examples of the protein-modifying enzyme include trypsin, chymotrypsin, arginyl endopeptidase, protein kinase, glycosidase, or the like. The Edman degradation using the Edman reagent (phenyl isothiocyanate), which is publicly known, can be used to delete the N-terminal amino acid.

The presence of the thus produced the polypeptide of the present invention can be assayed by an enzyme immunoassay using a specific antibody, or the like.

### (Antibody)

The present invention further provides antibodies against the polypeptide of the present invention.

The antibodies against the polypeptide of the present invention may be any of polyclonal antibodies and monoclonal antibodies, as long as they are capable of recognizing the polypeptide of the present invention.

The antibodies against the polypeptide of the present invention may be manufactured by publicly known methods for producing antibodies or antisera, using as antigens the polypeptide of the present invention.

### [Preparation of Monoclonal Antibody]

### (a) Preparation of Monoclonal Antibody-Producing Cell

The polypeptide of the present invention is administered to a mammal either solely or together with carriers or diluents to the site where antibody production is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the polypeptide of the present invention, which will be described below, with the antiserum followed by assaying the binding activity of the labeling agent bound to the antibody. The fusion may be operated, for example, by the known Koehler and Milstein method [Nature, 256, 495 (1975)]. Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to 40°C, preferably at about 30 to 37°C for about 1 to 10 minutes, an efficient cell fusion can be performed.

Various methods can be used for screening of the monoclonal antibody-producing hybridoma. Examples of such methods include a method which comprises adding the supernatant of hybridoma to a solid phase (e.g., microplate) adsorbed with an antigen of the polypeptide of the present invention directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the polypeptide of the present invention labeled with a radioactive substance or an enzyme and detecting the monoclonal antibody bound to the solid phase.

The monoclonal antibody can be selected by publicly known methods or by modifications of these methods. In general, selection can be effected in a medium for animal cells supplemented with HAT (hypoxanthine, aminopterin and thymidine). Any selection and growth medium can be used as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for incubation of a hybridoma (SFM-101, Nissui Seiyaku Co., Ltd.), etc. can be used for the selection and growth medium. Incubation is carried out generally at 20 to 40°C, preferably at about 37°C, for 5 days to 3 weeks, preferably 1 to 2 weeks. The incubation can be conducted normally under 5% carbon dioxide gas. The antibody titer of the culture supernatant of hybridomas can be determined in the same way as in the assay for the antibody titer in antisera described above.

### (b) Purification of Monoclonal Antibody

Separation and purification of the monoclonal antibody can be carried out by methods applied to conventional separation and purification of immunoglobulins, in the same way as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

### [Preparation of Polyclonal Antibody]

The polyclonal antibody of the present invention can be produced by publicly known methods or their modifications. For example, a complex of immunogen (polypeptide antigen of the present invention) and a carrier protein is prepared, and a mammal is immunized with the complex in a manner similar to the method described above for the production of monoclonal antibodies. The product containing the antibody against the polypeptide of the present invention is collected from the immunized animal followed by separation and purification of the antibody.

In the complex of an immunogen and a carrier protein used to immunize a mammal, the type of carrier protein and the mixing ratio of a carrier to hapten may be any type in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produced by the administration. In order to potentiate the antibody productivity upon the administration, complete Freund's adjuvant or incomplete Freund's adjuvant may be administered. The administration is usually made once approximately in every about 2 to 6 weeks and about 3 to about 10 times in total.

The polyclonal antibody can be collected from the blood, ascites, etc., preferably from the blood, etc. of mammals immunized by the method described above.

The polyclonal antibody titer in antiserum can be assayed by the same procedure as used for determination of the serum antibody titer described above. The separation and purification of the polyclonal antibody can be carried out in accordance with the method for the separation and purification of immunoglobulins performed as applied to the separation and purification of monoclonal antibodies described hereinabove.

The antibody of the present invention described above is capable of specifically recognizing the polypeptide of the present invention. Therefore, the antibody can be used to quantify the polypeptide of the present invention in a test fluid, especially by the sandwich immunoassay, etc. In other words, the present invention provides, for example, the following quantification methods: (i) a method of quantifying the polypeptide of the present invention in a test fluid, which comprises reacting the antibody of the present invention competitively with the test fluid and a labeled form of the polypeptide of the present invention, and measuring the ratio of the labeled polypeptide of the present invention bound to the antibody; and,
(ii) a method of quantifying the polypeptide of the present invention in a test fluid, which comprises reacting the test fluid with the antibody of the present invention immobilized on a carrier and a labeled form of the antibody of the present invention simultaneously or sequentially, and measuring the activity of the label on the immobilizing carrier.

In the quantification method (ii) described above, it is preferred that one antibody recognizes the N-terminal region of the polypeptide of the present invention, and another antibody reacts with the C-terminal region of the polypeptide of the present invention.

Using the monoclonal antibodies against the polypeptide of the present invention (hereinafter sometimes referred to as the monoclonal antibodies against the present invention), the polypeptide of the present invention can be quantified. In addition, the polypeptide of the present invention can also be detected by tissue staining, or the like. For these purposes, the antibody molecule itself may be used, or F(ab')₂, Fab' or Fab fraction of the antibody molecule may also be used.

The quantification methods of the polypeptide of the present invention using the antibodies are not particularly limited. Any quantification method can be used, so long as the amount of an antibody, antigen, or antibody-antigen complex corresponding to the amount of antigen (e.g., the amount of the polypeptide of the present invention) in a test fluid can be detected by chemical or physical means and the amount of the antigen can be calculated from a standard curve prepared from standard solutions containing known amounts of the antigen. For example, the nephrometry, competitive method, immunometric method and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [¹²⁵I], [¹³¹I], [³H], [¹⁴C], etc. are used. As the enzyme described above, stable enzymes with a high specific activity are preferred; for example, β-galactosidase, β-glucosidase, alkaline phosphatase, peroxidase, malate dehydrogenase , etc. are used. Examples of the fluorescent substance used are fluorescamine and fluorescein isothiocyanate are used. For the luminescent substance, there are used, for example, luminol, luminol derivatives, luciferin, and lucigenin. Furthermore, the biotin-avidin system may be used for binding an antibody or antigen to the labeling agent.

For immobilization of an antigen or antibody, physical adsorption may be used. Chemical binding methods conventionally used for insolubilization or immobilization of the protein, enzymes, etc. may also be used. For the carrier, for example, insoluble polysaccharides such as agarose, dextran, cellulose, etc.; synthetic resin such as polystyrene, polyacrylamide, silicon, etc., glass, and the like are used.

In the sandwich method, the monoclonal antibody of the present invention which is immobilized is reacted with a test fluid (primary reaction), then with a labeled form of the monoclonal antibody of the present invention (secondary reaction), and the activity of the label on the immobilizing carrier is measured, whereby the amount of the polypeptide of the present invention in the test fluid can be quantified. The order of the primary and secondary reactions may be reversed, and the reactions may be performed simultaneously or with an interval. The labeling agent and the method for immobilization can be performed by some modifications of those described above.

In immunoassay by the sandwich method, the antibody used for immobilized or labeled antibodies is not necessarily one species, but a mixture of two or more antibodies may be used to increase the measurement sensitivity.

In the methods of assaying the polypeptide of the present invention by the sandwich method according to the present invention, antibodies that bind to different sites of the polypeptide of the present invention are preferably used as the monoclonal antibodies of the present invention for the primary and secondary reactions. That is, in the antibodies used for the primary and secondary reactions are, for example, when the antibody used in the secondary reaction recognizes the C-terminal region of the polypeptide of the present invention, it is preferable to use the antibody recognizing the region other than the C-terminal region for the primary reaction, e.g., the antibody recognizing the N-terminal region.

The monoclonal antibody of the present invention can be used for other assay systems than the sandwich method, for example, for competitive method, immunometric method, nephrometry, or the like. In the competitive method, an antigen in a test fluid and a labeled antigen are competitively reacted with an antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, etc., and an immobilized method either using an immobilized antibody as the primary antibody, or using a soluble antibody as the primary antibody and immobilized antibody as the secondary antibody.

In the immunometric method, an antigen in a test fluid and an immobilized antigen are competitively reacted with a given amount of labeled antibody, the solid phase is separated from the liquid phase, or an antigen in a test fluid and an excess amount of labeled antibody are reacted, the immobilized antigen is then added to bind the unreacted labeled antibody to the solid phase, and the solid phase is separated from the liquid phase. Then, the amount of the label in either phase is measured to quantify the antigen in the test fluid.

In the nephrometry, the insoluble precipitate produced after the antigen-antibody reaction in gel or solution is quantified. When the amount of an antigen in the test fluid is small and only a small amount of the precipitate is obtained, laser nephrometry using scattering of laser is advantageously employed.

For applying these individual immunological methods to the quantification methods of the present invention, any particular conditions or procedures are not required. Systems for measuring the polypeptide of the present invention are constructed by adding the usual technical consideration in the art to the conventional conditions and procedures. For the details of these general technical means, reference can be made to the following reviews and texts. For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As above, the polypeptide of the present invention can be quantified with good sensitivity, by using the antibody of the present invention.

Moreover, quantification of the polypeptide of the present invention in vivo using the antibody of the present invention enables to diagnose various diseases associated with dysfunction of the polypeptide of the present invention.

The antibody of the present invention can also be used to detect the polypeptide of the present invention, which is present in a test sample such as a body fluid, a tissue, etc. The antibody can also be used to prepare an antibody column used for purification of the polypeptide of the present invention, detect the polypeptide of the present invention in each fraction upon purification, analyze the behavior of the polypeptide of the present invention in the cells under investigation; etc.

### (Antisense Polynucleotide)

The antisense polynucleotide comprising a complementary or substantially complementary base sequence to the base sequence of a polynucleotide encoding the polypeptide of the present invention or its partial peptide (e.g., DNA (hereinafter these DNAs are sometimes collectively referred to as the DNA of the present invention in the description of the antisense polynucleotide) can be any antisense polynucleotide, so long as it possesses a base sequence complementary or substantially complementary to the base sequence of the DNA of the present invention and capable of suppressing the expression of said DNA, but antisense DNA is preferred.

The base sequence substantially complementary to the DNA of the present invention includes, for example, a base sequence having at least about 70% homology, preferably at least about 80% homology, more preferably at least about 90% homology and most preferably at least about 95% homology, to the entire base sequence or to its partial base sequence (i.e., complementary strand to the DNA of the present invention), and the like. In particular, the antisense polynucleotide having at least about 70% homology, preferably at least about 80% homology, more preferably at least about 90% homology and most preferably at least about 95% homology, to the complementary strand of the base sequence which encodes the N-terminal region of the polypeptide of the present invention (e.g., the base sequence around the initiation codon, etc.) is preferred in the entire base sequence of the complementary strand to the DNA of the present invention.

Specific examples are antisense polynucleotides comprising the entire or part of base sequence complementary or substantially complementary to a base sequence of DNA comprising the base sequence represented by SEQ ID NO: 12, SEQ ID NO: 13, SEQ ID NO: 14, SEQ ID NO: 15, SEQ ID NO: 17 or SEQ ID NO: 18, etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. These antisense polynucleotides may be synthesized using a publicly known DNA synthesizer, etc.

According to the present invention, the antisense polynucleotide that can inhibit the replication or expression of a gene encoding the polypeptide of the present invention can be designed and synthesized based on the cloned or determined base sequence information of the DNA encoding the polypeptide. Such a polynucleotide can hybridize to RNA of a gene encoding the polypeptide of the present invention and inhibit RNA synthesis or the function of RNA, or can regulate/control the expression of a gene encoding the polypeptide of the present invention via interaction with RNAs associated with the polypeptide of the present invention. Polynucleotides complementary to the specified sequences of RNA associated with the polypeptide of the present invention and polynucleotides that can specifically hybridize to RNA associated with the polypeptide of the present invention are useful for regulating/controlling the expression of a gene encoding the polypeptide of the present invention in vivo and in vitro. These polynucleotides are also useful for the treatment or diagnosis of diseases, etc.

The term "corresponding" is used to mean homologous or complementary to a particular sequence of the nucleotide including the gene, base sequence or nucleic acid. The term "corresponding" between nucleotides, base sequences or nucleic acids and polypeptides usually refer to amino acids of a polypeptide under instructions derived from the sequence of nucleotides (nucleic acids) or their complements. The 5' end hairpin loop, 5' end 6-base-pair repeats, 5' end untranslated region, polypeptide translation initiation codon, polypeptide coding region, translation initiation codon, 3' untranslated region, 3' end palindrome region, and 3' end hairpin loop encoding the polypeptide can be selected as preferred target regions, though any other region can be selected as a target in the genes encoding the polypeptide.

The relationship between the targeted nucleic acid and the polynucleotide complementary to at least a part of the target region can be denoted to be "antisense." Examples of the antisense polynucleotide include a polynucleotide containing 2-deoxy-D-ribose, a polynucleotide containing D-ribose, any other type of polynucleotide which is N-glycoside of a purine or pyrimidine base, or other polymers having non-nucleotide backbones or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense polynucleotide of the present invention can be modified preferably based on the following design, that is, by (1) increasing the intracellular stability of the antisense polynucleotide, (2) enhancing the cell permeability of the antisense polynucleotide, (3) increasing the affinity of the nucleic acid to the targeted sense strand to a higher level, or (4) minimizing the toxicity, if any, of the antisense polynucleotide.

Most of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense polynucleotide of the present invention may contain altered or modified sugars, bases or linkages, may be provided in a specialized form such as liposomes, microspheres, or may be applied to gene therapy, or may be provided in combination with attached moieties. Such attached moieties used include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol, and the like.

The inhibitory activity of the antisense polynucleotide can be investigated using the transformant of the present invention, the in vivo or in vitro gene expression system of the present invention, or the in vivo or in vitro translation system of the polypeptide of the present invention. The nucleic acid can be applied to cells by various methods publicly known.

Hereinafter, (a) the polypeptide of the present invention, its partial peptide, its amide or its ester, or a salt thereof (hereinafter sometimes merely referred to as the polypeptide of the present invention), (b) the compound or its salt that promotes or inhibits the activity of the polypeptide of the present invention, (c) the antibody against the polypeptide of the present invention, (d) the polynucleotide of the present invention, (e) the antisense polynucleotide of the present invention, etc. are described in terms of their applications.

Polypeptide A of the present invention, its partial peptide, its amide or its ester, or a salt thereof (hereinafter sometimes simply referred to as polypeptide A of the present invention) possesses the vasopressin release inhibiting activity. Thus, polypeptide A of the present invention, the compound or its salt that promotes the activity of polypeptide A of the present invention, the polynucleotide comprising the polynucleotide encoding polypeptide A of the present invention or its partial peptide, etc. can be used, for example, as vasopressin release inhibitors, etc., such as agents for preventing/treating, e.g., renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc.

Moreover, the compound or a its salt that inhibits the activity of polypeptide A of the present invention, the antibody against polypeptide A of the present invention, the antisense polynucleotide of a gene encoding polypeptide A of the present invention, siRNA or shRNA for a gene encoding polypeptide A of the present invention, the ribozyme comprising a part of an RNA encoding polypeptide A of the present invention, the aptamer to polypeptide A of the present invention, etc. can be used, for example, as agents for preventing/treating urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, and the like.

Polypeptide B of the present invention, its partial peptide, its amide or its ester, or a salt thereof (hereinafter sometimes briefly referred to as polypeptide B of the present invention) possesses the vasopressin release inhibiting activity and eating stimulating activity. Thus, polypeptide B of the present invention, the compound or its salt that promotes the activity of polypeptide B of the present invention, the polynucleotide comprising the polynucleotide encoding polypeptide B of the present invention or its partial peptide, etc. can be used, for example, as vasopressin release inhibitors, etc., including, e.g., agents for preventing/treating renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc., agents for preventing/treating anorexia, eating (appetite) stimulants, etc., agents for preventing/treating sleeping disorders [e.g., primary insomnia, circadian rhythm disorders (e.g., change in physical conditions caused by three-shift work, time zone change syndrome (jet lag), etc.)], and the like.

The compound or its salt that inhibits the activity of polypeptide B of the present invention, the antibody against polypeptide B of the present invention, the antisense polynucleotide of a gene encoding polypeptide B of the present invention, siRNA or shRNA for a gene encoding polypeptide B of the present invention, the ribozyme comprising a part of an RNA encoding polypeptide B of the present invention, the aptamer to polypeptide B of the present invention, etc. can be used, for example, as agents for preventing/treating urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc., agents for preventing/treating obesity (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), hyperphagia, etc., agents for preventing/treating sleeping disorders [e.g., primary insomnia, circadian rhythm disorders (e.g., change in physical conditions caused by three-shift work, time zone change syndrome (jet lag), etc.)], and the like.

Furthermore, the polypeptide of the present invention is useful as a reagent for screening a compound that promotes or inhibits the activity of the polypeptide of the present invention.

Moreover, the polynucleotide of the present invention is useful as a reagent for screening a compound that promotes or inhibits the expression of the polypeptide gene of the present invention.

### (1) Medicament comprising the polypeptide of the present invention, its partial peptide, its amide or its ester, or a salt thereof

Polypeptide A of the present invention, its partial peptide, its amide or its ester, or a salt thereof possesses the vasopressin secretion inhibiting activity. Thus, polypeptide A of the present invention, its partial peptide, its amide or its ester, or salts thereof can be used, for example, as vasopressin release inhibitors, etc., such as agents for preventing/treating, e.g., renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc.

Polypeptide B of the present invention, its partial peptide, its amide or its ester, or a salt thereof possesses the vasopressin release inhibiting activity and eating stimulating activity. Thus, polypeptide B of the present invention, its partial peptide, its amide or its ester, or a salt thereof can be used, for example, as vasopressin release inhibitors, etc., including agents for preventing/treating renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc., agents for preventing/treating anorexia, eating (appetite) stimulants, etc., agents for preventing/treating sleeping disorders [e.g., primary insomnia, circadian rhythm disorders (e.g., change in physical conditions caused by three-shift work, time zone change syndrome (jet lag), etc.)], and the like.

As salts of the polypeptide of the present invention, its partial peptide, its amide or its ester, for example, pharmaceutically acceptable salts are used. Examples include salts with inorganic bases, salts with organic bases, salts with inorganic acids, salts with organic salts, salts with basic or acidic amino acids, etc.

Preferred examples of the salts with inorganic bases include alkali metal salts such as sodium salts, potassium salts, etc.; alkaline earth metal salts such as calcium salts, magnesium salts, etc.; aluminum salts, ammonium salts, and the like.

Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc.

Preferred examples of the salts with inorganic acids include salts with hydrochloric acid, hydrobromic acid, sulfuric acid, phosphoric acid, etc.

Preferred examples of the salts with organic acids include salts with formic acid, acetic acid, propionic acid, fumaric acid, oxalic acid, tartaric acid, maleic acid, citric acid, succinic acid, malic acid, methanesulfonic acid, benzenesulfonic acid, benzoic acid, etc.

Suitable examples of the salts with basic amino acids include salts with arginine, lysine, ornithine, etc. Preferred examples of the salts with acidic amino acids include salts with aspartic acid, glutamic acid, etc.

When the polypeptide of the present invention, its partial peptide, its amide or its ester, or a salt thereof is used as the aforesaid medicament (preventive/therapeutic agent, etc.), the polypeptide or the like can be prepared into pharmaceutical preparations by publicly known methods, including the methods described above. Specifically, they can be used as described below.

The polypeptide of the present invention can be administered orally, for example, in the form of tablets which may be sugar coated, if necessary, capsules, elixirs, microcapsules etc., or parenterally in the form of injections such as sterile solutions or suspensions in water or in pharmaceutically acceptable solutions other than water. For example, the polypeptide of the present invention can be mixed with carriers, flavoring agents, excipients, vehicles, preservatives, stabilizers, binders, etc. in a unit dosage form generally accepted. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, a flavoring agent such as peppermint, akamono oil and cherry, etc. When the unit dosage is in the form of a capsule, liquid carriers such as oils and fats may further be used together with the additives described above. A sterile composition for injection may be formulated in a conventional manner used to make pharmaceutical preparations, e.g., by dissolving or suspending the active ingredients in a vehicle such as water for injection with a naturally occurring vegetable oil such as sesame oil and coconut oil, etc. to prepare the pharmaceutical preparations.

Examples of an aqueous medium for injection include physiological saline and an isotonic solution containing glucose and other auxiliary agents (e.g., D-sorbitol, D-mannitol, sodium chloride, etc.), or the like and may be used in combination with an appropriate dissolution aid such as an alcohol (e.g., ethanol, etc.), a polyalcohol (e.g., propylene glycol and polyethylene glycol, etc.), a nonionic surfactant (e.g., polysorbate 80^{™}, HCO-50, etc.), or the like. Examples of the oily medium include sesame oil, soybean oil, etc., which may also be used in combination with a dissolution aid such as benzyl benzoate, benzyl alcohol, etc.

The polypeptide may further be formulated together with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the pharmaceutical preparation thus obtained is safe and low toxic, it can be administered to warm-blooded mammal (e.g., human, guinea pig, rat, mouse, swine, sheep, bovine, monkey, dog or fowl, etc.).

The dose of polypeptide A of the present invention, its partial peptide, its amide or its ester, or a salt thereof may vary depending upon conditions, etc. In the case of oral administration, polypeptide A or the like is administered to a patient (as 60 kg body weight) with, e.g., renal edema generally in a dose of about 0.1 to 1000 mg, preferably about 1.0 to 300 mg and more preferably about 3.0 to 50 mg, per day. In parenteral administration, its dose may vary depending upon subject to be administered, conditions, route of administration, etc. When it is administered to a patient (as 60 kg body weight) with, e.g., renal edema in the form of, e.g., an injectable preparation, it is generally advantageous to administer intravenously in a dose of about 0.01 to about 30 mg, preferably about 0.1 to about 20 mg and more preferably about 0.1 to about 10 mg, per day. For other animal species, the corresponding dose as converted per 60 kg weight can be administered.

The dose of polypeptide B of the present invention, its partial peptide, its amide or its ester, or a salt thereof may vary depending upon conditions, etc. In the case of oral administration, polypeptide B or the like is administered to a patient (as 60 kg body weight) with, e.g., renal edema generally in a dose of about 0.1 to 1000 mg, preferably about 1.0 to 300 mg and more preferably about 3.0 to 50 mg, per day. In parenteral administration, its dose may vary depending upon subject to be administered, conditions, route of administration, etc. When it is administered to a patient (as 60 kg body weight) with, e.g., renal edema in the form of, e.g., it is generally advantageous to administer intravenously in a dose of about 0.01 to about 30 mg, preferably about 0.1 to about 20 mg and more preferably about 0.1 to about 10 mg, per day. For other animal species, the corresponding dose as converted per 60 kg weight can be administered.

### (2) Medicament comprising the compound or its salt that promotes or inhibits the activity of the polypeptide of the present invention, its partial peptide, its amide or its ester, or a salt thereof

Polypeptide A of the present invention, its partial peptide, its amide or its ester, or a salt thereof possesses the vasopressin release inhibiting activity. Thus, the compound or its salt that promotes the activity of polypeptide A of the present invention, its partial peptide, its amide or its ester, or salts thereof can be used, for example, as vasopressin release inhibitors, etc., such as agents for preventing/treating, e.g., renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc.

The compound or its salt that inhibits the activity of polypeptide A of the present invention, its partial peptide, its amide or its ester, or a salt thereof is useful as an agent for preventing/treating, e.g. urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc.

Polypeptide B of the present invention, its partial peptide, its amide or its ester, or a salt thereof possesses the vasopressin release inhibiting activity and eating stimulating activity. Thus, the compound or its salt that promotes the activity of polypeptide B of the present invention, its partial peptide, its amide or its ester, or a salt thereof can be used, for example, as vasopressin release inhibitors, etc., including, e.g., agents for preventing/treating renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc., agents for preventing/treating anorexia, eating (appetite) stimulants, etc., agents for preventing/treating sleeping disorders [e.g., primary insomnia, circadian rhythm disorders (e.g., change in physical conditions caused by three-shift work, time zone change syndrome (jet lag), etc.)], and the like.

The compound or its salt that inhibits the activity of polypeptide B of the present invention, its partial peptide, its amide or its ester, or a salt thereof is useful, for example, as an agent for preventing/treating urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc., an agent for preventing/treating obesity (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), hyperphagia, etc., an agent for preventing/treating sleeping disorders [e.g., primary insomnia, circadian rhythm disorders (e.g., change in physical conditions caused by three-shift work, time zone change syndrome (jet lag), etc.)], and the like.

As salts of the compound that promotes or inhibits the activity of the polypeptide of the present invention, its partial peptide, its amide or its ester, or a salt thereof, for example, pharmaceutically acceptable salts are used. Examples include salts with inorganic bases, salts with organic bases, salts with inorganic acids, salts with organic acids, salts with basic or acidic amino acids, etc.

Preferred examples of the salts with inorganic bases include alkali metal salts such as sodium salts, potassium salts, etc.; alkaline earth metal salts such as calcium salts, magnesium salts, etc.; aluminum salts, ammonium salts, and the like.

Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc.

Preferred examples of the salts with inorganic acids include salts with hydrochloric acid, hydrobromic acid, sulfuric acid, phosphoric acid, etc.

Preferred examples of the salts with organic acids include salts with formic acid, acetic acid, propionic acid, fumaric acid, oxalic acid, tartaric acid, maleic acid, citric acid, succinic acid, malic acid, methanesulfonic acid, benzenesulfonic acid, benzoic acid, etc.

Suitable examples of the salts with basic amino acids include salts with arginine, lysine, ornithine, etc. Preferred examples of the salts with acidic amino acids include salts with aspartic acid, glutamic acid, etc.

When the compound or its salt that promotes the activity of the polypeptide of the present invention, its partial peptide, its amide or its ester, or a salt thereof and the compound or its salt that inhibits the activity of the polypeptide of the present invention, its partial peptide, its amide or its ester, or a salt thereof are used as the aforesaid medicaments, the use can be performed in a conventional manner. Specifically, these compounds can be used as described below.

For example, these compounds can be administered orally as tablets coated with sugar or with enteric coating if necessary, capsules, elixirs, microcapsules, etc., or parenterally in the form of injections such as sterile solutions or suspensions in water or in pharmaceutically acceptable solutions other than water. For example, the compound or its salts can be mixed with carriers, flavoring agents, excipients, vehicles, preservatives, stabilizers, binders, etc. in a unit dosage form required for generally accepted pharmaceutical practice to prepare pharmaceutical preparations. The amount of active ingredients in these preparations is adjusted so as to obtain appropriate doses within specified ranges.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of a capsule, liquid carriers such as oils and fats may further be used together with the additives described above. A sterile composition for injection may be formulated in a conventional manner used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc) and may be used in combination with an appropriate dissolution aid such as an alcohol (e.g., ethanol, etc.), a polyalcohol (e.g., propylene glycol, polyethylene glycol, etc.), a nonionic surfactant (e.g., polysorbate 80^{™}, HCO-50, etc.), or the like. Examples of the oily medium include sesame oil, soybean oil, etc., which may also be used in combination with a dissolution aid such as benzyl benzoate, benzyl alcohol, etc.

These preparations may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The thus obtained pharmaceutical preparation is safe and low toxic, and thus can be administered to warm-blooded mammal (e.g., human, guinea pig, rat, mouse, swine, sheep, bovine, monkey, dog or fowl), etc.

The dose of the compound or its salt that promotes the activity of polypeptide A of the present invention, its partial peptide, its amide or its ester, or a salt thereof may vary depending upon conditions, etc. For example, in the case of oral administration, it is administered to a patient (as 60 kg body weight) with, e.g., renal edema generally in a dose of about 0.1 to 1000 mg, preferably about 1.0 to 300 mg and more preferably about 3.0 to 50 mg, per day. In parenteral administration, the dose may vary depending upon subject to be administered, conditions, route of administration, etc. When it is administered to a patient (as 60 kg body weight) with, e.g., renal edema in the form of, e.g., an injectable preparation, it is generally advantageous to inject intravenously in a dose of about 0.01 to about 30 mg, preferably about 0.1 to about 20 mg and more preferably about 0.1 to about 10 mg, per day. For other animal species, the corresponding dose as converted per 60 kg weight can be administered.

The dose of the compound or its salt that inhibits the activity of polypeptide A of the present invention, its partial peptide, its amide or its ester, or a salt thereof may vary depending upon conditions, etc. In the case of oral administration, it is administered to a patient (as 60 kg body weight) with, e.g., diabetes insipidus generally in a dose of about 0.1 to 1000 mg, preferably about 1.0 to 300 mg and more preferably about 3.0 to 50 mg, per day. In parenteral administration, the dose may vary depending upon subject to be administered, conditions, route of administration, etc. When it is administered to a patient (as 60 kg body weight) with, e.g., diabetes insipidus in the form of, e.g., an injectable preparation, it is generally advantageous to inject intravenously in a dose of about 0.01 to about 30 mg, preferably about 0.1 to about 20 mg and more preferably about 0.1 to about 10 mg, per day. For other animal species, the corresponding dose as converted per 60 kg weight can be administered.

The dose of the compound or its salt that promotes the activity of polypeptide B of the present invention, its partial peptide, its amide or its ester, or a salt thereof may vary depending upon conditions, etc. In the case of oral administration, it is administered to a patient (as 60 kg body weight) with, e.g., renal edema generally in a dose of about 0.1 to 1000 mg, preferably about 1.0 to 300 mg and more preferably about 3.0 to 50 mg, per day. In parenteral administration, its dose may vary depending upon subject to be administered, conditions, route of administration, etc. When it is administered to a patient (as 60 kg body weight) with, e.g., renal edema in the form of, e.g., an injectable preparation, it is generally advantageous to inject intravenously in a dose of about 0.01 to about 30 mg, preferably about 0.1 to about 20 mg and more preferably about 0.1 to about 10 mg, per day. For other animal species, the corresponding dose as converted per 60 kg weight can be administered.

The dose of the compound or its salt that inhibits the activity of polypeptide B of the present invention, its partial peptide, its amide or its ester, or a salt thereof may vary depending upon conditions, etc. In the case of oral administration, it is administered to a patient (as 60 kg body weight) with, e.g., diabetes insipidus generally in a dose of about 0.1 to 1000 mg, preferably about 1.0 to 300 mg and more preferably about 3.0 to 50 mg, per day. In parenteral administration, its dose may vary depending upon subject to be administered, conditions, route of administration, etc. When it is administered to a patient (as 60 kg body weight) with, e.g., diabetes insipidus in the form of, e.g., an injectable preparation, it is generally advantageous to inject intravenously in a dose of about 0.01 to about 30 mg, preferably about 0.1 to about 20 mg and more preferably about 0.1 to about 10 mg, per day. For other animal species, the corresponding dose as converted per 60 kg weight can be administered.

### (3) Medicament comprising the antibody against the polypeptide of the present invention, its partial peptide, its amide or its ester, or a salt thereof

Polypeptide A of the present invention, its partial peptide, its amide or its ester, or a salt thereof possesses the vasopressin release inhibiting activity. Thus, the antibody against polypeptide A of the present invention, its partial peptide, its amide or its ester, or a salt thereof is useful as an agent for preventing/treating, e.g., urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc.

Polypeptide B of the present invention, its partial peptide, its amide or its ester, or a salt thereof possesses the vasopressin release inhibiting activity and eating stimulating activity. Thus, the antibody against polypeptide B of the present invention, its partial peptide, its amide or its ester, or a salt thereof is useful, for example, as an agent for preventing/treating urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc., an agent for preventing/treating obesity (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), hyperphagia, etc., an agent for preventing/treating sleeping disorders [e.g., primary insomnia, circadian rhythm disorders (e.g., change in physical conditions caused by three-shift work, time zone change syndrome (jet lag), etc.)], and the like.

When the antibody against the polypeptide of the present invention, its partial peptide, its amide or its ester, or a salt thereof is used as the aforesaid - medicament, the use can be performed in a conventional manner. Specifically, the polypeptide of the present invention can be used as described below.

The antibody can be administered orally, for example, in the form of tablets which may be sugar coated, if necessary, capsules, elixirs, microcapsules etc., or parenterally in the form of injections such as sterile solutions or suspensions in water or in pharmaceutically acceptable solutions other than water. For example, the compound or its salt can be mixed with carriers, flavoring agents, excipients, vehicles, preservatives, stabilizers, binders, etc. in a unit dosage form generally accepted. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of a capsule, liquid carriers such as oils and fats may further be used together with the additives described above. A sterile composition for injection may be formulated in a conventional manner used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.), etc. and may be used in combination with an appropriate dissolution aid such as an alcohol (e.g., ethanol, etc.), a polyalcohol (e.g., propylene glycol and polyethylene glycol, etc.), a nonionic surfactant (e.g., polysorbate 80^{™}, HCO-50, etc.), etc. Examples of the oily medium include sesame oil, soybean oil, etc., which may also be used in combination with a dissolution aid such as benzyl benzoate, benzyl alcohol, etc.

These preparations may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The thus obtained pharmaceutical preparation is safe and low toxic, and thus can be administered to warm-blooded mammal (e.g., human, guinea pig, rat, mouse, swine, sheep, bovine, monkey, dog or fowl), etc.

The dose of the antibody against polypeptide A of the present invention, its partial peptide, its amide or its ester, or a salt thereof may vary depending upon conditions, etc. In the case of oral administration, it is administered to a patient (as 60 kg body weight) with, e.g., diabetes insipidus generally in a daily dose of about 0.1 to 1000 mg, preferably about 1.0 to 300 mg and more preferably about 3.0 to 50 mg. In parenteral administration, its dose may vary depending upon subject to be administered, conditions, route of administration, etc. When it is administered to a patient (as 60 kg body weight) with, e.g., diabetes insipidus in the form of, e.g., an injectable preparation, it is generally advantageous to administer intravenously in a dose of about 0.01 to about 30 mg, preferably about 0.1 to about 20 mg and more preferably about 0.1 to about 10 mg, per day. For other animal species, the corresponding dose as converted per 60 kg weight can be administered.

The dose of the antibody against polypeptide B of the present invention, its partial peptide, its amide or its ester, or a salt thereof may vary depending upon conditions, etc. In the case of oral administration, it is administered to a patient (as 60 kg body weight) with, e.g., diabetes insipidus generally in a dose of about 0.1 to 1000 mg, preferably about 1.0 to 300 mg and more preferably about 3.0 to 50 mg, per day. In parenteral administration, its dose may vary depending upon subject to be administered, conditions, route of administration, etc. When it is administered to a patient (as 60 kg body weight) with, e.g., diabetes insipidus in the form of, e.g., an injectable preparation, it is generally advantageous to administer intravenously in a dose of about 0.01 to about 30 mg, preferably about 0.1 to about 20 mg and more preferably about 0.1 to about 10 mg, per day. For other animal species, the corresponding dose as converted per 60 kg weight can be administered.

### (4) Medicament comprising the polynucleotide of the present invention

Polypeptide A of the present invention, its partial peptide, its amide or its ester, or a salt thereof possesses the vasopressin release inhibiting activity. Thus, the polynucleotide comprising the polynucleotide encoding polypeptide A of the present invention or its partial peptide can be used, for example, as vasopressin release inhibitors, etc., such as agents for preventing/treating, e.g., renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc.

Polypeptide B of the present invention, its partial peptide, its amide or its ester, or a salt thereof possesses the vasopressin release inhibiting activity and eating stimulating activity. Thus, the polynucleotide comprising the polynucleotide encoding polypeptide B of the present invention or its partial peptide can be used, for example, as vasopressin release inhibitors, etc., including agents for preventing/treating renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc., agents for preventing/treating anorexia, eating (appetite) stimulants, etc., agents for preventing/treating sleeping disorders [e.g., primary insomnia, circadian rhythm disorders (e.g., change in physical conditions caused by three-shift work, time zone change syndrome (jet lag), etc.)], and the like.

Where the polynucleotide of the present invention is used as the preventive/therapeutic agent described above, the polynucleotide can be prepared into pharmaceutical preparations by publicly known methods, and the preparations can be provided for administration.

For instance, the polynucleotide may be administered alone; or after the polynucleotide is inserted into an appropriate vector such as retrovirus vector, adenovirus vector, adenovirus-associated virus vector, etc., the product can be administered to human or other warm-blooded animal in a conventional manner. The polynucleotide of the present invention may also be administered as it is, or with adjuvants to assist its uptake by gene gun or through a catheter such as a catheter with a hydrogel.

The dose of the polynucleotide comprising the polynucleotide encoding polypeptide A of the present invention or its partial peptide may vary depending upon conditions, etc. but generally is about 0.1 to 100 mg per day for the patient (as 60 kg body weight) with, e.g., renal edema.

The dose of the polynucleotide comprising the polynucleotide encoding polypeptide B of the present invention or its partial peptide may vary depending upon conditions, etc. but generally is about 0.1 to 100 mg per day for the patient (as 60 kg body weight) with, e.g., renal edema.

### (5) Medicament and diagnostic agent comprising the antisense polynucleotide, double-strand RNA, ribozyme, etc. of the present invention

The antisense polynucleotide of the present invention which can bind complementarily to the DNA encoding polypeptide A of the present invention (hereinafter referred to as "DNA(A) of the present invention") to suppress the expression of the DNA is low toxic and can suppress the function of polypeptide A of the present invention or DNA(A) of the present invention in vivo. Thus, the antisense polynucleotide can be used as an agent for preventing/treating, for example, urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc.

The antisense polynucleotide of the present invention which can bind complementarily to the DNA encoding polypeptide B of the present invention (hereinafter referred to as "DNA(B) of the present invention") to suppress the expression of the DNA is low toxic and can suppress the function of polypeptide B of the present invention or DNA(B) of the present invention in vivo. Thus, the antisense polynucleotide can be used, for example, as an agent for preventing/treating urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc., an agent for preventing/treating obesity (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), hyperphagia, etc., an agent for preventing/treating sleeping disorders [e.g., primary insomnia, circadian rhythm disorders (e.g., change in physical conditions caused by three-shift work, time zone change syndrome (jet lag), etc.)], and the like.

Where the antisense polynucleotide of the present invention is used as a preventive/therapeutic agent described above, it can be prepared into pharmaceutical preparations by publicly known methods, and the preparations can be provided for administration.

For example, when the antisense polynucleotide described above is used, the antisense polynucleotide alone is administered directly, or the antisense polynucleotide is inserted into an appropriate vector such as retrovirus vector, adenovirus vector, adenovirus-associated virus vector, etc., followed by treating in a conventional manner. The antisense polynucleotide may then be administered orally or parenterally to human or a mammal (e.g., rat, rabbit, sheep, swine, bovine, cat, dog, monkey, etc.) in a conventional manner. The antisense polynucleotide may also be administered as it stands, or may be prepared in pharmaceutical preparations together with a physiologically acceptable carrier to assist its uptake, which are then administered by gene gun or through a catheter such as a catheter with a hydrogel.

The dose of the antisense polynucleotide may vary depending on target disease, subject to be administered, route for administration, etc. Where the antisense polynucleotide of the present invention is administered for the purpose of treating, e.g., diabetes insipidus, the antisense polynucleotide is generally administered to an adult (60 kg body weight) in a daily dose of about 0.1 to 100 mg.

In addition, the antisense polynucleotide may also be used as an oligonucleotide probe for diagnosis to examine the presence of the DNA of the present invention in tissues or cells and states of its expression.

The present invention further provides the following features:
(1) a double-stranded RNA comprising a part of RNA encoding polypeptide A of the present invention;
(2) a medicament comprising the double-stranded RNA according to (1);
(3) a ribozyme comprising a part of RNA encoding polypeptide A of the present invention;
(4) a medicament comprising the ribozyme according to (3);
(5) a double-stranded RNA comprising a part of RNA encoding polypeptide B of the present invention;
(6) a medicament comprising the double-stranded RNA according to (5);
(7) a ribozyme comprising a part of RNA encoding polypeptide B of the present invention;
(8) a medicament comprising the ribozyme according to (7);

These double-stranded RNAs (RNAi; RNA interference method), ribozymes, etc. can suppress the expression of the polynucleotide (e.g., DNA) of the present invention in the same way as in the antisense polynucleotide of the present invention, and can inhibit the activity or function of the polypeptide of the present invention or the polynucleotide (e.g., DNA) of the present invention.

The double-stranded RNA described in (1) above and the ribozyme described in (3) above can be used as agents for preventing/treating, e.g., urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc.

The double-stranded RNA described in (5) above and the ribozyme described in (7) above can be used, for example, as agents for preventing/treating urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc., agents for preventing/treating obesity (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), hyperphagia, etc., agents for preventing/treating sleeping disorders [e.g., primary insomnia, circadian rhythm disorders (e.g., change in physical conditions caused by three-shift work, time zone change syndrome (jet lag), etc.)], and the like.

The double-stranded RNA can be designed based on the sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the peptide of the present invention. A part of the RNA encoding the peptide of the present invention includes a portion proximal to a cleavage site on the RNA of the present invention, which may be cleaved by a publicly known ribozyme (RNA fragment).

Where the double-stranded RNA or ribozyme described above is used as the preventive/therapeutic agents described above, the double-stranded RNA or ribozyme is prepared into a pharmaceutical preparation in the same way as in the antisense polynucleotide, and the preparation can be provided for administration.

As in the antisense polynucleotide described above, the aptamers against the polypeptide of the present invention, etc. can also suppress the activity or function of the protein used in the present invention. Thus, the aptamers against polypeptide A of the present invention, etc. can be used as medicaments such as agents for preventing/treating, for example, urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc.

The aptamers against polypeptide B of the present invention, etc. can be used as medicaments, for example, as agents for preventing/treating urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc., agents for preventing/treating obesity (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), hyperphagia, etc., agents for preventing/treating sleeping disorders [e.g., primary insomnia, circadian rhythm disorders (e.g., change in physical conditions caused by three-shift work, time zone change syndrome (jet lag), etc.)], and the like.

The aptamers are prepared by using publicly known methods, for example, the SELEX (systematic evolution of ligands by exponential enrichment) method (Annual Review of Medicine, 56, 555-583, 2005). Structures of the aptamers can be determined by using publicly known methods, and based on the structures, the aptamers are prepared according to publicly known methods.

Where the aptamer described above is used as a medicament, the aptamer is prepared into a pharmaceutical preparation in the same way as in the antisense polynucleotide, and the preparation can be provided for administration.

### (6) Screening method and screening kit for drug candidate compound

The method of screening drug candidate compounds, which comprises using the polypeptide of the present invention and the kit for screening drug candidate compounds comprising the polypeptide of the present invention are described below (hereinafter sometimes briefly referred to as the "screening method of the present invention" and the "screening kit of the present invention," respectively). [Method for screening a compound or its salt that promotes or inhibits the activity of the polypeptide of the present invention]

The polypeptide of the present invention is useful as a reagent for screening a compound or its salt that promotes or inhibits the function of the polypeptide of the present invention. That is, the present invention provides (1) a method of screening a compound or its salt that promotes or inhibits the activity (function) of the polypeptide of the present invention (hereinafter sometimes briefly referred to as a promoter or an inhibitor), which comprises using the polypeptide of the present invention. In particular, the present invention provides, for example: (2) a method for screening a promoter or an inhibitor, which comprises comparing the activity of the polypeptide of the present invention such as the vasopressin release inhibiting activity of cells, (i) in the case where the polypeptide of the present invention is brought in contact with the cells and (ii) in the case where the polypeptide of the present invention and a test compound are brought in contact with the cells. Specifically, in the screening method described above, for example, the cells are incubated in the cases (i) and (ii) and the amount of vasopressin released is measured. As the cells described above, the cells inducing vasopressin secretion are preferably used. For example, rat supraoptic nucleus tissue (e.g., static incubation of rat supraoptic nucleus described in the EXAMPLE below, etc.), rat paraventricular nucleus tissue (e.g., static incubation of rat paraventricular nucleus described in the EXAMPLE below, etc.), rat supraoptic nucleus neuron, rat paraventricular nucleus neuron, etc. are used. Any medium may be employed, so long as they do not interfere with the activities possessed by the polypeptide of the present invention, such as the vasopressin release inhibiting activity, etc., and DMEM (Dulbecco modified Eagle's medium) or the like is used, for example. The amount of vasopressin released is measured by publicly known methods, e.g., radioimmunoassay using an AVP RIA (radioimmunoassay) kit (manufactured by Mitsubishi Kagaku Iatron, Inc., etc.). Examples of the test compound include peptides, proteins, antibodies, non-peptide compounds, synthetic compounds, fermentation products, cell extracts, plant extracts, animal tissue extracts, blood plasma, and the like. These compounds may be novel or known compounds. The test compounds may form salts and as salts of the test compounds, there are, for example, metal salts, ammonium salts, salts with organic bases, salts with inorganic acids, salts with organic acids, salts with basic or acidic amino acids, etc. Preferred examples of the metal salts include alkali metal salts such as sodium salts, potassium salts, etc.; alkaline earth meal salts such as calcium salts, magnesium salts, barium salts, etc.; aluminum salts, etc. Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Preferred examples of the salts with inorganic acids include salts with hydrochloric acid, hydrobromic acid, nitric acid, sulfuric acid, phosphoric acid, etc. Preferred examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferred examples of the salts with basic amino acids include salts with arginine, lysine, ornithine, etc., and preferred examples of the salts with acidic amino acids include salts with aspartic acid, glutamic acid, etc.

For example, when a test compound promotes the activity of the polypeptide of the present invention (e.g., vasopressin release inhibiting activity) in the case (ii) described above by at least about 20%, preferably at least 30% and more preferably at least about 50%, as compared to the case (i) above, the test compound can be selected to be the compound or its salt that promotes the activity of the polypeptide of the present invention. For example, when a test compound inhibits the activity of the polypeptide of the present invention (e.g., the vasopressin release inhibiting activity) in the case (ii) described above by at least about 20%, preferably at least 30% and more preferably at least about 50%, as compared to the case (i) above, the test compound can be selected to be the compound or its salt that inhibits the activity of the polypeptide of the present invention.

The polynucleotide of the present invention is useful as a reagent for screening a compound or its salt that promotes or inhibits the expression of a gene for the polypeptide of the present invention. The present invention provides (3) a method of screening a compound or its salt that promotes or inhibits the expression of a gene for the polypeptide of the present invention (hereinafter sometimes briefly referred to as a promoter or an inhibitor, respectively), which comprises using the polynucleotide of the present invention. In particular, the present invention provides, for example: (4) a method of screening a promoter or an inhibitor, which comprises comparing (iii) the case where cells capable of producing the polypeptide of the present invention are cultured and (iv) the case where a mixture of cells capable of producing the polypeptide of the present invention and a test compound is cultured. In the screening method described above, for example, the expression level of the gene for the polypeptide of the present invention (e.g., the activities of enzymes such as alkaline phosphatase, luciferase, etc. inserted in the downstream of a promoter of the gene for the polypeptide of the present invention, the level of mRNA encoding the polypeptide of the present invention, etc.) is measured in the cases (iii) and (iv) and comparison is made therebetween. As a cell capable of producing the polypeptide of the present invention, there is employed, e.g., the aforesaid host (transformant) transformed with a vector containing a DNA encoding the polypeptide of the present invention. Preferably, animal cells such as CHO cells, etc. are employed as the host. For the screening, the transformant, in which the polypeptide of the present invention is expressed on the cell membrane, e.g., by culturing through the procedure described above, is preferably employed. More preferably, there is used a transformant into which a gene such as secreted alkaline phosphatase, luciferase, etc. is inserted at the downstream of a promoter of the gene for the polypeptide of the present invention, etc. Examples of the test compound include peptides, proteins, antibodies, non-peptide compounds, synthetic compounds, fermentation products, cell extracts, plant extracts, animal tissue extracts, blood plasma, and the like. These compounds may be novel or known compounds. The test compounds may form salts and as salts of the test compounds, the same examples as given hereinabove apply. To perform the screening method described above, the cell capable of producing the polypeptide of the present invention is cultured in an appropriate medium for screening and prepared. Any medium may be employed, so long as they do not interfere with the production of the polypeptide of the present invention, and DMEM medium, etc. is used, for example. The expression level of the gene for the polypeptide of the present invention can be determined by assaying the activity of an enzyme such as alkaline phosphatase, luciferase or the like, according to publicly known methods, which enzyme is inserted into the downstream of the promoter in the gene for the polypeptide of the present invention. The expression level of the gene for the polypeptide of the present invention can be determined by publicly known methods, e.g., in accordance with methods such as Northern blotting, reverse transcription-polymerase chain reaction (RT-PCR), real time PCR monitoring system (manufactured by ABI, TaqMan polymerase chain reaction), etc., or their modifications. For example, when a test compound that promotes the expression of the gene for the polypeptide of the present invention in the case (iv) described above by at least about 20%, preferably at least 30% and more preferably at least about 50%, as compared to the case (iii) above, the test compound can be selected to be the compound or its salt that promotes the expression of the gene for the polypeptide of the present invention. For example, when a test compound inhibits the expression of the gene for the polypeptide of the present invention in the case (iv) described above by at least about 20%, preferably at least 30% and more preferably at least about 50%, as compared to the case (iii) above, the test compound can be selected to be the compound or its salt that inhibits expression of the gene for the polypeptide of the present invention.

The polynucleotide of the present invention is useful as a reagent for screening a compound or its salt that promotes or inhibits the activity of the polypeptide of the present invention. That is, the present invention provides (5) a method of screening a compound or its salt that promotes or inhibits the activity of the polypeptide of the present invention (hereinafter sometimes briefly referred to as a promoter or an inhibitor, respectively), which comprises using the polynucleotide of the present invention. In particular, the present invention provides, for example: (6) a method of screening a promoter or an inhibitor of the activity possessed by the polypeptide of the present invention, which comprises (v) culturing cells capable of producing the polypeptide of the present invention in contact with other cells and (vi) culturing a mixture of cells capable of producing the polypeptide of the present invention and a test compound in contact with other cells, and comparing the activity (e.g., the vasopressin release inhibiting activity) of the polypeptide of the present invention in said other cells between the cases (v) and (vi). Specifically, in the screening method described above, for example, the cells (and tissues) are incubated in the cases (v) and (vi) and the amount of vasopressin released is measured. As the cells capable of producing the polypeptide of the present invention, the cells described in (4) above are used. As other cells, the cells described in (2) above are used. Test compounds, culturing methods, methods for measuring the vasopressin release inhibiting activity, etc. are as described in (2) above with modifications. For example, when a test compound promotes the activity of the polypeptide of the present invention (e.g., the vasopressin release inhibiting activity) in the case (vi) described above by at least about 20%, preferably at least 30% and more preferably at least about 50%, as compared to the case (v) above, the test compound can be selected to be the compound or its salt that promotes the activity of the polypeptide of the present invention. For example, when a test compound inhibits the activity of the polypeptide of the present invention in the case (vi) described above by at least about 20%, preferably at least 30% and more preferably at least about 50%, as compared to the case (v) above, the test compound can be selected to be the compound or its salt that inhibits the activity of the polypeptide of the present invention.

The present invention provides (7) a method of screening a compound or its salt that promotes or inhibits the expression (production) of the polypeptide of the present invention (hereinafter sometimes briefly referred to as a promoter or an inhibitor, respectively), which comprises using the antibody of the present invention. In particular, the present invention provides, for example: (8) a method of screening a promoter or an inhibitor, which comprises (vii) culturing cells capable of producing the polypeptide of the present invention and (viii) culturing a mixture of cells capable of producing the polypeptide of the present invention and a test compound and comparing the cases (vii) and (viii), using the antibody of the present invention. In the screening method described above, for example, the production amount of the polypeptide of the present invention (specifically, the amount of the polypeptide of the present invention) is measured and compared in the cases (vii) and (viii) using the antibody of the present invention.

To perform the screening method described above, the cell capable of producing the polypeptide of the present invention is cultured in an appropriate medium for screening and prepared. Any medium may be employed, so long as they do not interfere with the production of the polypeptide of the present invention, for example, DMEM medium, etc. is used. As the cell capable of producing the polypeptide of the present invention, there is employed, e.g., the aforesaid host (transformant) transformed with a vector containing a DNA encoding the polypeptide of the present invention. Preferably, animal cells such as CHO cells, etc. are used as the host. For the screening, the transformant, in which the polypeptide of the present invention is expressed on the cell membrane, e.g., by culturing through the procedure described above, is preferably employed. The polypeptide of the present invention can be determined by publicly known methods, e.g., by assaying the above-described polypeptide present in the cell extract, etc., using the antibody recognizing the polypeptide of the present invention, in accordance with methods like western blot analysis, ELISA, etc., or modifications thereof. For example, when a test compound promotes the production amount (expression level) of the polypeptide of the present invention in the case (viii) described above by at least about 20%, preferably at least 30% and more preferably at least about 50%, as compared to the case (vii) above, the test compound can be selected as the compound or its salt that promotes the expression of the polypeptide of the present invention. For example, when a test compound inhibits the production amount (expression level) of the polypeptide of the present invention in the case (viii) described above by at least about 20%, preferably at least 30% and more preferably at least about 50%, as compared to the case (vii) above, the test compound can be selected to be the compound or its salt that inhibits the expression of the polypeptide of the present invention. The screening kit of the present invention comprises the polypeptide of the present invention or its salt.

Examples of the test compounds using in screening described above include, for example, peptides, proteins, non-peptide compounds, synthetic compounds, fermentation products, cell extracts, plant extracts, animal tissue extracts, blood plasma, and the like, and these compounds may be novel compounds or publicly known compounds. The test compounds may form salts and as salts of the test compounds, the same examples as given hereinabove apply.

### (7) The medicament comprising the compound or its salt obtained by using the screening method or screening kit of the present invention

Examples of the compounds or salts thereof, which are obtained using the screening method or screening kit of the present invention, are compounds selected from, e.g., peptides, proteins, antibodies, non-peptide compounds, synthetic compounds, fermentation products, cell extracts, plant extracts, animal tissue extracts, blood plasma, and the like, and are the compounds or salts thereof that promote or inhibit the activity of the polypeptide of the present invention, the compounds or salts thereof that promote or inhibit the expression of a gene for the polypeptide of the present invention and the compounds or salts thereof that promote or inhibit the expression of the polypeptide of the present invention. For salts of these compounds, the same salts as those given for the polypeptide of the present invention above may be used. The compound or its salt that promotes the activity of polypeptide A of the present invention, the compound or its salt that promotes the expression of a gene for polypeptide A of the present invention and the compound or its salt that promotes the expression of polypeptide A of the present invention, which are obtainable using the screening method or screening kit of the present invention, can be used as safe and low-toxic agents, for example, as vasopressin release inhibitors, etc., such as agents for preventing/treating, e.g., renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc. On the other hand, the compound or its salt that inhibits the activity of polypeptide A of the present invention, the compound or its salt that inhibits the expression of a gene for polypeptide A of the present invention and the compound or its salt that inhibits the expression of polypeptide A of the present invention can be used as medicaments such as agents for preventing/treating, e.g., urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc.

The compound or its salt that promotes the activity of polypeptide B of the present invention, the compound or its salt that promotes the expression of a gene for polypeptide B of the present invention and the compound or its salt that promotes the expression of polypeptide B of the present invention can be used as safe and low-toxic agents, for example, as vasopressin release inhibitors, including agents for preventing/treating renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc., agents for preventing/treating anorexia, eating (appetite) stimulants, etc., agents for preventing/treating sleeping disorders [e.g., primary insomnia, circadian rhythm disorders (e.g., change in physical conditions caused by three-shift work, time zone change syndrome (jet lag), etc.)], and the like. On the other hand, the compound or its salt that inhibits the activity of polypeptide B of the present invention, the compound or its salt that inhibits the expression of a gene for polypeptide B of the present invention and the compound or its salt that inhibits the expression of polypeptide B of the present invention can be used as medicaments including, e.g., agents for preventing/treating urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc., agents for preventing/treating obesity (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), hyperphagia, etc., agents for preventing/treating sleeping disorders [e.g., primary insomnia, circadian rhythm disorders (e.g., change in physical conditions caused by three-shift work, time zone change syndrome (jet lag), etc.)], and the like.

The compound or its salt obtainable using the screening method or screening kit of the present invention is used as the therapeutic/prophylactic agent described above, which is performed in a conventional manner. The compound may be prepared in the form of tablets, capsules, elixirs, microcapsules, sterile solutions, suspensions, etc., for example, in a manner similar to the aforesaid medicament comprising the polypeptide of the present invention. Since the thus obtained pharmaceutical preparation is safe and low toxic, the preparation can be administered to, for example, mammal (e.g., human, mouse, rat, rabbit, sheep, swine, bovine, horse, cat, dog, monkey, etc.).

The doses of the compound or its salt that promotes the activity of polypeptide A of the present invention, the compound or its salt that promotes the expression of a gene for polypeptide A of the present invention, and the compound or its salt that promotes the expression of polypeptide A of the present invention may vary depending on its action, target disease, subject to be administered, route for administration, etc. In the case of oral administration, the compound is administered to a patient (as 60 kg body weight) with, e.g., renal edema normally in a dose of about 0.1 to about 100 mg, preferably about 1.0 to about 50 mg, more preferably about 1.0 to about 20 mg, per day. In the case of parenteral administration, it is generally advantageous to administer intravenously to a patient (as 60 kg body weight) with, e.g., renal edema in the form of, e.g., an injectable preparation in a dose of about 0.01 to about 30 mg, preferably about 0.1 to about 20 mg, more preferably about 0.1 to about 10 mg, per day. For other animal species, the corresponding dose as converted per 60 kg weight can be administered.

The doses of the compound or its salt that inhibits the activity of polypeptide A of the present invention, the compound or its salt that inhibits the expression of a gene for polypeptide A of the present invention, and the compound or its salt that inhibits the expression of polypeptide A of the present invention may vary depending on its action, target disease, subject to be administered, route for administration, etc. For example, in the case of oral administration, the compound is administered to a patient (as 60 kg body weight) with, e.g., diabetes insipidus normally in a dose of about 0.1 to about 100 mg, preferably about 1.0 to about 50 mg, more preferably about 1.0 to about 20 mg, per day. When the compound is parenterally administered to a patient (as 60 kg body weight) with, e.g., diabetes insipidus in the form of, e.g., an injectable preparation, it is generally advantageous to administer the compound intravenously at a daily dose of about 0.01 to about 30 mg, preferably about 0.1 to about 20 mg, more preferably about 0.1 to about 10 mg. For other animal species, the corresponding dose as converted per 60 kg weight can be administered.

The doses of the compound or its salt that promotes the activity of polypeptide B of the present invention, the compound or its salt that promotes the expression of a gene for polypeptide B of the present invention, and the compound or its salt that promotes the expression of polypeptide B of the present invention may vary depending on its action, target disease, subject to be administered, route for administration, etc. For example, in oral administration, the compound is administered to a patient (as 60 kg body weight) with, e.g., renal edema normally in a dose of about 0.1 to about 100 mg, preferably about 1.0 to about 50 mg, more preferably about 1.0 to about 20 mg, per day. When the compound is parenterally administered to a patient (as 60 kg body weight) with, e.g., renal edema in the form of, e.g., an injectable preparation, it is generally advantageous to administer intravenously at a daily dose of about 0.01 to about 30 mg, preferably about 0.1 to about 20 mg, more preferably about 0.1 to about 10 mg. For other animal species, the corresponding dose as converted per 60 kg weight can be administered.

The doses of the compound or its salt that inhibits the activity of polypeptide B of the present invention, the compound or its salt that inhibits the expression of a gene for polypeptide B of the present invention, and the compound or its salt that inhibits the expression of polypeptide B of the present invention may vary depending on its action, target disease, subject to be administered, route for administration, etc. For example, in the case of oral administration, the compound is administered to a patient (as 60 kg body weight) with, e.g., diabetes insipidus normally in a dose of about 0.1 to about 100 mg, preferably about 1.0 to about 50 mg, more preferably about 1.0 to about 20 mg, per day. When the compound is parenterally administered to a patient (as 60 kg body weight) with, e.g., diabetes insipidus in the form of, e.g., an injectable preparation, it is generally advantageous to administer intravenously at a daily dose of about 0.01 to about 30 mg, preferably about 0.1 to about 20 mg, more preferably about 0.1 to about 10 mg. For other animal species, the corresponding dose as converted per 60 kg weight can be administered.

### (8) Method for diagnosis using the antibody of the present invention

The antibody against the polypeptide of the present invention (hereinafter sometimes referred to as the "antibody of the present invention") can specifically recognize the polypeptide of the present invention. Therefore, the antibody can be used for detection or neutralization of the polypeptide of the present invention in a test fluid.

That is, the present invention provides, for example, the following methods of quantification:
(i) a method of quantifying the polypeptide of the present invention in a test fluid, which comprises competitively reacting the antibody of the present invention with the test fluid and a labeled form of the polypeptide of the present invention, and measuring the ratio of the labeled polypeptide of the present invention bound to the antibody; and,
(ii) a method of quantifying the polypeptide of the present invention in a test fluid, which comprises reacting the test fluid with the antibody of the present invention immobilized on a carrier and a labeled form of another antibody of the present invention simultaneously or sequentially, and measuring the activity of the label on the immobilized carrier.

In the quantifying method (ii) described above, it is preferred that one antibody is capable of recognizing the N-terminal region in the polypeptide of the present invention, whereas another antibody is capable of reacting with the C-terminal region in the polypeptide of the present invention.

Using a monoclonal antibody against the polypeptide of the present invention, the polypeptide of the present invention can be assayed and can further be detected by tissue staining, or the like. For these purposes, the antibody molecule itself may be used, or F(ab')₂, Fab' or Fab fractions of the antibody molecule may be used as well.

The quantification methods of the polypeptide of the present invention using the antibodies of the present invention are not particularly limited. Any quantification method can be used, so long as the amount of an antibody, antigen, or antibody-antigen complex corresponding to the amount of antigen (e.g., the amount of the polypeptide of the present invention) in a test fluid can be detected by chemical or physical means and the amount of the antigen can be calculated from a standard curve prepared from standard solutions containing known amounts of the antigen. For example, the nephrometry, competitive method, immunometric method and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [¹²⁵I], [¹³¹I], [³H], [¹⁴C], etc. are used. As the enzyme described above, stable enzymes with a high specific activity are preferred; for example, β-galactosidase, β-glucosidase, alkaline phosphatase, peroxidase, malate dehydrogenase , etc. are used. Examples of the fluorescent substance used are fluorescamine and fluorescein isothiocyanate are used. For the luminescent substance, there are used, for example, luminol, luminol derivatives, luciferin, and lucigenin. Furthermore, the biotin-avidin system may be used for binding an antibody or antigen to the labeling agent.

For immobilization of an antigen or antibody, physical adsorption may be used. Chemical binding methods conventionally used for insolubilization or immobilization of the polypeptide of the present invention, enzymes, etc. may also be used. For the carrier, for example, insoluble polysaccharides such as agarose, dextran, cellulose, etc.; synthetic resin such as polystyrene, polyacrylamide, silicon, etc., glass, and the like are used.

In the sandwich method, the monoclonal antibody of the present invention which is immobilized is reacted with a test fluid (primary reaction), then with a labeled form of the monoclonal antibody of the present invention (secondary reaction), and the activity of the label on the immobilizing carrier is measured, whereby the amount of the polypeptide of the present invention in the test fluid can be quantified. The order of the primary and secondary reactions may be reversed, and the reactions may be performed simultaneously or with an interval. The labeling agent and the method for immobilization can be performed by some modifications of those described above. Further in the immunoassay using the sandwich method, the antibody used as an antibody for immobilizationed or as an antibody for labeling is not always limited to a single type, and a mixture of two or more types of antibodies can be used for the purpose of improving the measurement sensitivity.

In the methods of assaying the polypeptide of the present invention by the sandwich method according to the present invention, antibodies that bind to different sites of the polypeptide of the present invention are preferably used as the monoclonal antibodies of the present invention for the primary and secondary reactions. That is, in the antibodies used for the primary and secondary reactions are, for example, when the antibody used in the secondary reaction recognizes the C-terminal region of the polypeptide of the present invention, it is preferable to use the antibody recognizing the region other than the C-terminal region for the primary reaction, e.g., the antibody recognizing the N-terminal region.

The monoclonal antibody of the present invention can be used for other assay systems than the sandwich method, for example, for competitive method, immunometric method, nephrometry, or the like.

In the competitive method, an antigen in a test fluid and a labeled antigen are competitively reacted with an antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, etc., and an immobilized method either using an immobilized antibody as the primary antibody, or using a soluble antibody as the primary antibody and immobilized antibody as the secondary antibody.

In the immunometric method, an antigen in a test fluid and an immobilized antigen are competitively reacted with a given amount of labeled antibody, the solid phase is separated from the liquid phase, or an antigen in a test fluid and an excess amount of labeled antibody are reacted, the immobilized antigen is then added to bind the unreacted labeled antibody to the solid phase, and the solid phase is separated from the liquid phase. Then, the amount of the label in either phase is measured to quantify the antigen in the test fluid.

In the nephrometry, the insoluble precipitate produced after the antigen-antibody reaction in gel or solution is quantified. When the amount of an antigen in the test fluid is small and only a small amount of the precipitate is obtained, laser nephrometry using scattering of laser is advantageously employed.

For applying these individual immunological methods to the quantification methods of the present invention, any particular conditions or procedures are not required. Systems for measuring the polypeptide of the present invention are constructed by adding the usual technical consideration in the art to the conventional conditions and procedures. For the details of these general technical means, reference can be made to the following reviews and texts.

Reference can be made to, e.g., Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As above, the polypeptide of the present invention can be quantified with good sensitivity, by using the antibody of the present invention.

Furthermore, when a change of level of the polypeptide of the present invention is detected by quantifying the level of the polypeptide of the present invention using the antibody of the present invention, it can be diagnosed that one suffers from the aforesaid diseases associated with the polypeptide of the present invention, or it is highly likely that one would suffer from these diseases in the future.

### (9) Production of a DNA transgenic animal of the present invention

The present invention provides a non-human mammal bearing the DNA of the present invention which is exogenous (hereinafter briefly referred to as the exogenous DNA of the present invention) or its variant DNA (sometimes briefly referred to as the exogenous variant DNA of the present invention).

That is, the present invention provides:
[1] a non-human mammal bearing the exogenous DNA of the present invention or its variant DNA;
[2] the mammal according to [1], wherein the non-human mammal is a rodent;
[3] the mammal according to [2], wherein the rodent is mouse or rat; and,
[4] a recombinant vector containing the exogenous DNA of the present invention or its variant DNA and capable of expressing in a mammal.

The non-human mammal bearing the exogenous DNA of the present invention or its variant DNA (hereinafter briefly referred to as the DNA transgenic animal of the present invention) can be produced by transferring a desired DNA into an unfertilized egg, a fertilized egg, a spermatozoon, a germinal cell containing a primordial germinal cell thereof, etc., preferably in the embryogenic stage in the development of a non-human mammal (more preferably in the single cell or fertilized cell stage and generally before the 8-cell phase), by means, such as the calcium phosphate method, the electric pulse method, the lipofection method, the agglutination method, the microinjection method, the particle gun method, the DEAE-dextran method, etc. Also, it is possible to transfer the exogenous DNA of the present invention into a somatic cell, a living organ, a tissue cell, etc. by the DNA transfer, and utilize the transformant for cell culture, tissue culture, etc. In addition, these cells may be fused with the above-described germinal cell by a publicly known cell fusion method to prepare the DNA transgenic animal of the present invention.

Examples of the non-human mammals that can be used include bovine, swine, sheep, goat, rabbits, dogs, cats, guinea pigs, hamsters, mice, rats, etc. Above all, preferred are rodents, especially mice (e.g., C57BL/6 strain, DBA2 strain, etc. for a pure line and for a cross line, B6C3F₁ strain, BDF₁ strain B6D2F₁ strain, BALB/c strain, ICR strain, etc.), rats (Wistar, SD, etc.) or the like, since they are relatively short in ontogeny and life cycle from a standpoint of producing model animals for human disease.

"Mammal" in a recombinant vector that can be expressed in the mammal includes the aforesaid non-human mammal and human, etc.

The exogenous DNA of the present invention refers to the DNA of the present invention that is once isolated/extracted from a mammal, not the DNA of the present invention inherently possessed by a non-human mammal.

The mutant DNA of the present invention includes mutants resulting from variation (e.g., mutation, etc.) in the base sequence of the original DNA of the present invention, specifically DNAs resulting from base addition, deletion, substitution with other bases, etc. and further includes abnormal DNA.

The abnormal DNA is intended to mean the DNA that expresses the abnormal polypeptide of the present invention and exemplified by such a DNA that expresses a polypeptide capable of suppressing the functions of the normal polypeptide of the present invention.

The exogenous DNA of the present invention may be any one of those derived from a mammal of the same species as, or a different species from, the mammal as the target animal. In transfecting the DNA of the present invention, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., a vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the polypeptide of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide elongation factor 1α (EF-1α) promoters, human and fowl β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus, and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal polypeptide of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using complementary DNA prepared by a publicly known method from RNA of liver, kidney, thyroid cell or fibroblast origin as a starting material. Also, an exogenous abnormal DNA can be obtained using complementary DNA prepared by a publicly known method from RNA of human fibroblast origin as a starting material. Alternatively, the translational region for a normal polypeptide translational region obtained by the cell or tissue described above can be made variant by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transferred at the fertilized egg cell stage in a manner such that the DNA is certainly present in all the germinal cells and somatic cells of the target mammal. The fact that the exogenous DNA of the present invention is present in the germinal cells of the animal prepared by DNA transfer means that all offspring of the prepared animal will maintain the exogenous DNA of the present invention in all of the germinal cells and somatic cells thereof the offspring of the animal that inherits the exogenous DNA of the present invention also have the exogenous DNA of the present invention in all of the germinal cells and somatic cells thereof.

The non-human mammal in which the normal exogenous DNA of the present invention has been transferred can be passaged as the DNA-bearing animal under ordinary rearing environment, by confirming that the exogenous DNA is stably retained by crossing.

By transfer of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfer means that the DNA of the present invention is excessively present in all of the germinal cells and somatic cells thereof the offspring of the animal that inherits the exogenous DNA of the present invention have excessively the DNA of the present invention in all of the germinal cells and somatic cells thereof.

It is possible to obtain homozygous animals having the transferred DNA in both homologous chromosomes and breed male and female of the animal so that all the progeny have this DNA in excess.

In a non-human mammal bearing the normal DNA of the present invention, the normal DNA of the present invention is abundantly expressed, and may eventually develop hyperfunction of the polypeptide of the present invention by promoting the functions of endogenous normal DNA. Therefore, the animal can be utilized as a pathologic model animal for such a disease. Specifically, using the normal DNA transgenic animal of the present invention, it becomes possible to elucidate the hyperfunction by the polypeptide of the present invention and to clarify the pathological mechanism of the disease associated with the polypeptide of the present invention and to determine how to treat the disease. A mammal transfected with the normal exogenous DNA of the present invention has a symptom of increasing the polypeptide of the present invention and is thus available also for tests of screening therapeutic agents for diseases associated with the polypeptide of the present invention.

On the other hand, non-human mammal having the exogenous abnormal DNA of the present invention can be passaged under normal breeding conditions as the DNA-bearing animal by confirming the stable retaining of the exogenous DNA via crossing. In addition, the objective exogenous DNA can be utilized as a starting material by inserting the DNA into the plasmid described above. The DNA construct with a promoter can be prepared using conventional DNA engineering techniques. The transfection of the abnormal DNA of the present invention at the fertilized egg cell stage is preserved to be present in all of the germinal and somatic cells of the mammals to be subjected. The fact that the abnormal DNA of the present invention is present in the germinal cells of the animal after DNA transfection means that all of the offspring of the prepared animal have the abnormal DNA of the present invention in all of the germinal and somatic cells. Such an offspring passaged the exogenous DNA of the present invention contains the abnormal DNA of the present invention in all of the germinal and somatic cells. A homozygous animal having the introduced DNA on both of homologous chromosomes can be acquired and then by mating these male and female animals, all the offspring can be bred to have the DNA.

Since the non-human mammal having the abnormal DNA of the present invention expresses the abnormal DNA of the present invention at a high level, the animal may cause the function inactive type inadaptability of the polypeptide of the present invention by inhibiting the functions of the endogenous normal DNA, and can be utilized as its disease model animal. For example, using the abnormal DNA-transferred animal of the present invention, it is possible to elucidate the mechanism of the function inactive type inadaptability of the polypeptide of the present invention and to study a method for treatment of this disease.

More specifically, the transgenic animal of the present invention expressing the abnormal DNA of the present invention to a high level is also expected to serve as an experimental model for the elucidation of the mechanism of the functional inhibition (dominant negative effect) of a normal polypeptide by the abnormal polypeptide of the present invention in the function inactive type inadaptability of the polypeptide of the present invention.

A mammal transfected with the abnormal exogenous DNA of the present invention has a symptom of the function inactive type inadaptability of the polypeptide of the present invention, and is thus available also for screening test of an agent for preventing/treating the function inactive type inadaptability of the polypeptide of the present invention.

Other potential applications of two kinds of the transgenic animals described above include:
(i) use as a cell source for tissue culture;
(ii) elucidation of the association with a polypeptide that is specifically expressed or activated by the polypeptide of the present invention, through direct analysis of DNA or RNA in tissue of the DNA transgenic animal of the present invention or by analysis of the polypeptide tissue expressed by the DNA;
(iii) research in the function of cells derived from tissues that are cultured usually only with difficulty, using cells of tissue bearing the DNA cultured by a standard tissue culture technique;
(iv) screening for a drug that enhances the functions of cells using the cells described in 3) above; and,
(v) isolation and purification of the variant polypeptide of the present invention and preparation of an antibody thereto.

Furthermore, clinical conditions of a disease associated with the polypeptide of the present invention, including the function inactive type inadaptability of the polypeptide of the present invention can be determined using the DNA transgenic animal of the present invention. Also, pathological findings on each organ in a disease model associated with the polypeptide of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve as identification of cells capable of producing the polypeptide of the present invention, and as studies on association with apoptosis, differentiation or propagation or on the mechanism of signal transduction in these properties to inspect any abnormality therein. Thus the DNA transgenic animal can provide an effective research material for the polypeptide of the present invention and for elucidating the function and effect thereof.

To develop pharmaceuticals for the treatment of diseases associated with the polypeptide of the present invention, including the function inactive type inadaptability of the polypeptide of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the polypeptide of the present invention, using the DNA transgenic animal of the present invention or a vector capable of expressing the exogenous DNA of the present invention.

### (10) Knockout animal

The present invention provides a non-human mammal embryonic stem cell bearing the DNA of the present invention inactivated and a non-human mammal deficient in expressing the DNA of the present invention.

Thus, the present invention provides:
1) a non-human embryonic stem cell in which the DNA of the present invention is inactivated;
2) the embryonic stem cell according to 1), wherein the DNA is inactivated by introducing a reporter gene (e.g., β-galactosidase gene derived from *Escherichia coli*);
3) the embryonic stem cell according to 1), which is resistant to neomycin;
4) the embryonic stem cell according to 1), wherein the non-human mammal is a rodent;
5) the embryonic stem cell according to 4), wherein the rodent is mouse;
6) a non-human mammal deficient in expressing the DNA of the present invention, wherein the DNA is inactivated;
7) the non-human mammal according to 6), wherein the DNA is inactivated by inserting a reporter gene (e.g., β-galactosidase derived from *Escherichia coli*) therein and the reporter gene is capable of being expressed under control of a promoter for the DNA of the present invention;
8) the non-human mammal according to 6), which is a rodent;
9) the non-human mammal according to 8), wherein the rodent is mouse; and,
10) a method of screening a compound or its salt that promotes or inhibits the promoter activity to the DNA of the present invention, which comprises administering a test compound to the mammal according to 7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the polypeptide of the present invention (hereinafter sometimes referred to as the knockout DNA of the present invention) by substantially inactivating the activities of the polypeptide of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the subject animal by, e.g., homologous recombination, a DNA sequence which terminates gene transcription (e.g., polyA additional signal, etc.) in the intron between exons to, thus inhibit the synthesis of complete messenger RNA and eventually destroy the gene (hereinafter simply referred to as targeting vector). The thus-obtained ES cells to the Southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may be originally established in accordance with a modification of the known method by Evans and Kaufman *supra.* For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera and are therefore preferred. It is desirable to identify sexes as soon as possible also in order to save painstaking culture time.

Methods for sex identification of the ES cell include the method in which a gene in the sex-determining region on the Y-chromosome is amplified by the PCR process and detected. When this method is used, one colony ofES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Second selection can be achieved by, for example, number of chromosome confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is rendered knockout.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence ofLIF (1-10000 U/ml) on appropriate feeder cells such as STO fibroblasts, treated with a trypsin/EDTA solution (normally about 0.001 to about 0.5% trypsin/about 0.1 to about 5 mM EDTA, preferably about 0.1% trypsin/1 mM EDTA) at the time of passage to obtain separate single cells, which are then seeded on freshly prepared feeder cells. This passage is normally conducted every 1 to 3 days; it is desirable that cells be monitored at passage and cells found to be morphologically abnormal in culture, if any, be abandoned.

By allowing ES cells to reach a high density in mono-layers or to form cell aggregates in suspension under appropriate conditions, it is possible to spontaneously differentiate them to various cell types, for example, pariental and visceral muscles, cardiac muscle, or the like [M. J. Evans and M. H. Kaufman, Nature, 292, 154, 1981; G R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtainable from the differentiated ES cells of the present invention, are useful for studying the functions of the polypeptide of the present invention cytologically or molecular biologically.

The non-human mammal deficient in expression of the DNA of the present invention can be identified from a normal animal by measuring the amount of mRNA in the subject animal by a publicly known method, and indirectly comparing the degrees of expression.

As the non-human mammal, the same examples as described above apply.

With respect to the non-human mammal deficient in expression of the DNA of the present invention, the DNA of the present invention can be made knockout by transfecting a targeting vector, prepared as described above, to non-human mammal embryonic stem cells or oocytes thereof, and conducting homologous recombination in which a targeting vector DNA sequence, wherein the DNA of the present invention is inactivated by the transfection, is replaced with the DNA of the present invention on a chromosome of a non-human mammal embryonic stem cell or embryo thereof.

The knockout cells with the DNA of the present invention disrupted can be identified by Southern hybridization analysis with a DNA fragment on or near the DNA of the present invention as a probe, or by PCR analysis using a DNA sequence on the targeting vector and another DNA sequence which is not included in the targeting vector as primers. When non-human mammalian embryonic stem cells are used, a cell line wherein the DNA of the present invention is inactivated by homologous recombination is cloned; the resulting cloned cell line is injected to, e.g., a non-human mammalian embryo or blastocyte, at an appropriate stage such as the 8-cell stage. The resulting chimeric embryos are transplanted to the uterus of the pseudopregnant non-human mammal. The resulting animal is a chimeric animal composed of both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the polypeptide of the present invention. The individuals deficient in homozygous expression of the polypeptide of the present invention can be obtained from offspring of the intercross between the heterozygotes.

When an oocyte or egg cell is used, a DNA solution may be injected, e.g., to the prenucleus by microinjection thereby to obtain a transgenic non-human mammal having a targeting vector introduced in a chromosome thereof From such transgenic non-human mammals, those having a mutation at the locus of the DNA of the present invention can be obtained by selection based on homologous recombination.

As described above, individuals in which the DNA of the present invention is rendered knockout permit passage rearing under ordinary rearing conditions, after the individuals obtained by their crossing have proven to have been knockout.

Furthermore, the genital system may be obtained and maintained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell in which the DNA of the present invention is inactivated is very useful for preparing a non-human mammal deficient in expression of the DNA of the present invention.

Since the non-human mammal in which the DNA of the present invention is inactivated lacks various biological activities derived from the polypeptide of the present invention, such an animal can be a disease model suspected of inactivated biological activities of the polypeptide of the present invention and thus, offers an effective study to investigate causes for and therapy for these diseases.

### (a) Method of screening a compound having therapeutic/prophylactic effects on diseases caused by deficiency, damages, etc. of the DNA of the present invention

The non-human mammal deficient in expression of the DNA of the present invention can be employed for the screening of compounds having therapeutic/prophylactic effects for diseases caused by deficiency, damages, etc. of the DNA of the present invention.

That is, the present invention provides a method for screening of a compound or its salt having therapeutic/preventive effects on diseases caused by deficiency, damages, etc. of the DNA of the present invention, which comprises administering a test compound to the non-human mammal deficient in expression of the DNA of the present invention and monitoring/measuring a change occurred in the animal.

As the non-human mammal deficient in expression of the DNA of the present invention, which can be employed for the screening method, the same examples as given hereinabove apply.

Examples of the test compounds include peptides, proteins, antibodies, non-peptide compounds, synthetic compounds, fermentation products, cell extracts, plant extracts, animal tissue extracts, blood plasma, and the like and these compounds may be novel compounds or publicly known compounds. The test compounds may form salts and salts of the test compounds used are the same salts as those of the test compounds used in the screening method, etc. for the drug candidate compounds described above.

Specifically, the non-human mammal deficient in the expression of the DNA of the present invention is treated with a test compound, comparison is made with an intact animal for control and changes in each organ, tissue, disease conditions, etc. of the animal is used as an indicator to assess therapeutic/prophylactic effects of the test compound.

For treating an animal to be tested with a test compound, for example, oral administration, intravenous injection, etc. are applied and the treatment is appropriately selected depending upon conditions of the test animal, properties of the test compound, etc. Furthermore, an amount of a test compound administered can be selected depending on administration route, property of the test compound, and the like.

The compound obtained using the screening method above is a compound selected from the test compounds described above and exhibits therapeutic/preventive effects on the diseases caused by deficiencies, damages, etc. of the polypeptide of the present invention. Therefore, the compound can be used as a safe and low toxic agent for the treatment/prevention of these diseases. Furthermore, compounds derived from said compound obtained by the screening described above can be used as well.

The test compound obtained by the screening method above may form salts and salts of the compound used are the same salts as those given for the test compound described above.

A medicament comprising the compound or its salts obtained by the above screening method may be manufactured in the same way as in the manufacturing of the medicament comprising the polypeptide of the present invention described above.

The pharmaceutical preparation thus obtained is safe and low toxic, and can be administered to human or other mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound is orally administered, the compound is generally administered to an adult patient (as 60 kg body weight) with, e.g., renal edema at a daily dose of about 0.1 to 100 mg, preferably about 1.0 to 50 mg, more preferably about 1.0 to 20 mg. For parenteral administration, the dose of the compound may vary depending upon subject to be administered, target disease, etc. When the compound is administered to an adult patient (as 60 kg body weight) with, e.g., renal edema in the form of, e.g., an injectable preparation, it is advantageous to administer the compound intravenously at a daily dose of about 0.01 to 30 mg, preferably about 0.1 to 20 mg, more preferably about 0.1 to 10 mg. For other animal species, the corresponding dose as converted per 60 kg weight can be administered.

### (b) Method for screening a compound that promotes or inhibits the activity of a promoter to the DNA of the present invention

The present invention provides the method for screening a compound or its salt that promotes or inhibits the activities of a promoter to the DNA of the present invention, which comprises administering a test compound to a non-human mammal deficient in expression of the DNA of the present invention and detecting expression of the reporter gene.

In the screening method described above, the non-human mammal deficient in expression of the DNA of the present invention is selected from the aforesaid non-human mammal deficient in expression of the DNA of the present invention, as an animal in which the DNA of the present invention is inactivated by introducing a reporter gene and the reporter gene can be expressed under control of a promoter to the DNA of the present invention.

The same examples of the test compound apply to those given above. The test compound may form salts and salts of the test compound used are the same salts as those of the test compounds used in the screening method of drug candidate compounds described above.

As the reporter gene, the same specific examples described above apply, and β-galactosidase (lacZ), soluble alkaline phosphatase gene, luciferase gene and the like are preferably employed.

Since a reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the polypeptide of the present invention is substituted with, e.g., β-galactosidase gene (lacZ) derived from *Escherichia coli,* β-galactosidase is expressed in a tissue where the polypeptide of the present invention should originally be expressed, instead of the polypeptide of the present invention. Thus, the state of expression of the polypeptide of the present invention can be readily monitored in vivo of an animal by staining with a reagent, e.g., 5-bromo-4-chloro-3-indolyl-β-galactopyranoside (X-gal), which is substrate for β-galactosidase. Specifically, a mouse deficient in the polypeptide of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is monitored. Alternatively, mRNA encoding lacZ may be detected in a conventional manner.

The compound or its salt obtained using the screening method above is a compound, which is selected from the test compounds described above and promotes or inhibits the promoter activity to the DNA of the present invention.

The compound obtained by the screening method above may form salts and salts of the test compound used are the same salts as those given for the test compound described above.

The compound or its salt that promotes the promoter activity to the DNA of the present invention can promote the expression of the polypeptide of the present invention to promote the function of said polypeptide, and is thus useful as a medicament, for example, as a vasopressin release inhibitor, etc., such as an agent for preventing/treating renal edema, dysuria (e.g., bladder contraction dysfunction, urinary tract obstruction, urinary disturbances, urodynia, urinary obstruction, etc.), hyponatremia, syndrome of inappropriate secretion of antidiuretic hormone (SIADH), hypertension, etc., an agent for preventing/treating anorexia, eating (appetite) stimulants, etc., an agent for preventing/treating sleeping disorders [e.g., primary insomnia, circadian rhythm disorders (e.g., change in physical conditions caused by three-shift work, time zone change syndrome (jet lag), etc.)], and the like.

Furthermore, the compound or its salt that inhibits the promoter activity to the DNA of the present invention can inhibit the expression of the polypeptide of the present invention to inhibit the function of said polypeptide, and is thus useful, for example, as a low-toxic and safe medicament such as an agent for preventing/treating urine storage disorders [e.g., pollakiuria, urinary incontinence (e.g., urge urinary incontinence, stress urinary incontinence, functional urinary incontinence, etc.), etc.], polyuria, diabetes insipidus (e.g., pituitary diabetes insipidus, nephrogenic diabetes insipidus, etc.), hypernatremia, metabolic alkalosis, hypokalemia, Cushing syndrome, etc., an agent for preventing/treating obesity (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), hyperphagia, etc., an agent for preventing/treating sleeping disorders [e.g., primary insomnia, circadian rhythm disorders (e.g., change in physical conditions caused by three-shift work, time zone change syndrome (jet lag), etc.)], and the like

In addition, any compound derived from the compounds obtained by the screening above may be employed as well.

The medicament comprising the compound or its salt obtained by the screening method described above may be manufactured in a manner similar to the method for preparing the medicament comprising the polypeptide of the present invention or its salt described above.

The pharmaceutical preparation thus obtained is safe and low toxic, and can be administered to human or other mammal (e.g., rat, mouse, guinea pig, rabbit, sheep, swine, bovine, horse, cat, dog, monkey, etc.).

The dose of the compound or its salt may vary depending on target disease, subject to be administered, route for administration, etc. For example, when the compound that promotes the promoter activity to the DNA of the present invention is orally administered, the dose is normally about 0.1 to about 100 mg, preferably about 1.0 to about 50 mg, more preferably about 1.0 to about 20 mg, per day, for an adult patient (as 60 kg body weight) with, e.g., renal edema. In parenteral administration, the dose of the compound may vary depending on subject to be administered, target disease, etc. but when the compound that promotes the promoter activity to the DNA of the present invention is administered to an adult patient (as 60 kg body weight) with, e.g., renal edema in the form of, e.g., an injectable preparation, it is advantageous to administer the compound intravenously at a daily dose of about 0.01 to about 30 mg, preferably about 0.1 to about 20 mg and more preferably about 0.1 to about 10 mg. For other animal species, the corresponding dose as converted per 60 kg weight can be administered.

On the other hand, when the compound that inhibits the promoter activity to the DNA of the present invention is orally administered, the dose is normally about 0.1 to about 100 mg, preferably about 1.0 to about 50 mg, more preferably about 1.0 to about 20 mg, per day, for the adult patient (as 60 kg body weight) with diabetes insipidus. In parenteral administration, the dose of the compound varies depending on subject to be administered, target disease, etc. When the compound that inhibits the promoter activity to the DNA of the present invention is administered in the form of, e.g., an injectable preparation, it is advantageous to administer the compound intravenously to the adult patient (as 60 kg body weight) with diabetes insipidus at a daily dose of about 0.01 to about 30 mg, preferably about 0.1 to about 20 mg, more preferably about 0.1 to about 10 mg. For other animal species, the corresponding dose as converted per 60 kg weight can be administered.

As described above, the non-human mammal deficient in expression of the DNA of the present invention is extremely useful for screening the compound or its salt that promotes or inhibits the activity of a promoter to the DNA of the present invention and can greatly contribute to the elucidation of causes for various diseases suspected of deficiency in expression of the DNA of the present invention and for the development of prophylactic/therapeutic agent for these diseases.

In addition, a so-called transgenic animal (gene transferred animal) can be prepared by using the DNA containing a promoter region of the polypeptide of the present invention, ligating genes encoding various proteins downstream and injecting the same into oocytes of an animal. It is then possible to synthesize the polypeptide therein specifically and study its activity in vivo. When an appropriate reporter gene is ligated to the promoter site above and a cell line that express the gene is established, the resulting system can be utilized as the survey system for a low molecular compound having the action of specifically promoting or inhibiting the in vivo productivity of the polypeptide per se of the present invention.

Throughout the specification and drawings, where bases, amino acids, etc. are shown by their codes, these codes are denoted in accordance with the IUPAC-IUB Commission on Biochemical Nomenclature or by the common codes in the art, examples of which are given below. For amino acids that may have the optical isomer, L form is presented unless otherwise indicated.
- DNA :: deoxyribonucleic acid
- cDNA :: complementary deoxyribonucleic acid
- A :: adenine
- T :: thymine
- G :: guanine
- C :: cytosine
- Y :: thymine or cytosine
- N :: thymine, cytosine, adenine or guanine
- R :: adenine or guanine
- M :: cytosine or adenine
- W :: thymine or adenine
- S :: cytosine or guanine
- RNA :: ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP :: adenosine triphosphate
- EDTA :: ethylenediaminetetraacetic acid
- SDS :: sodium dodecyl sulfate
- TFA :: trifluoroacetic acid
- EIA :: enzyme immunoassay
- Gly or G:: glycine
- Ala or A:: alanine
- Val or V:: valine
- Leu or L:: leucine
- Ile or I:: isoleucine
- Ser or S :: serine
- Thr or T :: threonine
- Cys or C:: cysteine
- Met or M:: methionine
- Glu or E:: glutamic acid
- Asp or D:: aspartic acid
- Lys or K:: lysine
- Arg or R:: arginine
- His or H:: histidine
- Phe or F:: phenylalanine
- Tyr or Y:: tyrosine
- Trp or W:: tryptophan
- Pro or P :: proline
- Asn or N:: asparagine
- Gin or Q:: glutamine
- pGlu or pE:: pyroglutamic acid
- Me :: methyl group
- Et :: ethyl group
- Bu :: butyl group
- Ph :: phenyl group
- TC :: thiazolidine-4(R)-carboxamido group
- Bom :: benzyloxymethyl
- NMP :: N-methylpyrrolidone
- PAM :: phenylacetamidomethyl

Substituents, protecting groups and reagents frequently used in this specification are presented as the codes below.
- Tos :: p-toluenesulfonyl
- HONB :: 1-hydroxy-5-norbornene-2,3-dicarboxyimide
- Bzl :: benzyl
- Z :: :benzyloxycarbonyl
- Br-Z :: 2-bromobenzyl oxycarbonyl
- CI-Z :: 2-chlorobenzyloxycarbonyl
- Boc :: t-butoxycarbonyl
- HOBt :: 1-hydroxybenztriazole
- DCC :: N,N'-dicyclohexylcarbodiimide
- TFA :: trifluoroacetic acid
- Fmoc :: N-9-fluorenyl methoxycarbonyl
- DNP :: dinitrophenol
- Bum :: t-butoxymethyl
- Trt :: trityl
- BSA :: bovine serum albumin
- CHAPS :: 3-[(3-cholamidopropyl)dimethylammonio]-1-propane sulfonate
- PMSF :: phenylmethylsulfonyl fluoride
- E64 :: (L-3-trans-carboxiran-2-carbonyl) L-leucyl-agmatin
- GDP :: guanosine-5'-diphosphate
- MEMα :: Minimum Essential Medium alpha
- Fura-2AM :: pentacetoxymethyl 1-[6-amino-2-(5-carboxy-2-oxazolyl)-5-benzofuranyloxy]-2-(2-amino-5-methylphen oxy)-ethane-N,N,N',N'-tetraacetate
- HBSS :: Hanks' balanced salt
- Fluo-3AM :: pentacetoxymethyl 1-[2-amino-5-(2,7-dichloro-6-hydroxy-3-oxy-9-xanthenyl)phenoxy]-2-(2-amino-5-methylphenoxy)ethane-N,N,N',N'-tetraac etate
- HEPES :: 2-[4-(2-hydroxyethyl)-1-piperazinyl]ethanesulfonic acid
- MeBzl :: 4-methylbenzyl
- NMP :: N-methylpyrrolidone

The sequence identification numbers in the sequence listing of the specification indicate the following sequences.
[SEQ ID NO: 1]
   This shows the amino acid sequence of human VGF.
[SEQ ID NO: 2]
   This shows the amino acid sequence of rat VGF.
[SEQ ID NO: 3]
   This shows the amino acid sequence of mouse VGF.
[SEQ ID NO: 4]
   This shows the amino acid sequence of human NERP-1.
[SEQ ID NO: 5]
   This shows the amino acid sequence of rat NERP-1.
[SEQ ID NO: 6]
   This shows the amino acid sequence of mouse NERP-1.
[SEQ ID NO: 7]
   This shows the amino acid sequence of human NERP-2.
[SEQ ID NO: 8]
   This shows the amino acid sequence of rat NERP-2.
[SEQ ID NO: 9]
   This shows the amino acid sequence of mouse NERP-2.
[SEQ ID NO: 10]
   This shows the base sequence of DNA encoding human VGF.
   [SEQ ID NO: 11]
   This shows the base sequence of DNA encoding rat VGF.
[SEQ ID NO: 12]
   This shows the base sequence of DNA encoding human NERP-1.
[SEQ ID NO: 13]
   This shows the base sequence of DNA encoding rat NERP-1.
[SEQ ID NO: 14]
   This shows the base sequence of DNA encoding human NERP-2.
[SEQ ID NO: 15]
   This shows the base sequence of DNA encoding rat NERP-2.
[SEQ ID NO: 16]
   This shows the base sequence of DNA encoding mouse VGF.
[SEQ ID NO: 17]
   This shows the base sequence of DNA encoding mouse NERP-1
[SEQ ID NO: 18]
   This shows the base sequence of DNA encoding mouse NERP-2.
[SEQ ID NO: 19]
   This shows the amino acid sequence of the polypeptide used as the antigen in EXAMPLE 2 below.
[SEQ ID NO: 20]
   This shows the amino acid sequence of the polypeptide used as the antigen in EXAMPLE 2 below.
[SEQ ID NO: 21]
   This shows the amino acid sequence of the polypeptide used as the antigen in EXAMPLE 2 below.
[SEQ ID NO: 22]
   This shows the amino acid sequence of ¹²⁵I-labeled polypeptide used for RIA in EXAMPLE 2 below.
[SEQ ID NO: 23]
   This shows the amino acid sequence of ¹²⁵I-labeled polypeptide used for RIA in EXAMPLE 2 below.
[SEQ ID NO: 24]
   This shows the amino acid sequence of ¹²⁵I-labeled polypeptide used for RIA in EXAMPLE 2 below.

### EXAMPLES

Hereinafter the present invention will be described more specifically by referring to EXAMPLES below but is not deemed to be limited thereto.

### EXAMPLE 1

Isolation of NERP peptides from culture supernatant of human thyroid medullary carcinoma cell line TT and structural determination

A peptide fraction was prepared from the supernatant of human thyroid medullary carcinoma cell line TT (J. Biol. Chem., 261, 14386-14391 (1986)) cultured in serum-free culture and separated by reversed phase HPLC (RP-HPLC) into 50 fractions. These fractions were analyzed with a tandem mass spectrometer to identify the sequences of 282 peptides. These peptide sequences turned out to be the sequences of known secretory proteins or physiologically active peptide precursors, suggesting that secretory proteins or peptides would be abundantly contained in the culture supernatant used. In the peptides identified, 20 peptides were carboxy-terminally amidated. This cell line produced calcitonin gene-related peptide α and calcitonin; in the amidated peptides, 16 were the full-length or fragmented peptides of these peptides. In addition, two novel peptides were further identified. In mass spectrometry, monoisotopic masses were detected on these peptides, respectively, at 2677.4 and 4062.2 and at 2522.2 and 3406.7 as their fragments, which were identified to be peptides derived from neurosecretory protein VGF (GenBank Accession No.NP_003369, Nat. Cell Biol., 2, 703-708 (2000)). The sequences of these VGF-derived peptides showed a high degree of conservation in human and rodent. Based on their physiological actions described herein, these peptides were designated as neuroendocrine regulatory peptide-1 (NERP-1) and neuroendocrine regulatory peptide-2 (NERP-2). The amino acid sequences of human, rat and mouse NTF-RP- are shown by SEQ ID NO: 4, SEQ ID NO: 5 and SEQ ID NO: 6, and the amino acid sequences of human, rat and mouse NERP-2 are shown by SEQ ID NO: 7, SEQ ID NO: 8 and SEQ ID NO: 9, respectively.

### EXAMPLE 2

### (1) Preparation of anti-NERP antisera and identification of NERP in tissues and plasma using the antisera prepared

First, antibodies against the C terminus of rat NERP-1 and NERP-2 and human NERP-2 were prepared. Specifically, rabbits were immunized with synthetic QGLAQVEA-NH₂ (SEQ ID NO: 19), C+QGGARQRDLG-NH₂ (SEQ ID NO: 20) and GC+YLLQGGARQRGLG-NH₂ (SEQ ID NO: 21) conjugated with intact (rat NERP-1) or maleimide-activated Mariculture KLH (keyhole limpet hemocyanin; Sigma and Pierce) to produce the polyclonal antibodies described above. The antibody against rat NERP-1 showed 100% cross-reactivity with human NERP-1 since the C-terminal sequences were identical. RIA (radioimmunoassay) was performed using as a ligand ¹²⁵I-radiolabeled YLLQQGLAQVEA-NH₂ (SEQ ID NO: 22) for NERP-1, YLLQGGARQRDLG-NH₂ (SEQ ID NO: 23) or YLLQGGARQRGLG-NH₂ (SEQ ID NO: 24) for NERP-2. Peptides from various tissues of male Wistar rats were extracted and then loaded on a Sep-Pak C-18 cartridge.

An aliquot of the eluate from the cartridge was provided to quantify tissue levels ofNERP. The rest of the eluate was separated by RP-HPLC coupled with RIA to identify NERP peptides. The "NERP" as used herein refers to the amidated form.

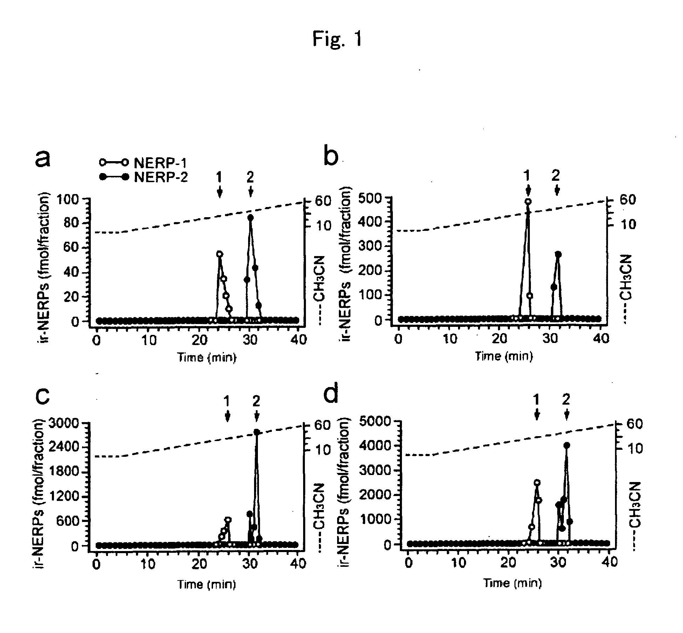

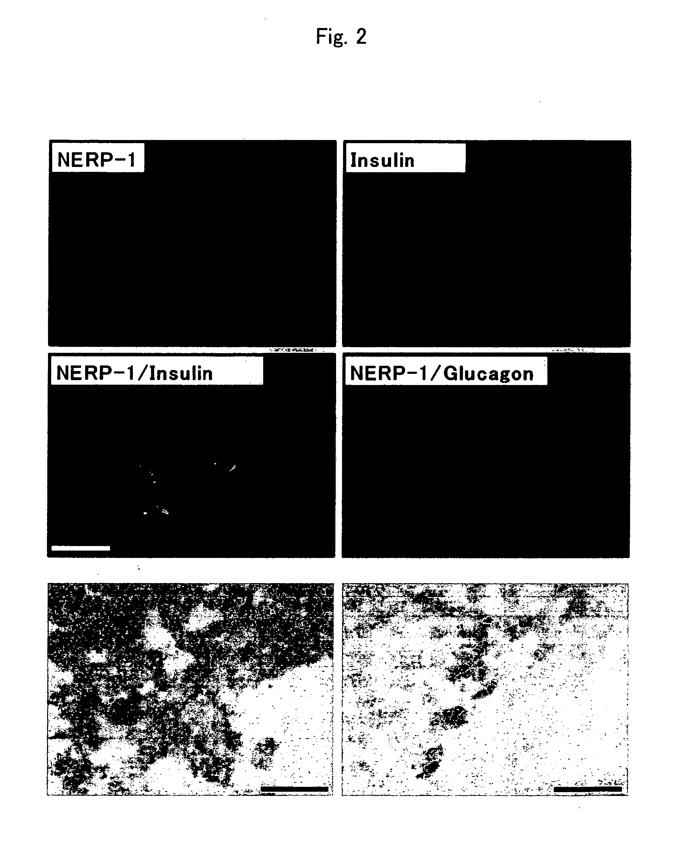

The immunoreactive NERP in human plasma and rat tissues was all coincident with standard NERP peptides (FIGS. 1a-1d) obtained by RP-HPLC, indicating that NERP is endogenously present in human and rat. The plasma levels of immunoreactive NERP-1 and NERP-2 in human were 9.8±1.1 fmol/ml and 10.8±1.0 fmol/ml, respectively. It was verified by mass spectrometry of immunoprecipitates obtained from the whole brain extract that major endogenous forms ofNERP in the rat brain are amidated 25- and 38-amino acid peptides (SEQ ID NO: 5 and SEQ ID NO: 8, respectively) corresponding to NERP-1 and NERP-2, respectively. NERP-1 and NERP-2 were detected most abundantly in the rat hypothalamus and pituitary gland, abundantly also in the brain and stomach and in all of the organs assayed (TABLE 1 and FIG 2).

### (2) Verification of endogenous presence and localization ofNERP by immunohistochemistry

Brains were removed from rats treated with colchicine (200 µg) following perfusion with either 2% paraformaldehyde for immunofluorescence microscopy or 0.1% glutaraldehyde-containing 4% paraformaldehyde for electron microscopy. Other organs were removed from rats perfused with 4% paraformaldehyde. A double immunofluorescence staining technique was performed using antibodies against NERP-1 (dilution ratio of 1: 2,500), NERP-2 (dilution ratio of 1 : 5,000), oxytocin (Chemicon), 1: 15,000), vasopressin (Peninsula Laboratories), 1: 80,000), MCH (Chemicon), 1: 2,000) and orexin-A (Santa Cruz), 1: 1,000) following the procedure described in Endocrinology, 144, 1506-1512 (2003). Images were taken by an Olympus AX-70 fluorescence microscope. Ultrathin sections (100 nm) of double-labeled antibodies for an immunogold electron microscopy were prepared by incubating with antibodies against NERP-1 (1 : 1,000), NERP-2 (1 : 3,000) or oxytocin (1 : 300) or vasopressin anti-sera (1 : 300) in accordance with the procedure described in Endocrinology, 144, 1506-1512 (2003) and then incubating with 5 nm or 10 nm colloidal gold conjugated IgG (British Biocell). The ultrathin sections were examined with a Hitachi H-7000 electron microscope.

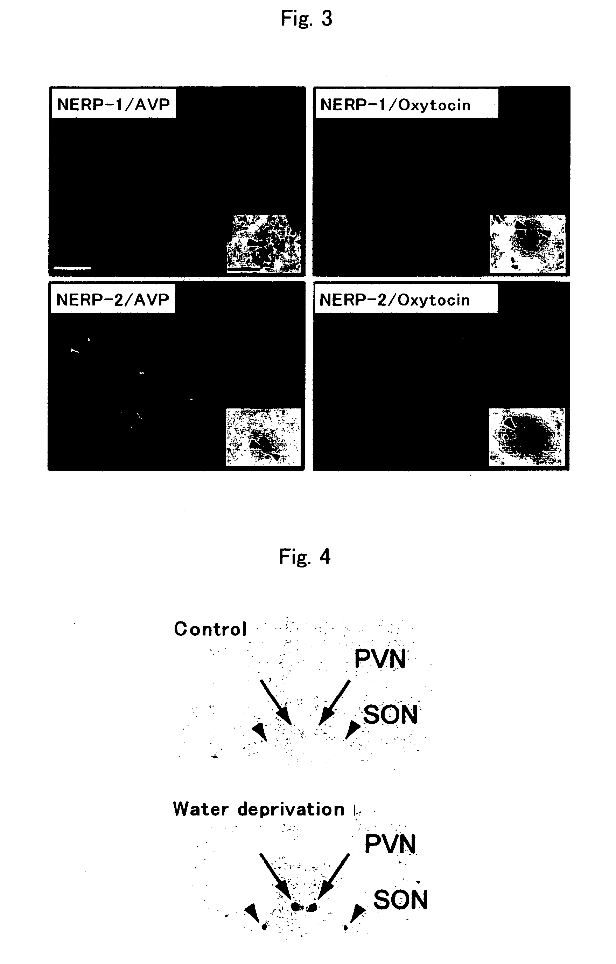

Immunoreactive cells of NERP-1 and NERP-2 were present in the supraoptic nucleus (SON) of the rat hypothalamus (FIG. 3). The NERP was also present in the magnocellular division of the paraventricular nucleus (PVN). Colloidal immunogold staining revealed the colocalization of NERP with arginine-vasopressin (AVP) and oxytocin, which are resident hormones.

**TABLE 1**

| Tissues | NERP-1 (pmol/g wet weight) | NERP-2 (pmol/g wet weight) |
|---|---|---|
| Hypothalamus | 15.59 ± 1.21 | 12.87 ± 1.50 |
| Brain | 6.60 ± 0.32 | 4.14 ± 0.25 |

| Pituitary | | |
|---|---|---|
| Anterior | 5.99 ± 0.40 | 3.11 ± 0.36 |
| Posterior | 34.73 ± 2.82 | 14.45 ± 1.91 |

| Heart | | |
|---|---|---|
| Atrium | 0.69 ± 0.18 | 0.21 ± 0.04 |
| Ventricle | 0.11 ± 0.01 | 0.03 ± 0.01 |
| Lung | 0.22 ± 0.02 | 0.07 ± 0.01 |

| Stomach | | |
|---|---|---|
| Fundus | 0.79 ± 0.02 | 0.30 ± 0.03 |
| Antrum | 0.48 ± 0.12 | 0.26 ± 0.05 |
| Duodenum | 0.97 ± 0.04 | 0.38 ± 0.13 |
| Jejunum | 1.11 ± 0.12 | 0.26 ± 0.02 |
| Ileum | 1.63 ± 0.26 | 0.18 ± 0.01 |

| Colon | | |
|---|---|---|
| Ascending | 1.98 ± 0.35 | 0.23 ± 0.03 |
| Descending | 7.14 ± 0.46 | 3.94 ± 0.30 |
| Pancreas | 0.15 ± 0.02 | 0.05 ± 0.01 |
| Liver | 0.15 ± 0.05 | 0.05 ± 0.01 |
| Spleen | 0.28 ± 0.04 | 0.06 ± 0.01 |

### EXAMPLE 3

### Assay for changes in mRNA levels of VGF by in situ hybridization

Sections of 12 µm containing PVN and SON were prepared from 6 rats for control and 6 rats deprived of water for 48 hours. Two deoxyoligonucleotide probes (1741-1785 and 1825-1870; GenBank Accession No.M74223) labeled with ³³P at the 3' end, which are complementary to the VGF-encoding transcript, were used in an equimolar ratio. In situ hybridization ofAVP was performed by the method described in Brain Res., 772, 161-6 (1997). The images obtained were analyzed on an MCID imaging analyzer (Nature, 409, 194-198 (2001)).

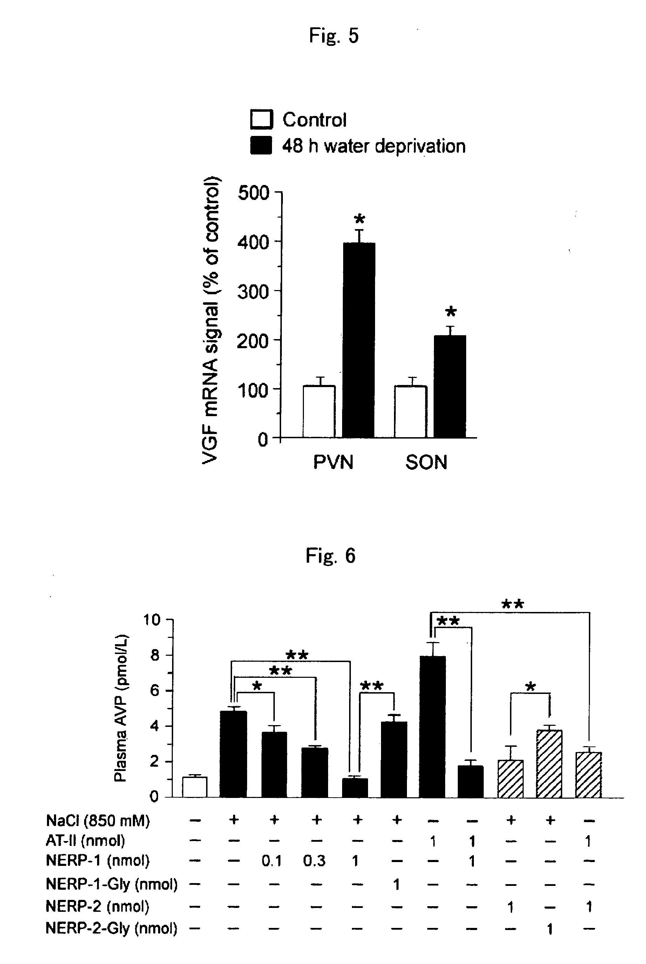

The alteration of mRNA levels of VGF which is a precursor of NERP, were assessed by quantitative in situ hybridization histochemistry. The VGF mRNA levels in both the PVN and SON were upregulated in response to water deprivation (FIGS. 4 and 5).

### EXAMPLE 4

### Measurements of AVP secretion in vivo and in vitro

NERP-1, NERP-1-Gly, NERP-2 or NERP-2-Gly was ICV-administered to rats, followed by ICV administration of hypertonic saline (850 mM NaCl/10 µl) or angiotensin II (AT-II) (1 nmol/10 µl) 5 minutes after.

Blood samples were taken 15 minutes after the injection ofNERPs. Plasma AVP was measured using an RIA kit (manufactured by Mitsubishi). Six pieces of PVN and SON were taken from the rat hypothalamus. Each nucleus was preincubated for an hour at 37°C in medium 199 (25 mM HEPES buffer, pH 7.4, 2.5% fetal calf serum, 2.5% bovine serum and 1% penicillin-streptomycin) equilibrated under 95% O₂ (n = 4 wells/group). These nuclei were sequentially stimulated 3 times each for 5 minutes, using one of NERP-1 (10⁻⁶ M), NERP-1 (10⁻⁶ M) + AT-II (10⁻⁶ M) and AT-II (10⁻⁶ M). Stimulation periods were separated by 5-minute recovery periods. Similarly, these nuclei were stimulated using one of NERP-2 (10⁻⁶ M), NERP-2 (10⁻⁶ M)+AT-II (10⁻⁶ M), NERP-1-Gly (10⁻⁶ M) and NERP-2-Gly (10⁻⁶ M). After they were thus stimulated, KCI of 6 x 10⁻² M was added to confirm depolarization-induced secretion. The two pieces from rat posterior pituitary were stimulated by NERPs and AT-II under the same conditions as described above. Aliquots of the medium were provided for RIA to measure AVP. The experiment was replicated 5 times.

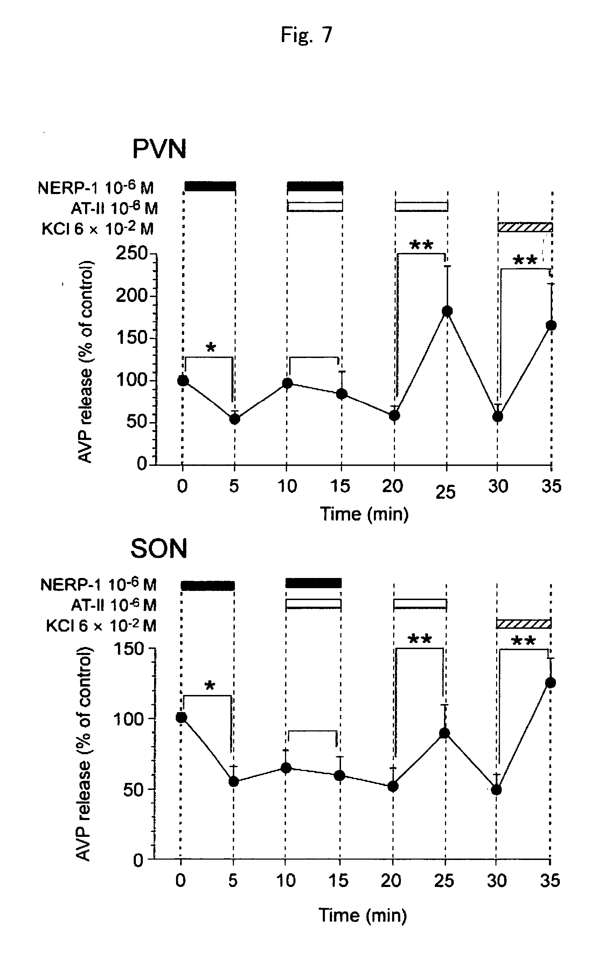

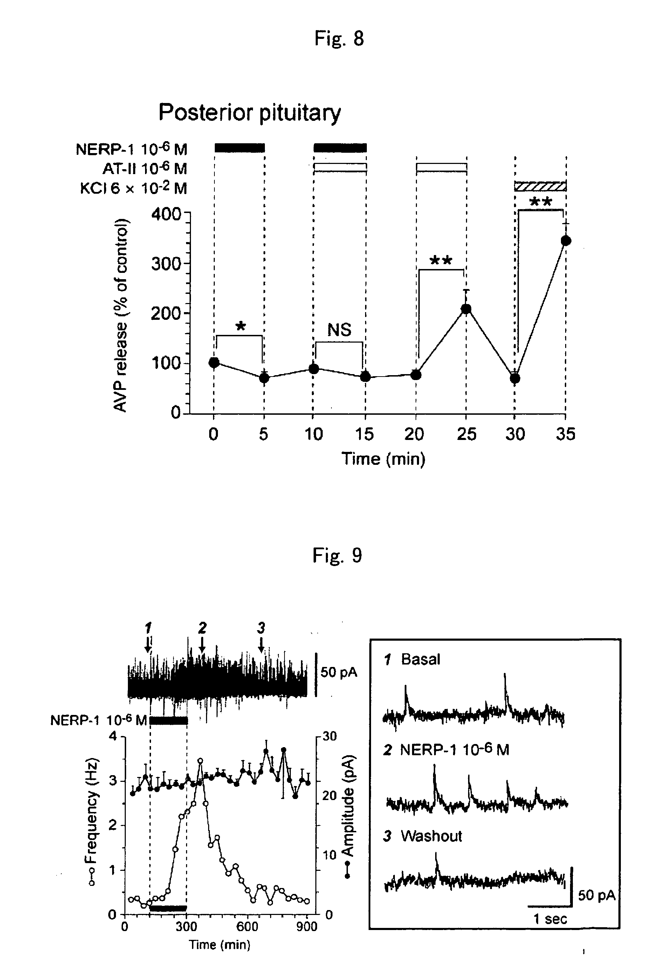

In intracerebroventricular (ICV) administration, NERP-1 dose-dependently suppressed AVP release induced by high salt administration (FIG 6). Preadministration of NERP-1 suppressed AT-II induced AVP release. Also, ICV administration of NERP-2 inhibited AVP secretion induced by high salt and AT-II (FIG 6). In an in vitro static incubation of the PAN and SON, both NERP-1 and NERP-2 suppressed basal and AT-II-induced AVP secretion (FIG 7). NERP-1 also suppressed AVP secretion from the posterior pituitary (FIG 8).

The foregoing results suggest that NERPs are involved in the regulation of AVP secretion.

### EXAMPLE 5

### Confirmation of mechanism of NERPs by electrophysiology

Slice preparations including SON from young male Wistar rats were fixed in the recording chamber in accordance with the method described in J. Physiol., 504, 113-126 (1997). The perfusion speed was set at 1.4 ml/min. Magnocellular neurosecretory neurons in the SON were identified using an upright microscope (BX50, Olympus). The measurement period of the substance was made to be 5 minutes, unless otherwise indicated. The electrodes were prepared by triple-pulling with a puller (P-87, Sutter Instrument Co.) from glass capillary. Recordings of postsynaptic currents were initiated about 5 minutes after membrane rupture when the currents reached a steady state. Currents and voltages were recorded with an EPC-10 amplifier (HEKA). Signals were filtered at 3 kHz, digitized at 1 kHz using an analog-digital converter (MacLab/v.3.5). For quantitative analysis of synaptic currents, only the AC components using a 1-Hz high pass filter were used with a software (AxoGraph v.3.6.1, Axon Instruments)).

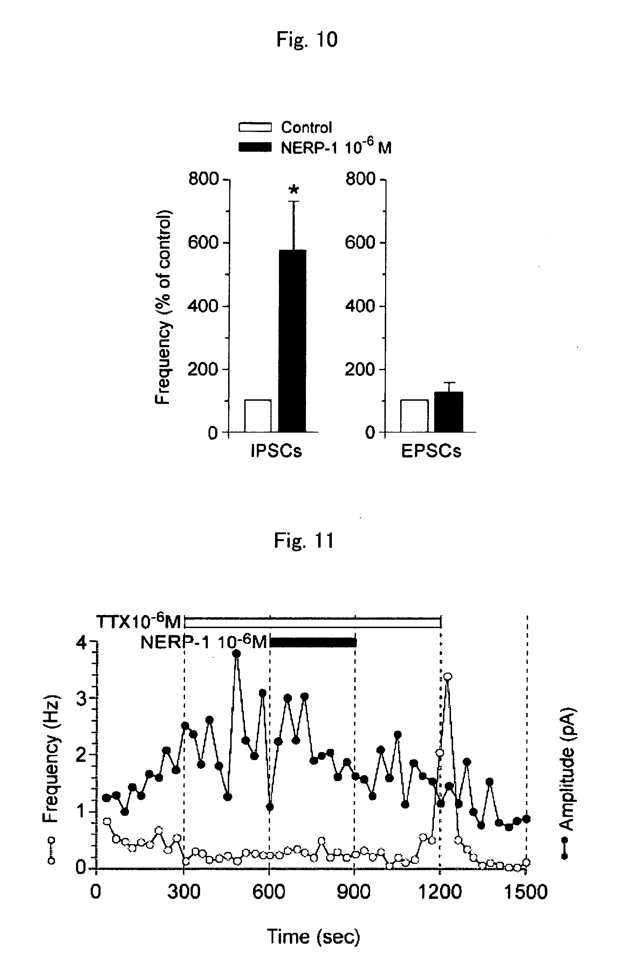

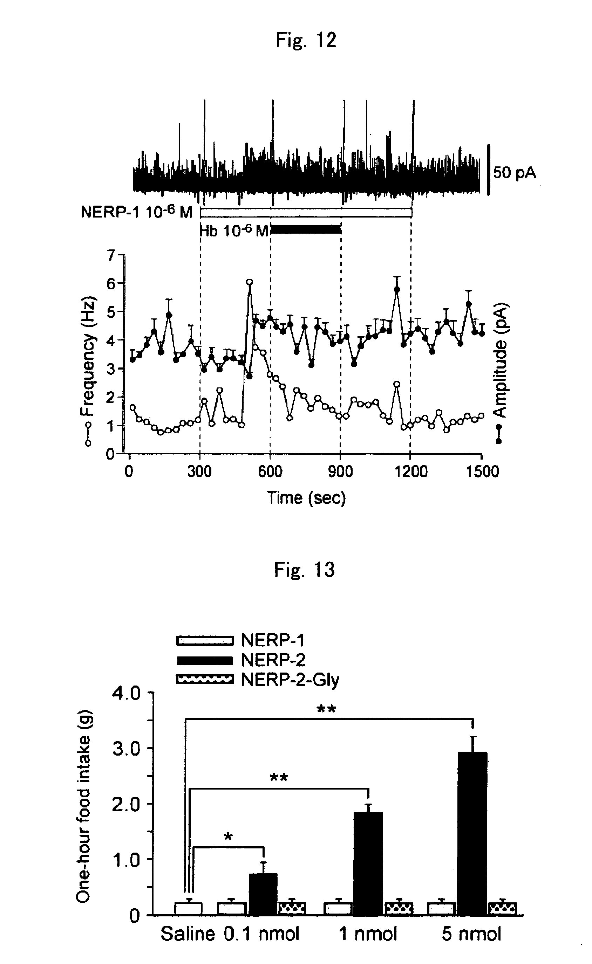

To clarify the inhibitory mechanism of NERPs associated with AVP secretion, afferent input into AVP neurons in the SON was examined using the patch-clamp technique (J. Physiol., 504, 113-126 (1997)) on an in vitro slice preparation. The neuronal activity of vasopressin neurons is regulated by excitatory glutamic inputs and inhibitory GABAergic inputs. The spontaneous excitatory and inhibitory postsynaptic currents (EPSC and IPSC) from vasopressin neurons were selectively recorded by setting the holding potentials at -70 mV and -20 mV, respectively. NERP-1 increased GABAergic IPSC but did not increase EPSC in the SON neurons (FIGS. 9 and 10). NERP-2 also increased IPSC. NERP-1 increased the frequency of IPSC but did not increase the amplitude (FIG 9). This indicates that NERPs activated presynaptic GABAergic currents. The presynaptic GABAergic currents induced by NERP-1 remained unchanged by tetrodotoxin, a Na⁺ channel blocker. This indicates that interneurons are not involved in the inhibitory effects of NERPs on the SON neurons (FIG 11). Effects of NERP-1 on nitric oxide (NO)-mediated presynaptic GABAergic currents of SON neurons were examined since the excitatory effect of NERP-1 on IPSC was similar to that effect of nitric oxide (NO). IPSCs induced by NERP-1 were not affected by application of hemoglobin (Hb), an NO scavenger (FIG 12). The above results reveal that NERPs are the local modulators of GABAergic activity in the SON neurons and elicit their effects independent of NO-mediated pathway. It was confirmed by the foregoing results that NERPs are novel biologically active peptides which induce the GABAergic inputs like NO.

### EXAMPLE 6

### Measurement of feeding-stimulating activity by feeding experiments

To assess feeding, NERP-1, NERP-2 or NERP-2-Gly (0.1- 5 nmol/10 µl) was ICV-injected to rats (n = 15 per group) at 09:30. Anti-orexin A IgG (0.25 µg/10 µl, Santa Cruz) and anti-orexin B IgG (0.25 µg/10 µl, Santa Cruz) or anti-MCH IgG (0.5 µg/10 µl, Phoenix) was ICV-injected at 09:00 to rats (n = 12 per group). In all cases, IgG was injected 3 hours before the NERP-2 administration. Food intake was monitored for an hour. In mouse experiments, NERP-2 (1 nmol/2 µl saline) or MCH (1 nmol/2 µl saline) was ICV-injected to orexin knockout mice (Cell, 92, 573-585 (1998)) and wild-type littermates (n=5 per group, 4 month-old male), then one-hour food intake was measured. Orexin knockout mice were prepared by targeted mutation in ES cells (Cell, 92, 573-585 (1998)).

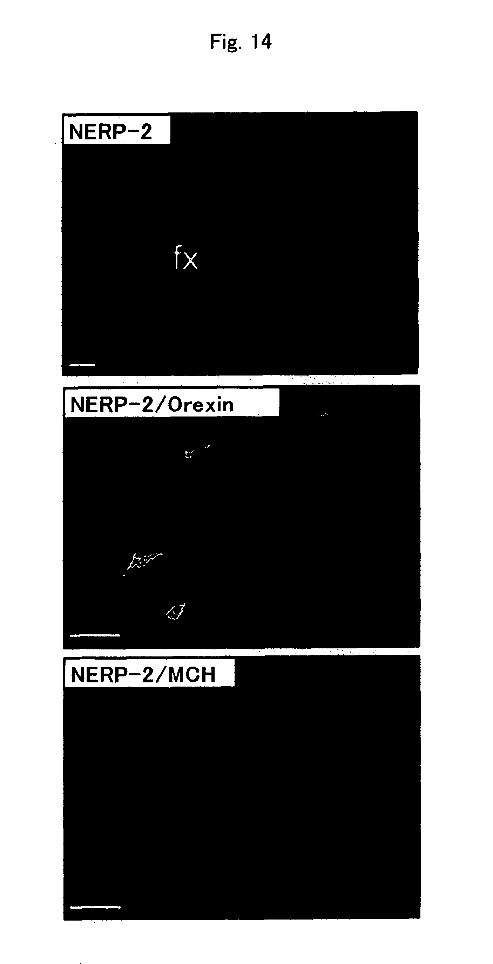

ICV injection of NERP-2 to free-feeding rats dose-dependently increased food intake during the light phase with a lowest effective dose of 0.1 nmol (FIG 13). The orexigenic effect of NERP-2 did not last longer than an hour after the ICV injection. The rats receiving ICV injection of NERP-2 remained alert and showed no unusual behavior compared to the control group. NERP-2 immunoreactive cells were present in the lateral hypothalamus (J. Physiol., 504, 113-126 (1997)), which is important in the regulation of feeding behaviors and energy metabolism (FIG 14). Double immunostaining revealed that NERP-2 is colocalized with orexin, a neuropeptide stimulating the feeding and locomotor activity (FIG 14), whereas NERP-2 is not colocalized with melanin-concentrating hormone (MCH), another orexigenic peptide produced in the lateral hypothalamus (FIG 14).

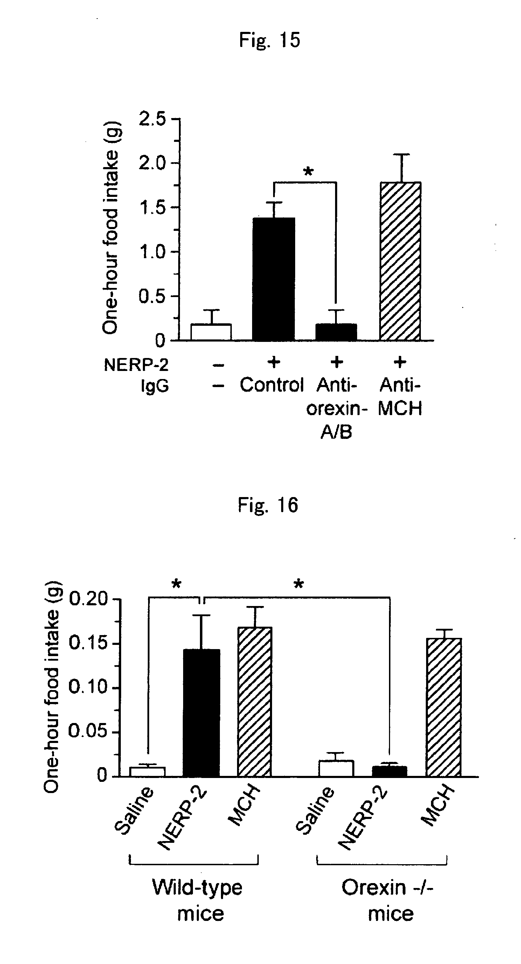

Anti-orexin-A IgG and anti-orexin-B IgG or anti-MCH IgG was intracerebroventricularly injected 3 hours before administration of NERP-2. The orexin antibodies cancelled the NERP-2-induced feeding but the MCH antibody did not (FIG 15). The ICV injection of NERP-2 stimulated food intake in wild-type control mice but did not stimulate in orexin knockout mice (FIG 16).

### EXAMPLE 7

### Assay for locomotor stimulating activity

Movement of mice given an ICV administration of NERP-2 (5 nmol/2 µl saline) or vehicle was measured in sound- and light-proof cages equipped with an infrared detector (Muromachi Co. Ltd., Tokyo, Japan) as previously described according to the method described in Endocrinology, 144, 4729-4733 (2003). Locomotor activity counts were measured every 15 minutes and summed for the period of 30 minutes to 120 minutes after the administration.

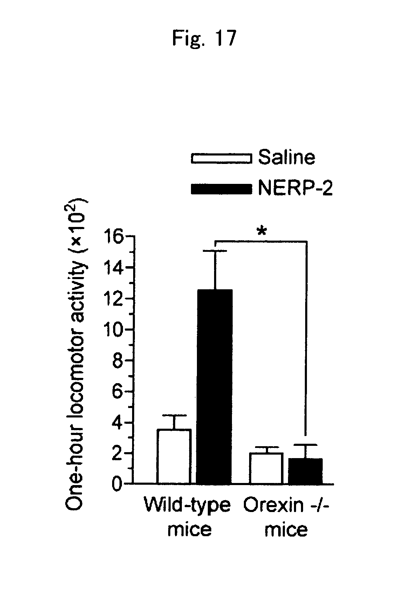

The ICV administration of NERP-2 stimulated total locomotor activity in the wild-type mice but did not stimulate in the orexin knockout mice (FIG 17).

The foregoing results demonstrate that NERP-2 interacts with the orexin system to stimulate the feeding and locomotor activity.

### INDUSTRIAL APPLICABILITY

In the present invention, for example, the polypeptide comprising the same or substantially the same amino acid sequence as the amino acid sequence represented by SEQ ID NO: 4, SEQ ID NO: 5 or SEQ ID NO: 6, its amide or its ester, or a salt thereof; the compound or its salt that promotes the activity of said polypeptide or its partial peptide, its amide or its ester, or a salt thereof; the polynucleotide comprising the polynucleotide encoding said polypeptide or its partial peptide; and the like can be used, for example, as vasopressin release inhibitors, etc., such as agents for preventing/treating renal edema, dysuria, hyponatremia, syndrome of inappropriate secretion of antidiuretic hormone (SIADH), hypertension, etc.

Also, the compound or its salt that inhibits the activity of the polypeptide comprising the same or substantially the same amino acid sequence as the amino acid sequence represented by SEQ ID NO: 4, SEQ ID NO: 5 or SEQ ID NO: 6, its amide or its ester, or a salt thereof; the antibody against the polypeptide, its partial peptide, its amide or its ester, or a salt thereof; the antisense polynucleotide comprising an entire or part of the base sequence complementary or substantially complementary to the base sequence of the polynucleotide encoding the polypeptide or its partial peptide; and the like can be used as agents for preventing/treating, for example, urine storage disorders, polyuria, diabetes insipidus, hypernatremia, metabolic alkalosis, hypokalemia, Cushing syndrome, etc.

In addition, the polypeptide comprising the same or substantially the same amino acid sequence as the amino acid sequence represented by SEQ ID NO: 7, SEQ ID NO: 8 or SEQ ID NO: 9, its amide or its ester, or a salt thereof; the compound or its salt that promotes the activity of said polypeptide or its partial peptide, its amide or its ester, or a salt thereof; the polynucleotide comprising the polynucleotide encoding said polypeptide or its partial peptide; and the like can be used, for example, vasopressin release inhibitors, etc., such as agents for preventing/treating renal edema, dysuria, hyponatremia, syndrome of inappropriate secretion of antidiuretic hormone, hypertension or anorexia, an eating (appetite) stimulant, agents for preventing/treating sleeping disorders [e.g., primary insomnia, circadian rhythm disorders (e.g., change in physical conditions caused by three-shift work, time zone change syndrome (jet lag), etc.)], and the like.

Furthermore, the compound or its salt that inhibits the activity of the polypeptide comprising the same or substantially the same amino acid sequence as the amino acid sequence represented by SEQ ID NO: 7, SEQ ID NO: 8 or SEQ ID NO: 9, its amide or its ester, or a salt thereof; the antibody against the polypeptide, its partial peptide, its amide or its ester, or a salt thereof; the antisense polynucleotide comprising an entire or part of the base sequence complementary or substantially complementary to the base sequence of the polynucleotide encoding the polypeptide or its partial peptide; and the like can be used as preventive/therapeutic agents such as agents for preventing/treating, for example, urine storage disorders, polyuria, diabetes insipidus, hypernatremia, metabolic alkalosis, hypokalemia, Cushing syndrome, obesity, hyperphagia, sleeping disorders [e.g., primary insomnia, circadian rhythm disorders (e.g., change in physical conditions caused by three-shift work, time zone change syndrome (jet lag), etc.)], and the like.

Moreover, the polypeptide comprising the same or substantially the same amino acid sequence as the amino acid sequence represented by SEQ ID NO: 4, SEQ ID NO: 5 or SEQ ID NO: 6, SEQ ID NO: 7, SEQ ID NO: 8 or SEQ ID NO: 9, its amide or its ester, or a salt thereof, etc. are useful for screening, e.g., preventive/therapeutic agents for the diseases associated with these polypeptides, etc. described above, and so on.

Furthermore, the antibodies to the polypeptide comprising the same or substantially the same amino acid sequence as the amino acid sequence represented by SEQ ID NO: 4, SEQ ID NO: 5 or SEQ ID NO: 6, SEQ ID NO: 7, SEQ ID NO: 8 or SEQ ID NO: 9, its amide or its ester, or a salt thereof, etc. are useful for diagnosis of, e.g., the diseases described above.

## Claims

1. A polypeptide comprising the same or substantially the same amino acid sequence as the amino acid sequence represented by SEQ ID NO: 4, SEQ ID NO: 5 or SEQ ID NO: 6 (with the proviso that a polypeptide consisting of the amino acid sequence represented by SEQ ID NO: 1, SEQ ID NO: 2 or SEQ ID NO: 3 is excluded), its amide or its ester, or a salt thereof.

2. The polypeptide according to claim 1, which consists of the same or substantially the same amino acid sequence as the amino acid sequence represented by SEQ ID NO: 4, SEQ ID NO: 5 or SEQ ID NO: 6; its amide or its ester, or a salt thereof.

3. The polypeptide according to claim 1, which consists of the amino acid sequence represented by SEQ ID NO: 4, SEQ ID NO: 5 or SEQ ID NO: 6; its amide or its ester, or a salt thereof.

4. A partial peptide of the polypeptide according to claim 1, its amide or its ester, or a salt thereof.

5. A polypeptide comprising the same or substantially the same amino acid sequence as the amino acid sequence represented by SEQ ID NO: 7, SEQ ID NO: 8 or SEQ ID NO: 9 (with the proviso that a polypeptide consisting of the amino acid sequence represented by SEQ ID NO: 1, SEQ ID NO: 2 or SEQ ID NO: 3 is excluded), its amide or its ester, or a salt thereof.

6. The polypeptide according to claim 5, which consists of the same or substantially the same amino acid sequence as the amino acid sequence represented by SEQ ID NO: 7, SEQ ID NO: 8 or SEQ ID NO: 9; its amide or its ester, or a salt thereof.

7. The polypeptide according to claim 5, which consists of the amino acid sequence represented by SEQ ID NO: 7, SEQ ID NO: 8 or SEQ ID NO: 9; its amide or its ester, or a salt thereof.

8. A partial peptide of the polypeptide according to claim 5, its amide or its ester, or a salt thereof.

9. A polynucleotide comprising a polynucleotide encoding the polypeptide according to claim 1 or its partial peptide.

10. The polynucleotide according to claim 9, which is a DNA.

11. The DNA according to claim 10, which comprises the base sequence represented by SEQ ID NO: 12 or SEQ ID NO: 13.

12. The DNA according to claim 10, which consists of the base sequence represented by SEQ ID NO: 12 or SEQ ID NO: 13.

13. A polynucleotide comprising a polynucleotide encoding the polypeptide according to claim 5 or its partial peptide.

14. The polynucleotide according to claim 13, which is a DNA.

15. The DNA according to claim 14, which comprises the base sequence represented by SEQ ID NO: 14 or SEQ ID NO: 15.

16. The DNA according to claim 14, which consists of the base sequence represented by SEQ ID NO: 14 or SEQ ID NO: 15.

17. A recombinant vector comprising the polynucleotide according to claim 9 or 13.

18. A transformant transformed with the recombinant vector according to claim 17.

19. A method for manufacturing the polypeptide according to claim 1 or 5, its partial peptide, or a salt thereof, which comprises culturing the transformant according to claim 18, forming and accumulating said polypeptide or the partial peptide thereof, and recovering it.

20. An antibody against the polypeptide according to claim 1, its partial peptide, its amide or its ester, or a salt thereof.

21. A method for quantifying the polypeptide according to claim 1, which comprises using the antibody according to claim 20.

22. A method for diagnosing diseases associated with the function of the polypeptide according to claim 1, which comprises using the quantifying method according to claim 21.

23. An antibody against the polypeptide according to claim 5, its partial peptide, its amide or its ester, or a salt thereof.

24. A method for quantifying the polypeptide according to claim 5, which comprises using the antibody according to claim 23.

25. A method for diagnosing diseases associated with the function of the polypeptide according to claim 5, which comprises using the quantifying method according to claim 24.

26. An antisense polynucleotide comprising an entire or part of a base sequence complementary or substantially complementary to the base sequence of the polynucleotide according to claim 9.

27. An antisense polynucleotide comprising an entire or part of a base sequence complementary or substantially complementary to the base sequence of the polynucleotide according to claim 13.

28. A method for screening a compound or a salt thereof that promotes or inhibits the activity of the polypeptide according to claim 1, which comprises using said polypeptide, its partial peptide, its amide or its ester, or a salt thereof.

29. A kit for screening a compound or a salt thereof that promotes or inhibits the activity of the polypeptide according to claim 1, which comprises said polypeptide, its partial peptide, its amide or its ester, or a salt thereof.

30. A method for screening a compound or a salt thereof that promotes or inhibits the activity of the polypeptide according to claim 5, which comprises using said polypeptide, its partial peptide, its amide or its ester, or a salt thereof.

31. A kit for screening a compound or a salt thereof that promotes or inhibits the activity of the polypeptide according to claim 5, which comprises said polypeptide, its partial peptide, its amide or its ester, or a salt thereof.

32. A medicament comprising the polypeptide according to claim 1, its partial peptide, its amide or its ester, or a salt thereof.

33. A medicament comprising the polynucleotide according to claim 9.

34. A medicament comprising a compound or a salt thereof that promotes the activity of the polypeptide according to claim 1, its partial peptide, its amide or its ester, or a salt thereof.

35. The medicament according to any one of claims 32 to 34, which is an agent for preventing/treating renal edema, dysuria, hyponatremia, syndrome of inappropriate secretion of antidiuretic hormone or hypertension.

36. A medicament comprising a compound or a salt thereof that inhibits the activity of the polypeptide according to claim 1, its partial peptide, its amide or its ester, or a salt thereof.

37. A medicament comprising the antibody according to claim 20.

38. A medicament comprising the antisense polynucleotide according to claim 26.

39. The medicament according to any one of claims 36 to 38, which is an agent for preventing/treating urine storage disorders, polyuria, diabetes insipidus, hypernatremia, metabolic alkalosis, hypokalemia or Cushing syndrome.

40. A medicament comprising the polypeptide according to claim 5, its partial peptide, its amide or its ester, or a salt thereof.

41. A medicament comprising the polynucleotide according to claim 13.

42. A medicament comprising a compound or a salt thereof that promotes the activity of the polypeptide according to claim 5, its partial peptide, its amide or its ester, or a salt thereof.

43. The medicament according to any one of claims 40 to 42, which is an agent for preventing/treating renal edema, dysuria, hyponatremia, syndrome of inappropriate secretion of antidiuretic hormone, hypertension or anorexia, or eating stimulant.

44. A medicament comprising a compound or a salt thereof that inhibits the activity of the polypeptide according to claim 5, its partial peptide, its amide or its ester, or a salt thereof.

45. A medicament comprising the antibody according to claim 23.

46. A medicament comprising the antisense polynucleotide according to claim 27.

47. The medicament according to any one of claims 44 to 46, which is an agent for preventing/treating urine storage disorders, polyuria, diabetes insipidus, hypernatremia, metabolic alkalosis, hypokalemia, Cushing syndrome, obesity or hyperphagia.

48. A non-human transgenic animal bearing the polynucleotide according to claim 9 or 13, which is exogenous.

49. A method for preventing/treating renal edema, dysuria, hyponatremia, syndrome of inappropriate secretion of antidiuretic hormone or hypertension, which comprises promoting the activity of the polypeptide according to claim 1, its partial peptide, its amide or its ester, or a salt thereof.

50. A method for preventing/treating renal edema, dysuria, hyponatremia, syndrome of inappropriate secretion of antidiuretic hormone or hypertension, which comprises administering to a mammal an effective dose of the polypeptide according to claim 1, its partial peptide, its amide or its ester, or a salt thereof, the polynucleotide according to claim 9, or a compound or a salt thereof that promotes the activity of said polypeptide, its partial peptide, its amide or its ester, or a salt thereof.

51. Use of the polypeptide according to claim 1, its partial peptide, its amide or its ester, or a salt thereof, the polynucleotide according to claim 9, or a compound or a salt thereof that promotes the activity of said polypeptide, its partial peptide, its amide or its ester, or a salt thereof, for the manufacture of an agent for preventing/treating renal edema, dysuria, hyponatremia, syndrome of inappropriate secretion of antidiuretic hormone or hypertension.

52. A method for preventing/treating urine storage disorders, polyuria, diabetes insipidus, hypernatremia, metabolic alkalosis, hypokalemia or Cushing syndrome, which comprises inhibiting the activity of the polypeptide according to claim 1, its partial peptide, its amide or its ester, or a salt thereof.

53. A method for preventing/treating urine storage disorders, polyuria, diabetes insipidus, hypernatremia, metabolic alkalosis, hypokalemia or Cushing syndrome, which comprises administering to a mammal an effective dose of a compound or a salt thereof that inhibits the activity of the polypeptide according to claim 1, its partial peptide, its amide or its ester, or a salt thereof, the antibody according to claim 20, or the antisense polynucleotide according to claim 26.

54. Use of a compound or a salt thereof that inhibits the activity of the polypeptide according to claim 1, its partial peptide, its amide or its ester, or a salt thereof, the antibody according to claim 20, or the antisense polynucleotide according to claim 26, for the manufacture of an agent for preventing/treating urine storage disorders, polyuria, diabetes insipidus, hypernatremia, metabolic alkalosis, hypokalemia or Cushing syndrome.

55. A method for preventing/treating renal edema, dysuria, hyponatremia, syndrome of inappropriate secretion of antidiuretic hormone, hypertension or anorexia, or a method for promoting eating, which comprises promoting the activity of the polypeptide according to claim 5, its partial peptide, its amide or its ester, or a salt thereof.

56. A method for preventing/treating renal edema, dysuria, hyponatremia, syndrome of inappropriate secretion of antidiuretic hormone, hypertension or anorexia, or a method for promoting eating, which comprises administering to a mammal an effective dose of the polypeptide according to claim 5, its partial peptide, its amide or its ester, or a salt thereof, the polynucleotide according to claim 13, or a compound or a salt thereof that promotes the activity of said polypeptide, its partial peptide, its amide or its ester, or a salt thereof.

57. Use of the polypeptide according to claim 5, its partial peptide, its amide or its ester, or a salt thereof, the polynucleotide according to claim 13, or a compound of a salt thereof that promotes the activity of said polypeptide, its partial peptide, its amide or its ester, or a salt thereof, for the manufacture of an agent for preventing/treating renal edema, dysuria, hyponatremia, syndrome of inappropriate secretion of antidiuretic hormone, hypertension or anorexia, or an eating stimulant.

58. A method for preventing/treating urine storage disorders, polyuria, diabetes insipidus, hypernatremia, metabolic alkalosis, hypokalemia, Cushing syndrome, obesity or hyperphagia, which comprises inhibiting the activity of the polypeptide according to claim 5, its partial peptide, its amide or its ester, or a salt thereof.

59. A method for preventing/treating urine storage disorders, polyuria, diabetes insipidus, hypernatremia, metabolic alkalosis, hypokalemia, Cushing syndrome, obesity or hyperphagia, which comprises administering to a mammal an effective dose of a compound or a salt thereof that inhibits the activity of the polypeptide according to claim 5, its partial peptide, its amide or its ester, or a salt thereof, the antibody according to claim 23, or the antisense polynucleotide according to claim 27.

60. Use of a compound or a salt thereof that inhibits the activity of the polypeptide according to claim 5, its partial peptide, its amide or its ester, or a salt thereof, the antibody according to claim 23, or the antisense polynucleotide according to claim 27, for the manufacture of an agent for preventing/treating urine storage disorders, polyuria, diabetes insipidus, hypernatremia, metabolic alkalosis, hypokalemia, Cushing syndrome, obesity or hyperphagia.


SEQUENCE LISTING

<110> Takeda Pharmaceutical Company Limited

<120> Novel polypeptides and use thereof

<130> G07-0075

<140> PCT/JP2006/309192
<141> 2006-04-26

<150> JP2005-130191
<151> 2005-04-27

<160> 24

<210> 1
<211> 615
<212> PRT
<213> Homo sapiens

<400> 1
Met Lys Ala Leu Arg Leu Ser Ala Ser Ala Leu Phe Cys Leu Leu Leu
                5                   10                  15
Ile Asn Gly Leu Gly Ala Ala Pro Pro Gly Arg Pro Glu Ala Gln Pro
            20                  25                  30
Pro Pro Leu Ser Ser Glu His Lys Glu Pro Val Ala Gly Asp Ala Val
        35                  40                  45
Pro Gly Pro Lys Asp Gly Ser Ala Pro Glu Val Arg Gly Ala Arg Asn
    50                  55                  60
Ser Glu Pro Gln Asp Glu Gly Glu Leu Phe Gln Gly Val Asp Pro Arg

65                  70                  75                  80
Ala Leu Ala Ala Val Leu Leu Gln Ala Leu Asp Arg Pro Ala Ser Pro
                85                  90                  95
Pro Ala Pro Ser Gly Ser Gln Gln Gly Pro Glu Glu Glu Ala Ala Glu
            100                 105                 110
Ala Leu Leu Thr Glu Thr Val Arg Ser Gln Thr His Ser Leu Pro Ala
        115                 120                 125
Pro Glu Ser Pro Glu Pro Ala Ala Pro Pro Arg Pro Gln Thr Pro Glu
    130                 135                 140
Asn Gly Pro Glu Ala Ser Asp Pro Ser Glu Glu Leu Glu Ala Leu Ala
145                 150                 155                 160
Ser Leu Leu Gln Glu Leu Arg Asp Phe Ser Pro Ser Ser Ala Lys Arg
                165                 170                 175
Gln Gln Glu Thr Ala Ala Ala Glu Thr Glu Thr Arg Thr His Thr Leu
            180                 185                 190
Thr Arg Val Asn Leu Glu Ser Pro Gly Pro Glu Arg Val Trp Arg Ala
        195                 200                 205
Ser Trp Gly Glu Phe Gln Ala Arg Val Pro Glu Arg Ala Pro Leu Pro
    210                 215                 220
Pro Pro Ala Pro Ser Gln Phe Gln Ala Arg Met Pro Asp Ser Gly Pro
225                 230                 235                 240
Leu Pro Glu Thr His Lys Phe Gly Glu Gly Val Ser Ser Pro Lys Thr
                245                 250                 255
His Leu Gly Glu Ala Leu Ala Pro Leu Ser Lys Ala Tyr Gln Gly Val
            260                 265                 270
Ala Ala Pro Phe Pro Lys Ala Arg Arg Pro Glu Ser Ala Leu Leu Gly
        275                 280                 285
Gly Ser Glu Ala Gly Glu Arg Leu Leu Gln Gln Gly Leu Ala Gln Val
    290                 295                 300
Glu Ala Gly Arg Arg Gln Ala Glu Ala Thr Arg Gln Ala Ala Ala Gln
305                 310                 315                 320

Glu Glu Arg Leu Ala Asp Leu Ala Ser Asp Leu Leu Leu Gln Tyr Leu
                325                 330                 335
Leu Gln Gly Gly Ala Arg Gln Arg Gly Leu Gly Gly Arg Gly Leu Gln
            340                 345                 350
Glu Ala Ala Glu Glu Arg Glu Ser Ala Arg Glu Glu Glu Glu Ala Glu
        355                 360                 365
Gln Glu Arg Arg Gly Gly Glu Glu Arg Val Gly Glu Glu Asp Glu Glu
    370                 375                 380
Ala Ala Glu Ala Glu Ala Glu Ala Glu Glu Ala Glu Arg Ala Arg Gln
385                 390                 395                 400
Asn Ala Leu Leu Phe Ala Glu Glu Glu Asp Gly Glu Ala Gly Ala Glu
                405                 410                 415
Asp Lys Arg Ser Gln Glu Glu Thr Pro Gly His Arg Arg Lys Glu Ala
            420                 425                 430
Glu Gly Thr Glu Glu Gly Gly Glu Glu Glu Asp Asp Glu Glu Met Asp
        435                 440                 445
Pro Gln Thr Ile Asp Ser Leu Ile Glu Leu Ser Thr Lys Leu His Leu
    450                 455                 460
Pro Ala Asp Asp Val Val Ser Ile Ile Glu Glu Val Glu Glu Lys Arg
465                 470                 475                 480
Lys Arg Lys Lys Asn Ala Pro Pro Glu Pro Val Pro Pro Pro Arg Ala
                485                 490                 495
Ala Pro Ala Pro Thr His Val Arg Ser Pro Gln Pro Pro Pro Pro Ala
            500                 505                 510
Pro Ala Pro Ala Arg Asp Glu Leu Pro Asp Trp Asn Glu Val Leu Pro
        515                 520                 525
Pro Trp Asp Arg Glu Glu Asp Glu Val Tyr Pro Pro Gly Pro Tyr His
    530                 535                 540
Pro Phe Pro Asn Tyr Ile Arg Pro Arg Thr Leu Gln Pro Pro Ser Ala
545                 550                 555                 560
Leu Arg Arg Arg His Tyr His His Ala Leu Pro Pro Ser Arg His Tyr

                565                 570                 575
Pro Gly Arg Glu Ala Gln Ala Arg Arg Ala Gln Glu Glu Ala Glu Ala
            580                 585                 590
Glu Glu Arg Arg Leu Gln Glu Gln Glu Glu Leu Glu Asn Tyr Ile Glu
        595                 600                 605
His Val Leu Leu Arg Arg Pro
    610                 615

<210> 2
<211> 617
<212> PRT
<213> Rattus norvegicus

<400> 2
Met Lys Thr Phe Thr Leu Pro Ala Ser Val Leu Phe Cys Phe Leu Leu
                5                   10                  15
Leu Ile Arg Gly Leu Gly Ala Ala Pro Pro Gly Arg Ser Asp Val Tyr
            20                  25                  30
Pro Pro Pro Leu Gly Ser Glu His Asn Gly Gln Val Ala Glu Asp Ala
        35                  40                  45
Val Ser Arg Pro Lys Asp Asp Ser Val Pro Glu Val Arg Ala Ala Arg
    50                  55                  60
Asn Ser Glu Pro Gln Asp Gln Gly Glu Leu Phe Gln Gly Val Asp Pro
65                  70                  75                  80
Arg Ala Leu Ala Ala Val Leu Leu Gln Ala Leu Asp Arg Pro Ala Ser
                85                  90                  95
Pro Pro Ala Val Pro Ala Gly Ser Gln Gln Gly Thr Pro Glu Glu Ala
            100                 105                 110
Ala Glu Ala Leu Leu Thr Glu Ser Val Arg Ser Gln Thr His Ser Leu
        115                 120                 125
Pro Ala Ser Glu Ile Gln Ala Ser Ala Val Ala Pro Pro Arg Pro Gln

130                 135                 140
Thr Gln Asp Asn Asp Pro Glu Ala Asp Asp Arg Ser Glu Glu Leu Glu
145                 150                 155                 160
Ala Leu Ala Ser Leu Leu Gln Glu Leu Arg Asp Phe Ser Pro Ser Asn
                165                 170                 175
Ala Lys Arg Gln Gln Glu Thr Ala Ala Ala Glu Thr Glu Thr Arg Thr
            180                 185                 190
His Thr Leu Thr Arg Val Asn Leu Glu Ser Pro Gly Pro Glu Arg Val
        195                 200                 205
Trp Arg Ala Ser Trp Gly Glu Phe Gln Ala Arg Val Pro Glu Arg Ala
    210                 215                 220
Pro Leu Pro Pro Ser Val Pro Ser Gln Phe Gln Ala Arg Met Ser Glu
225                 230                 235                 240
Asn Val Pro Leu Pro Glu Thr His Gln Phe Gly Glu Gly Val Ser Ser
                245                 250                 255
Pro Lys Thr His Leu Gly Glu Thr Leu Thr Pro Leu Ser Lys Ala Tyr
            260                 265                 270
Gln Ser Leu Ser Ala Pro Phe Pro Lys Val Arg Arg Leu Glu Gly Ser
        275                 280                 285
Phe Leu Gly Gly Ser Glu Ala Gly Glu Arg Leu Leu Gln Gln Gly Leu
    290                 295                 300
Ala Gln Val Glu Ala Gly Arg Arg Gln Ala Glu Ala Thr Arg Gln Ala
305                 310                 315                 320
Ala Ala Gln Glu Glu Arg Leu Ala Asp Leu Ala Ser Asp Leu Leu Leu
                325                 330                 335
Gln Tyr Leu Leu Gln Gly Gly Ala Arg Gln Arg Asp Leu Gly Gly Arg
            340                 345                 350
Gly Leu Gln Glu Thr Gln Gln Glu Arg Glu Asn Glu Arg Glu Glu Glu
        355                 360                 365
Ala Glu Gln Glu Arg Arg Gly Gly Gly Glu Asp Glu Val Gly Glu Glu
    370                 375                 380

Asp Glu Glu Ala Ala Glu Ala Glu Ala Glu Ala Glu Glu Ala Glu Arg
385                 390                 395                 400
Ala Arg Gln Asn Ala Leu Leu Phe Ala Glu Glu Glu Asp Gly Glu Ala
                405                 410                 415
Gly Ala Glu Asp Lys Arg Ser Gln Glu Glu Ala Pro Gly His Arg Arg
            420                 425                 430
Lys Asp Ala Glu Gly Thr Glu Glu Gly Gly Glu Glu Asp Asp Asp Asp
        435                 440                 445
Glu Glu Met Asp Pro Gln Thr Ile Asp Ser Leu Ile Glu Leu Ser Thr
    450                 455                 460
Lys Leu His Leu Pro Ala Asp Asp Val Val Ser Ile Ile Glu Glu Val
465                 470                 475                 480
Glu Glu Lys Arg Lys Arg Lys Lys Asn Ala Pro Pro Glu Pro Val Pro
                485                 490                 495
Pro Pro Arg Ala Ala Pro Ala Pro Thr His Val Arg Ser Pro Gln Pro
            500                 505                 510
Pro Pro Pro Ala Pro Ala Arg Asp Glu Leu Pro Asp Trp Asn Glu Val
        515                 520                 525
Leu Pro Pro Trp Asp Arg Glu Glu Asp Glu Val Phe Pro Pro Gly Pro
    530                 535                 540
Tyr His Pro Phe Pro Asn Tyr Ile Arg Pro Arg Thr Leu Gln Pro Pro
545                 550                 555                 560
Ala Ser Ser Arg Arg Arg His Phe His His Ala Leu Pro Pro Ala Arg
                565                 570                 575
His His Pro Asp Leu Glu Ala Gln Ala Arg Arg Ala Gln Glu Glu Ala
            580                 585                 590
Asp Ala Glu Glu Arg Arg Leu Gln Glu Gln Glu Glu Leu Glu Asn Tyr
        595                 600                 605
Ile Glu His Val Leu Leu His Arg Pro
    610                 615

<210> 3

<211> 617

<212> PRT

<213> Mus musculus

<400> 3

Met Lys Thr Phe Thr Leu Pro Ala Ser Val Leu Phe Cys Phe Leu Leu
                5                   10                  15

Leu Ile Gln Gly Leu Gly Ala Ala Pro Pro Gly Arg Pro Asp Val Phe
            20                  25                  30

Pro Pro Pro Leu Ser Ser Glu His Asn Gly Gln Val Ala Glu Asp Ala
        35                  40                  45

Val Ser Arg Pro Lys Asp Asp Gly Val Pro Glu Val Arg Ala Ala Arg
    50                  55                  60

Asn Pro Glu Pro Gln Asp Gln Gly Glu Leu Phe Gln Gly Val Asp Pro
65                  70                  75                  80

Arg Ala Leu Ala Ser Val Leu Leu Gln Ala Leu Asp Arg Pro Ala Ser
                85                  90                  95

Pro Pro Ser Val Pro Gly Gly Ser Gln Gln Gly Thr Pro Glu Glu Ala
            100                 105                 110

Ala Glu Ala Leu Leu Thr Glu Ser Val Arg Ser Gln Thr His Ser Leu
        115                 120                 125

Pro Ala Pro Glu Ile Gln Ala Pro Ala Val Ala Pro Pro Arg Pro Gln
    130                 135                 140

Thr Gln Asp Arg Asp Pro Glu Glu Asp Asp Arg Ser Glu Glu Leu Glu
145                 150                 155                 160

Ala Leu Ala Ser Leu Leu Gln Glu Leu Arg Asp Phe Ser Pro Ser Asn
                165                 170                 175

Ala Lys Arg Gln Gln Glu Thr Ala Ala Ala Glu Thr Glu Thr Arg Thr
            180                 185                 190

His Thr Leu Thr Arg Val Asn Leu Glu Ser Pro Gly Pro Glu Arg Val

        195                 200                 205
Trp Arg Ala Ser Trp Gly Glu Phe Gln Ala Arg Val Pro Glu Arg Ala
    210                 215                 220
Pro Leu Pro Pro Pro Val Pro Ser Gln Phe Gln Ala Arg Met Ser Glu
225                 230                 235                 240
Ser Ala Pro Leu Pro Glu Thr His Gln Phe Gly Glu Gly Val Ser Ser
                245                 250                 255
Pro Lys Thr His Leu Gly Glu Thr Leu Thr Pro Leu Ser Lys Ala Tyr
            260                 265                 270
Gln Ser Leu Gly Gly Pro Phe Pro Lys Val Arg Arg Leu Glu Gly Ser
        275                 280                 285
Phe Leu Gly Gly Ser Glu Ala Gly Glu Arg Leu Leu Gln Gln Gly Leu
    290                 295                 300
Ala Gln Val Glu Ala Gly Arg Arg Gln Ala Glu Ala Thr Arg Gln Ala
305                 310                 315                 320
Ala Ala Gln Glu Glu Arg Leu Ala Asp Leu Ala Ser Asp Leu Leu Leu
                325                 330                 335
Gln Tyr Leu Leu Gln Gly Gly Ala Arg Gln Arg Asp Leu Gly Gly Arg
            340                 345                 350
Glu Leu Gln Glu Thr Gln Gln Glu Arg Glu Asn Glu Arg Glu Glu Glu
        355                 360                 365
Ala Glu Gln Glu Arg Arg Gly Gly Gly Glu Asp Asp Val Gly Glu Glu
    370                 375                 380
Asp Glu Glu Ala Ala Glu Ala Glu Ala Glu Ala Glu Glu Ala Glu Arg
385                 390                 395                 400
Ala Arg Gln Asn Ala Leu Leu Phe Ala Glu Glu Glu Asp Gly Glu Ala
                405                 410                 415
Gly Ala Glu Asp Lys Arg Ser Gln Glu Glu Ala Pro Gly His Arg Arg
            420                 425                 430
Lys Asp Ala Glu Gly Ala Glu Glu Gly Gly Glu Glu Asp Asp Asp Asp
        435                 440                 445

Glu Glu Met Asp Pro Gln Thr Ile Asp Ser Leu Ile Glu Leu Ser Thr
450                 455                 460
Lys Leu His Leu Pro Ala Asp Asp Val Val Ser Ile Ile Glu Glu Val
465                 470                 475                 480
Glu Glu Lys Arg Lys Arg Lys Lys Asn Ala Pro Pro Glu Pro Val Pro
                485                 490                 495
Pro Pro Arg Ala Ala Pro Ala Pro Thr His Val Arg Ser Pro Gln Pro
            500                 505                 510
Pro Pro Pro Ala Pro Ala Arg Asp Glu Leu Pro Asp Trp Asn Glu Val
        515                 520                 525
Leu Pro Pro Trp Asp Arg Glu Glu Asp Glu Val Phe Pro Pro Gly Pro
    530                 535                 540
Tyr His Pro Phe Pro Asn Tyr Ile Arg Pro Arg Thr Leu Gln Pro Pro
545                 550                 555                 560
Ala Ser Ser Arg Arg Arg His Phe His His Ala Leu Pro Pro Ala Arg
                565                 570                 575
His His Pro Asp Leu Glu Ala Gln Ala Arg Arg Ala Gln Glu Glu Ala
            580                 585                 590
Asp Ala Glu Glu Arg Arg Leu Gln Glu Gln Glu Glu Leu Glu Asn Tyr
        595                 600                 605
Ile Glu His Val Leu Leu His Arg Pro
    610                 615

<210> 4
<211> 26
<212> PRT
<213> Homo sapiens

<400> 4
Arg Pro Glu Ser Ala Leu Leu Gly Gly Ser Glu Ala Gly Glu Arg Leu
                5                   10                  15

Leu Gln Gln Gly Leu Ala Gln Val Glu Ala
            20                  25

<210> 5
<211> 25
<212> PRT
<213> Rattus norvegicus

<400> 5
Leu Glu Gly Ser Phe Leu Gly Gly Ser Glu Ala Gly Glu Arg Leu Leu
                5                   10                  15
Gln Gln Gly Leu Ala Gln Val Glu Ala
            20                  25

<210> 6
<211> 25
<212> PRT
<213> Mus musculus

<400> 6
Leu Glu Gly Ser Phe Leu Gly Gly Ser Glu Ala Gly Glu Arg Leu Leu
                5                   10                  15
Gln Gln Gly Leu Ala Gln Val Glu Ala
            20                  25

<210> 7
<211> 38
<212> PRT
<213> Homo sapiens

<400> 7

Gln Ala Glu Ala Thr Arg Gln Ala Ala Ala Gln Glu Glu Arg Leu Ala
                5                   10                  15
Asp Leu Ala Ser Asp Leu Leu Leu Gln Tyr Leu Leu Gln Gly Gly Ala
            20                  25                  30
Arg Gln Arg Gly Leu Gly
        35

<210> 8
<211> 38
<212> PRT
<213> Rattus norvegicus

<400> 8
Gln Ala Glu Ala Thr Arg Gln Ala Ala Ala Gln Glu Glu Arg Leu Ala
                5                   10                  15
Asp Leu Ala Ser Asp Leu Leu Leu Gln Tyr Leu Leu Gln Gly Gly Ala
            20                  25                  30
Arg Gln Arg Asp Leu Gly
        35

<210> 9
<211> 38
<212> PRT
<213> Mus musculus

<400> 9
Gln Ala Glu Ala Thr Arg Gln Ala Ala Ala Gln Glu Glu Arg Leu Ala
                5                   10                  15
Asp Leu Ala Ser Asp Leu Leu Leu Gln Tyr Leu Leu Gln Gly Gly Ala
            20                  25                  30
Arg Gln Arg Asp Leu Gly

        35

<210> 10
<211> 1845
<212> DNA
<213> Homo sapiens

<400> 10
   atgaaagccc tcagattgtc ggcttccgcc ctcttctgcc ttctgctgat caacgggtta 60
   ggggcagcac cccctggtcg ccctgaggcg cagcctcctc ctctcagctc tgagcataaa 120
   gagccggtag ccggggacgc agtgcccggg ccaaaggatg gcagcgcccc agaggtccga 180
   ggcgctcgga attccgagcc gcaggacgag ggagagcttt tccagggcgt ggatccccgg 240
   gcgctggccg cggtgctgct gcaggcactc gaccgtcccg cctcaccccc ggcaccaagc 300
   ggctcccagc aggggccgga ggaagaagca gctgaagctc tgctgaccga gaccgtgcgc 360
   agccagaccc acagcctccc ggcgccggag agcccggagc ccgcggctcc gcctcgccct 420
   cagactccgg agaatgggcc cgaggcgagc gatccctccg aggagctcga ggcgctagcg 480
   tccctgctcc aggaactgcg agatttcagt ccaagtagcg ccaagcgcca gcaggagacg 540
   gcggcagcag agacggaaac ccgcacgcac acgctgaccc gagtgaatct ggagagcccg 600
   gggccagagc gcgtatggcg cgcttcctgg ggagagttcc aggcgcgtgt cccggagcgc 660
   gcgcccctgc cgcccccggc cccctctcaa ttccaggcgc gtatgcccga cagcgggccc 720
   cttcccgaaa cccacaagtt cggggaagga gtgtcctccc ccaaaacaca cctaggcgag 780
   gcattggcac ccctgtccaa ggcgtaccaa ggcgtggccg ccccgttccc caaggcgcgc 840
   cggccggaga gcgcactcct gggcggctcc gaggcgggcg agcgccttct ccagcaaggg 900
   ctggcgcagg tggaggccgg gcggcggcag gcggaggcca cgcggcaggc cgcggcgcag 960
   gaagagcggc tggccgacct cgcctcggac ctgctgctcc agtatttgct gcagggcggg 1020
   gcccggcagc gcggcctcgg gggtcggggg ctgcaggagg cggcggagga gcgagagagt 1080
   gcaagggagg aggaggaggc ggagcaggag agacgcggcg gggaggagag ggtgggggaa 1140
   gaggatgagg aggcggccga ggcggaggca gaggcggagg aggcggagag ggcgcggcag 1200
   aacgcgctcc tgttcgcgga ggaggaggac ggggaagccg gcgccgagga caagcgctcc 1260
   caggaggaga cgccgggcca ccggcggaag gaggccgagg ggacagagga gggcggggag 1320
   gaggaggacg acgaggagat ggatccgcag acgatcgaca gcctcattga gctgtccacc 1380
   aaactccacc tgccagcgga cgacgtggtc agcatcatcg aggaggtgga ggagaagcgg 1440
   aagcggaaga agaacgcccc tcccgagccc gtgccgcccc cccgtgccgc ccccgccccc 1500
   acccacgtcc gctccccgca gcccccgccc cccgcccccg ctcccgcacg agacgagctg 1560
   ccggactgga acgaggtgct cccgccctgg gatcgggagg aggacgaggt gtacccgcca 1620
   gggccgtacc acccttcccc caactacatc cggccgcgga cactgcagcc gccctcggcc 1680
   ttgcgccgcc gccactacca ccacgccttg ccgccttcgc gccactatcc cggccgggag 1740
   gcccaggcgc ggcgcgcgca ggaggaggcg gaggcggagg agcgccggct gcaggagcag 1800
   gaggagctgg agaattacat cgagcacgtg ctgctccggc gcccg 1845

<210> 11
<211> 1851
<212> DNA
<213> Rattus norvegicus

<400> 11
atgaaaacct tcacgttgcc agcatccgtc ctcttctgct tccttctact catccggggg 60
ttgggagcag caccccccgg gcgctccgat gtttatcctc ctcccctcgg ctctgagcat 120
aatgggcagg tagctgagga cgcagtgtcc cggccaaagg atgacagcgt cccagaggtc 180
cgagcggctc ggaattccga gcctcaggac cagggagagc tcttccaggg cgtggatccc 240
cgggcgctgg ccgcggtact gttgcaggca ctggaccgtc cggcctcgcc cccggctgtc 300
ccggcaggtt cccagcaggg aacacccgaa gaagcagcag aagctctgct gaccgagtcc 360
gtgcgcagtc agacccatag cctcccggca tcagaaatcc aagcgtccgc tgtggcgccc 420
cctcgccctc agactcagga caacgatccc gaggcagacg accgctcaga agagctggag 480
gcactagcat ccttgctcca agaacttcga gatttcagtc cgagtaatgc taagcgccag 540
caagagacgg cggcagcaga gactgaaacc cgcacgcaca cgctgacccg agtcaatctg 600
gagagccccg ggccagagcg cgtatggcgc gcttcctggg gagagttcca ggcgcgcgtc 660
ccggagcgtg ctcctctgcc gccctcggtc ccttctcaat tccaggctcg aatgtccgaa 720
aacgttcccc ttcccgaaac ccatcagttc ggggaaggag tgtcctcccc taaaacacat 780
cttggtgaga ctttgacacc cttatccaag gcgtaccaaa gtctaagtgc ccccttcccc 840
aaggtgcgtc ggctcgaggg ctcattcctg ggcggttccg aggcaggaga gcgcctgctt 900
caacaagggt tagctcaggt agaggcaggg aggaggcagg cggaggccac ccggcaggcc 960

gcagcgcaag aagagcggct ggccgatctc gcctccgacc tgctgctcca gtatttgctg 1020
cagggcggcg cccggcagcg cgatctcggg ggtcgcgggc tgcaggagac gcagcaagag 1080
cgggagaacg agagggagga ggaggcggag caggagagac gcggtggtgg ggaggacgag 1140
gtgggggaag aggatgagga ggcggcagag gcggaggcgg aggcagagga ggcggagagg 1200
gcgcggcaga acgcgctcct gttcgccgag gaggaggacg gggaagccgg agccgaggac 1260
aagcgctccc aggaggaggc gccaggccat cggcggaagg atgctgaggg gacagaggag 1320
ggcggggagg aggatgacga cgacgaagag atggatccgc agacgatcga tagtctcatt 1380
gaactgtcca ccaaactcca cctgccagca gacgatgtgg tcagcatcat cgaagaggtg 1440
gaggagaaac ggaagcggaa gaagaacgcc cctcccgagc cggtgccgcc ccccagggct 1500
gccccagccc cgacccatgt ccgctccccg cagcccccac ctcccgcccc ggcccgggat 1560
gagttgccgg actggaacga agtactccca ccctgggatc gggaggagga tgaggtgttt 1620
cccccggggc cctatcaccc cttcccaaac tacattcggc cgcggacact gcagccgccc 1680
gcatcctccc gccgccgtca cttccatcac gcgttgccac ctgcgcgcca ccatcccgat 1740
ctggaggccc aggccaggcg cgcgcaggag gaagcggacg cggaggagcg ccggctgcag 1800
gagcaggagg agctggagaa ttacattgag cacgtgctgc tgcaccgccc g 1851

<210> 12
<211> 78
<212> DNA
<213> Homo sapiens

<400> 12
cggccggaga gcgcactcct gggcggctcc gaggcgggcg agcgccttct ccagcaaggg 60
ctggcgcagg tggaggcc 78

<210> 13
<211> 75
<212> DNA
<213> Rattus norvegicus

<400> 13

ctcgagggct cattcctggg cggttccgag gcaggagagc gcctgcttca acaagggtta 60
gctcaggtag aggca 75

<210> 14
<211> 114
<212> DNA
<213> Homo sapiens

<400> 14
caggcggagg ccacgcggca ggccgcggcg caggaagagc ggctggccga cctcgcctcg 60
gacctgctgc tccagtattt gctgcagggc ggggcccggc agcgcggcct cggg 114

<210> 15
<211> 114
<212> DNA
<213> Rat

<400> 15
caggcggagg ccacccggca ggccgcagcg caagaagagc ggctggccga tctcgcctcc 60
gacctgctgc tccagtattt gctgcagggc ggcgcccggc agcgcgatct cggg 114

<210> 16
<211> 1851
<212> DNA
<213> Mouse

<400> 16
atgaaaacct tcacgttgcc ggcatccgtc ctcttctgct tccttctact gatccagggg 60
ttgggagcag cgcccccggg gcgccccgat gtttttcctc ctcccctcag ctctgagcat 120
aatgggcagg tagctgagga cgcagtgtcc cggccaaagg atgacggcgt accagaggtc 180
cgagctgctc ggaatcccga gcctcaggac cagggagagc tcttccaggg cgtggatccc 240

cgggcgctgg cctcggtact gttgcaggca ctggaccgtc cggcctcgcc cccgtcggtc 300
ccgggaggtt cccagcaggg aacacccgaa gaagcagcag aagctctgct gaccgagtcc 360
gtgcgcagtc agacccatag cctcccggca ccagaaatcc aagcgcccgc tgtggccccc 420
cctcgccctc agactcagga ccgcgatccc gaggaggacg accgctcaga agagctggag 480
gcgctagcat ccttgctcca agaacttcga gatttcagtc cgagcaatgc taagcgccaa 540
caagagacgg cggcagcaga gactgaaacc cgcacgcaca cgctgacccg agtcaatctg 600
gagagccccg ggccagagcg cgtgtggcgc gcttcctggg gagagttcca ggcgcgcgtt 660
ccggagcgcg ctcctcttcc gcccccggtc ccttcccaat tccaggctcg aatgtccgaa 720
agcgctcccc ttcccgaaac tcatcagttc ggggaaggag tgtcctcccc taaaacacat 780
ttaggtgaga ctttgacacc cttatccaag gcgtatcaaa gtctaggtgg ccccttccct 840
aaggtgcgcc ggctcgaggg ctcattcctg ggcggctccg aggcaggaga gcgcctgctt 900
cagcaaggat tagctcaggt agaggcaggg aggagacagg cggaggccac ccggcaggcc 960
gcggcgcaag aagagcggct ggccgatctc gcctccgacc tgctgctcca gtatctgttg 1020
cagggcggag cccggcagcg cgatctcggg ggtcgcgagc tgcaggagac gcagcaagag 1080
cgggagaacg agagggagga ggaggcagag caggagaggc gaggtggtgg ggaggacgat 1140
gtgggggaag aggatgagga ggcggcggag gcggaggcgg aggcagagga ggcggagagg 1200
gcgcggcaga acgcactcct gttcgccgag gaggaggacg gggaagccgg agccgaggac 1260
aaacgctccc aggaggaggc gccaggccat cggcggaagg atgctgaggg ggcagaggag 1320
ggcggggagg aggatgacga cgacgaggag atggatccgc agacgatcga tagtctcatt 1380
gaactgtcca ccaaactcca cctgccagca gacgatgtgg tcagcatcat cgaagaggtg 1440
gaggagaaac ggaagcggaa gaagaacgcc cctcccgagc cggtgccgcc tcccagggct 1500
gccccagccc cgacccatgt ccgctctccg cagcccccac ctcccgcccc agcccgggat 1560
gagttgccgg actggaacga agtgctccca ccctgggatc gggaggagga tgaggtgttt 1620
cccccggggc cctatcaccc cttcccaaac tacattcggc cgcggacact gcagccgccc 1680
gcatcctccc gccgccgtca cttccatcac gcgttgccac ctgcgcgcca ccatccggat 1740
ctggaggccc aggccaggcg cgcgcaggag gaagcggacg cggaggagcg ccggctgcag 1800
gagcaggagg agctggagaa ttacattgag cacgtgctgc tgcaccgccc g 1851

<210> 17
<211> 75
<212> DNA

<213> Mouse

<400> 17
ctcgagggct cattcctggg cggctccgag gcaggagagc gcctgcttca gcaaggatta 60
gctcaggtag aggca 75

<210> 18
<211> 114
<212> DNA
<213> Mouse

<400> 18
caggcggagg ccacccggca ggccgcggcg caagaagagc ggctggccga tctcgcctcc 60
gacctgctgc tccagtatct gttgcagggc ggagcccggc agcgcgatct cggg 114

<210> 19
<211> 8
<212> PRT
<213> artificial

<220>
<223> antigen

<220>
<221> MOD_RES
<222> (8)..(8)
<223> AMIDATION

<400> 19
Gln Gly Leu Ala Gln Val Glu Ala
1               5

<210> 20

<211> 11

<212> PRT

<213> artificial

<220>

<223> antigen

<220>

<221> MOD_RES

<222> (11)..(11)

<223> AMIDATION

<400> 20

Cys Gln Gly Gly Ala Arg Gln Arg Asp Leu Gly

1               5                   10

<210> 21

<211> 15

<212> PRT

<213> artificial

<220>

<223> antigen

<220>

<221> MOD_RES

<222> (15)..(15)

<223> AMIDATION

<400> 21
Gly Cys Tyr Leu Leu Gln Gly Gly Ala Arg Gln Arg Gly Leu Gly
1               5                   10                  15

<210> 22
<211> 12
<212> PRT
<213> artificial

<220>
<223> probe

<220>
<221> MOD_RES
<222> (12)..(12)
<223> AMIDATION

<400> 22
Tyr Leu Leu Gln Gln Gly Leu Ala Gln Val Glu Ala
1               5                   10

<210> 23
<211> 13
<212> PRT
<213> artificial

<220>
<223> probe

<220>
<221> MOD_RES

<222>  (13)..(13)
<223>  AMIDATION

<400>  23
Tyr Leu Leu Gln Gly Gly Ala Arg Gln Arg Asp Leu Gly
1               5                   10

<210>  24
<211>  13
<212>  PRT
<213>  artificial

<220>
<223>  probe

<220>
<221>  MOD_RES
<222>  (13)..(13)
<223>  AMIDATION

<400>  24
Tyr Leu Leu Gln Gly Gly Ala Arg Gln Arg Gly Leu Gly
1               5                   10